# EUROPEAN PATENT APPLICATION

(11) **EP 4 763 220 A2**
(43) Date of publication of application: **24.06.2026**
(21) Application number: 26174604.4
(22) Date of filing: 10.06.2022
(51) Int. Cl.: A61K 39/39

(54) **ANTIGEN RECOGNIZING RECEPTORS TARGETING UPAR AND USES THEREOF**

(30) Priority: 11.06.2021 US 202163209924 P
(62) Divisional of application: 22821083.7
(71) Applicant: Memorial Sloan-Kettering Cancer Center, New York, NY 10065 (US); Sloan-Kettering Institute for Cancer Research, New York, NY 10065 (US); Memorial Hospital for Cancer and Allied Diseases, New York, NY 10065 (US); Tri-institutional Therapeutics Discovery Institute, Inc., New York, NY 10021 (US)
(72) Inventor: LOWE, Scott, W., New York, NY 10065 (US); SADELAIN, Michel, New York, NY 10024 (US); AMOR VEGAS, Corina, New York, NY 11724 (US); BALDERES, Paul, New York, NY 10021 (US); LORENZ, Ivo, C., New York, NY 10021 (US); ZHANG, Zeda, New York, NY 10025 (US)
(74) Representative: Barker Brettell LLP

(57) **Abstract**

The presently disclosed subject matter provides for antigen-recognizing receptors that specifically target uPAR and cells comprising such uPAR-targeted antigen-recognizing receptors. The presently disclosed subject matter further provides uses of the uPAR-targeted antigen-recognizing receptors for treatment.

## Description

### CROSS-REFERENCE TO RELATED APPLICATIONS

This application claims priority to U.S. Provisional Application No.: 63/209,924, filed June 11, 2021, the content of which is incorporated by reference in its entirety, and to which priority is claimed.

### SEQUENCE LISTING

This application contains a Sequence Listing which has been submitted in ASCH format via EFS-Web and is hereby incorporated by reference in its entirety. Said ASCH copy, created on June 10, 2022, is named 072734.1360_ST25.txt and is 107,223bytes in size.

### 1. INTRODUCTION

The presently disclosed subject matter provides methods and compositions for immunotherapies. It relates to antigen-recognizing receptors (e.g., chimeric antigen receptors (CARs)) that specifically target uPAR, cells comprising such receptors, and methods of using such cells for treatments.

### 2. BACKGROUND OF THE INVENTION

Cell-based immunotherapy is a therapy with curative potential for the treatment of cancer. T cells and other immune cells may be modified to target tumor antigens through the introduction of genetic material coding for artificial or synthetic receptors for antigen, termed chimeric antigen receptors (CARs), specific to selected antigens. Targeted T cell therapy using CARs has shown recent clinical success in treating hematologic malignancies and solid tumors.

uPAR is associated with tumor growth or metastasis in various different types of cancers, including breast cancer, endometrial cancer, ovarian cancer, colon cancer, rectal cancer, lung cancer, stomach cancer, prostate cancer, renal cancer, pancreatic cancer, rectal cancer, acute lymphoblastic leukemia (ALL), chronic lymphocytic leukemia (CLL), and acute myeloid leukemia (AML). It also plays a role in aging, such as its association with senescence-related diseases associated with aging. It can also regulate immune response and cell-matrix interaction and promote tumor cell proliferation and emergence from dormancy.

Given the significant role for uPAR in various diseases or disorders, immunotherapies (e.g., CARs) targeting uPAR, are desired.

### 3. SUMMARY OF THE INVENTION

The presently disclosed subject matter provides antigen-recognizing receptors that specifically target uPAR and cells comprising such uPAR-targeted antigen-recognizing receptors. The presently disclosed subject matter further provides uses of the uPAR-targeted antigen-recognizing receptors for treatment.

The presently disclosed subject matter provides an antigen-recognizing receptor, comprising an extracellular antigen-binding domain, a transmembrane domain, and an intracellular signaling domain, wherein the extracellular antigen-binding domain specifically binds to uPAR. In certain embodiments, the extracellular antigen-binding domain is a single-chain variable fragment (scFv). In certain embodiments, the extracellular antigen-binding domain is a human scFv. In certain embodiments, the extracellular antigen-binding domain is a Fab, which is optionally crosslinked. In certain embodiments, the extracellular antigen-binding domain is a F(ab)₂. In certain embodiments, one or more of the scFv, Fab and F(ab)₂ are comprised in a fusion protein with a heterologous sequence to form the extracellular antigen-binding domain.

In certain embodiments, the extracellular antigen-binding domain comprises a heavy chain variable region comprising:
(a) a CDR1 comprising the amino acid sequence set forth in SEQ ID NO: 1 or a conservative modification thereof, a CDR2 comprising the amino acid sequence set forth in SEQ ID NO: 2 or a conservative modification thereof, and a CDR3 comprising the amino acid sequence set forth in SEQ ID NO: 3 or a conservative modification thereof;
(b) a CDR1 comprising the amino acid sequence set forth in SEQ ID NO: 11 or a conservative modification thereof, a CDR2 comprising the amino acid sequence set forth in SEQ ID NO: 2 or a conservative modification thereof, and a CDR3 comprising the amino acid sequence set forth in SEQ ID NO: 12 or a conservative modification thereof;
(c) a CDR1 comprising the amino acid sequence set forth in SEQ ID NO: 19 or a conservative modification thereof, a CDR2 comprising the amino acid sequence set forth in SEQ ID NO: 2 or a conservative modification thereof, and a CDR3 comprising the amino acid sequence set forth in SEQ ID NO: 20 or a conservative modification thereof;
(d) a CDR1 comprising the amino acid sequence set forth in SEQ ID NO: 19 or a conservative modification thereof, a CDR2 comprising the amino acid sequence set forth in SEQ ID NO: 27 or a conservative modification thereof, and a CDR3 comprising the amino acid sequence set forth in SEQ ID NO: 28 or a conservative modification thereof;
(e) a CDR1 comprising the amino acid sequence set forth in SEQ ID NO: 35 or a conservative modification thereof, a CDR2 comprising the amino acid sequence set forth in SEQ ID NO: 36 or a conservative modification thereof, and a CDR3 comprising the amino acid sequence set forth in SEQ ID NO: 37 or a conservative modification thereof;
(f) a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 47 or a conservative modification thereof;
(g) a CDR1 comprising the amino acid sequence set forth in SEQ ID NO: 54 or a conservative modification thereof, a CDR2 comprising the amino acid sequence set forth in SEQ ID NO: 55 or a conservative modification thereof, and a CDR3 comprising the amino acid sequence set forth in SEQ ID NO: 56 or a conservative modification thereof;
(h) a CDR1 comprising the amino acid sequence set forth in SEQ ID NO: 63 or a conservative modification thereof, a CDR2 comprising the amino acid sequence set forth in SEQ ID NO: 64 or a conservative modification thereof, and a CDR3 comprising the amino acid sequence set forth in SEQ ID NO: 65 or a conservative modification thereof;
(i) a CDR1 comprising the amino acid sequence set forth in SEQ ID NO: 73 or a conservative modification thereof, a CDR2 comprising the amino acid sequence set forth in SEQ ID NO: 74 or a conservative modification thereof; and a CDR3 comprising the amino acid sequence set forth in SEQ ID NO: 75 or a conservative modification thereof;
(j) a CDR1 comprising the amino acid sequence set forth in SEQ ID NO: 45 or a conservative modification thereof, a CDR2 comprising the amino acid sequence set forth in SEQ ID NO: 82 or a conservative modification thereof, and a CDR3 comprising the amino acid sequence set forth in SEQ 10 NO: 83 or a conservative modification thereof;
(k) a CDR1 comprising the amino acid sequence set forth in SEQ ID NO: 90 or a conservative modification thereof, a CDR2 comprising the amino acid sequence set forth in SEQ ID NO: 91 or a conservative modification thereof, and a CDR3 comprising the amino acid sequence set forth in SEQ ID NO: 92 or a conservative modification thereof;
(l) a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 99 or a conservative modification thereof; or
(m) a CDR1 comprising the amino acid sequence set forth in SEQ ID NO: 105 or a conservative modification thereof, a CDR2 comprising the amino acid sequence set forth in SEQ ID NO: 106 or a conservative modification thereof, and a CDR3 comprising the amino acid sequence set forth in SEQ ID NO: 107 or a conservative modification thereof.

In certain embodiments, the extracellular antigen-binding domain comprises a light chain variable region comprising:
(a) a CDR1 comprising the amino acid sequence set forth in SEQ ID NO: 4 or a conservative modification thereof; a CDR2 comprising the amino acid sequence set forth in SEQ ID NO: 5 or a conservative modification thereof; and a CDR3 comprising the amino acid sequence set forth in SEQ ID NO: 6 or a conservative modification thereof,
(b) a CDR1 comprising the amino acid sequence set forth in SEQ ID NO: 13 or a conservative modification thereof; a CDR2 comprising the amino acid sequence set forth in SEQ ID NO: 5 or a conservative modification thereof; and a CDR3 comprising SEQ ID NO: 14 or a conservative modification thereof;
(c) a CDR1 comprising the amino acid sequence set forth in SEQ ID NO: 21 or a conservative modification thereof, a CDR2 comprising the amino acid sequence set forth in SEQ ID NO: 5 or a conservative modification thereof; and a CDR 3 comprising the amino acid sequence set forth in SEQ ID NO: 22 or a conservative modification thereof;
(d) a CDR1 comprising the amino acid sequence set forth in SEQ ID NO: 29 or a conservative modification thereof; a CDR2 comprising SEQ ID NO: 5 or a conservative modification thereof; and a CDR3 comprising the amino acid sequence set forth in SEQ ID NO: 30 or a conservative modification thereof;
(e) a CDR1 comprising the amino acid sequence set forth in SEQ ID NO: 38 or a conservative modification thereof; a CDR2 comprising the amino acid sequence set forth in SEQ ID NO: 39 or a conservative modification thereof; and a CDR3 comprising the amino acid sequence set forth in SEQ ID NO: 40 or a conservative modification thereof;
(f) a CDR1 comprising the amino acid sequence set forth in SEQ ID NO: 48 or a conservative modification thereof; a CDR2 comprising the amino acid sequence set forth in SEQ ID NO: 5 or a conservative modification thereof; and a CDR3 comprising the amino acid sequence set forth in SEQ ID NO: 49 or a conservative modification thereof;
(g) a CDR1 comprising the amino acid sequence set forth in SEQ ID NO: 57 or a conservative modification thereof; a CDR2 comprising the amino acid sequence set forth in SEQ ID NO: 5 or a conservative modification thereof; and a CDR3 comprising the amino acid sequence set forth in SEQ ID NO: 58 or a conservative modification thereof;
(h) a CDR1 comprising the amino acid sequence set forth in SEQ ID NO: 66 or a conservative modification thereof; a CDR2 comprising the amino acid sequence set forth in SEQ ID NO: 67 or a conservative modification thereof; and a CDR3 comprising the amino acid sequence set forth in SEQ ID NO: 68 or a conservative modification thereof,
(i) a CDR1 comprising the amino acid sequence set forth in SEQ ID NO: 76 or a conservative modification thereof; a CDR2 comprising the amino acid sequence set forth in SEQ ID NO: 67 or a conservative modification thereof, and a CDR3 comprising the amino acid sequence set forth in SEQ ID NO: 77 or a conservative modification thereof;
(j) a CDR1 comprising the amino acid sequence set forth in SEQ ID NO: 84 or a conservative modification thereof; a CDR2 comprising the amino acid sequence set forth in SEQ ID NO: 67 or a conservative modification thereof; and a CDR3 comprising the amino acid sequence set forth in SEQ ID NO: 85 or a conservative modification thereof;
(k) a CDR1 comprising the amino acid sequence set forth in SEQ ID NO: 93 or a conservative modification thereof; a CDR2 comprising the amino acid sequence set forth in SEQ ID NO: 67 or a conservative modification thereof; and a CDR3 comprising the amino acid sequence set forth in SEQ ID NO: 94 or a conservative modification thereof; or
(l) a CDR1 comprising the amino acid sequence set forth in SEQ ID NO: 66 or a conservative modification thereof; a CDR2 comprising the amino acid sequence set forth in SEQ ID NO: 67 or a conservative modification thereof; and a CDR3 comprising the amino acid sequence set forth in SEQ ID NO: 100 or a conservative modification thereof.

In certain embodiments, the extracellular antigen-binding domain comprises:
(a) a heavy chain variable region comprising a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; and a light chain variable region comprising a CDR1 comprising the amino acid sequence set forth in SEQ ID NO: 4, a CDR2comprising the amino acid sequence set forth in SEQ ID NO: 5, and a CDR3comprising the amino acid sequence set forth in SEQ ID NO: 6;
(b) a heavy chain variable region comprising a CDR1 comprising the amino acid sequence set forth in SEQ ID NO: 11, a CDR2 comprising the amino acid sequence set forth in SEQ ID NO: 2, and a CDR3 comprising the amino acid sequence set forth in SEQ ID NO: 12; and a light chain variable region comprising a CDR1 comprising the amino acid sequence set forth in SEQ ID NO: 13, a CDR2comprising the amino acid sequence set forth in SEQ ID NO: 5, and a CDR3comprising the amino acid sequence set forth in SEQ ID NO: 14;
(c) a heavy chain variable region comprising a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; and a light chain variable region comprising a CDR1 comprising the amino acid sequence set forth in SEQ ID NO: 21, a CDR2comprising the amino acid sequence set forth in SEQ ID NO: 5, and a CDR3comprising the amino acid sequence set forth in SEQ ID NO: 22;
(d) a heavy chain variable region comprising a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28; and a light chain variable region comprising a CDRI comprising the amino acid sequence set forth in SEQ ID NO: 29, a CDR2comprising the amino acid sequence set forth in SEQ ID NO: 5, and a CDR3comprising the amino acid sequence set forth in SEQ ID NO: 30;
(e) a heavy chain variable region comprising a CDR 1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; and a light chain variable region comprising a CDR1 comprising the amino acid sequence set forth in SEQ ID NO: 38, a CDR2comprising the amino acid sequence set forth in SEQ ID NO: 39, and a CDR3comprising the amino acid sequence set forth in SEQ ID NO: 40;
(f) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; and a light chain variable region comprising a CDR1 comprising the amino acid sequence set forth in SEQ ID NO: 48, a CDR2comprising the amino acid sequence set forth in SEQ ID NO: 5, and a CDR3comprising the amino acid sequence set forth in SEQ ID NO: 49;
(g) a heavy chain variable region comprising a CDR1 comprising the amino acid sequence set forth in SEQ ID NO: 54, a CDR2 comprising the amino acid sequence set forth in SEQ ID NO: 55, and a CDR3 comprising the amino acid sequence set forth in SEQ ID NO: 56: and a light chain variable region comprising a CDR1 comprising the amino acid sequence set forth in SEQ ID NO: 57, a CDR2comprising the amino acid sequence set forth in SEQ ID NO: 5, and a CDR3comprising the amino acid sequence set forth in SEQ ID NO: 58;
(h) a heavy chain variable region comprising a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65; and a light chain variable region comprising a CDR1 comprising the amino acid sequence set forth in SEQ ID NO: 66, a CDR2comprising the amino acid sequence set forth in SEQ ID NO: 67, and a CDR3comprising the amino acid sequence set forth in SEQ ID NO: 68;
(i) a heavy chain variable region comprising a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; and a light chain variable region comprising a CDR1 comprising the amino acid sequence set forth in SEQ ID NO: 76, a CDR2comprising the amino acid sequence set forth in SEQ ID NO: 67, and a CDR3comprising the amino acid sequence set forth in SEQ ID NO: 77;
(j) a heavy chain variable region comprising a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83; and a light chain variable region comprising a CDR1 comprising the amino acid sequence set forth in SEQ ID NO: 84, a CDR2comprising the amino acid sequence set forth in SEQ ID NO: 67, and a CDR3comprising the amino acid sequence set forth in SEQ ID NO: 85;
(k) a heavy chain variable region comprising a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92; and a light chain variable region comprising a CDR1 comprising the amino acid sequence set forth in SEQ ID NO: 93, a CDR2comprising the amino acid sequence set forth in SEQ ID NO: 67, and a CDR3comprising the amino acid sequence set forth in SEQ ID NO: 94; or
(l) a heavy chain variable region comprising a CDR1 comprising the amino acid sequence set forth in SEQ ID NO: 45, a CDR2 comprising the amino acid sequence set forth in SEQ ID NO: 46. and a CDR3 comprising the amino acid sequence set forth in SEQ ID NO: 99; and a light chain variable region comprising a CDR1 comprising the amino acid sequence set forth in SEQ ID NO: 66, a CDR2comprising the amino acid sequence set forth in SEQ ID NO: 67, and a CDR3comprising the amino acid sequence set forth in SEQ ID NO: 100.

In certain embodiments, the extracellular antigen-binding domain comprises a heavy chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86. SEQ ID NO: 95, SEQ ID NO: 101, or SEQ ID NO: 108. In certain embodiments, the extracellular antigen-binding domain comprises a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78. SEQ ID NO: 86, SEQ ID NO: 95, SEQ ID NO: 101, or SEQ ID NO: 108.

In certain embodiments, the extracellular antigen-binding domain comprises a light chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 8, SEQ ID NO: 16, SEQ ID NO: 24, SEQ ID NO: 32. SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102. In certain embodiments, the extracellular antigen-binding domain comprises a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 5 1, SEQ ID NO: 60, SEQ ID NO: 70, SEQ ID NO: 79, SEQ ID NO: 87, SEQ ID NO: 96, or SEQ ID NO: 102.

In certain embodiments. the extracellular antigen-binding domain comprises: (a) a heavy chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108; and (b) a light chain variable region comprising an amino acid sequence that is at least about 80%, about 81%, about 82%, about 83%. about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79. SEQ ID NO: 87, SEQ ID NO: 96, or SEQ ID NO: 102. In certain embodiments, the extracellular antigen-binding domain comprises: (a) a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108; and (b) a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

In certain embodiments, the extracellular antigen-binding domain comprises:
(a) a heavy chain variable region comprising the amino acid sequence set forth in SEQ ID NO: 7, and a light chain variable region comprising the amino acid sequence set forth in SEQ ID NO: 8:
(b) a heavy chain variable region comprising the amino acid sequence set forth in SEQ ID NO: 15, and a light chain variable region comprising the amino acid sequence set forth in SEQ ID NO: 16;
(c) a heavy chain variable region comprising the amino acid sequence set forth in SEQ ID NO: 23, and a light chain variable region comprising the amino acid sequence set forth in SEQ ID NO: 24;
(d) a heavy chain variable region comprising the amino acid sequence set forth in SEQ ID NO: 31, and a light chain variable region comprising the amino acid sequence set forth in SEQ ID NO: 32;
(e) a heavy chain variable region comprising the amino acid sequence set forth in SEQ ID NO: 41, and a light chain variable region comprising the amino acid sequence set forth in SEQ ID NO: 42;
(f) a heavy chain variable region comprising the amino acid sequence set forth in SEQ ID NO: 50, and a light chain variable region comprising the amino acid sequence set forth in SEQ ID NO: 51;
(g) a heavy chain variable region comprising the amino acid sequence set forth in SEQ ID NO: 59, and a light chain variable region comprising the amino acid sequence set forth in SEQ ID NO: 60:
(h) a heavy chain variable region comprising the amino acid sequence set forth in SEQ ID NO: 69, and a light chain variable region comprising the amino acid sequence set forth in SEQ ID NO: 70;
(i) a heavy chain variable region comprising the amino acid sequence set forth in SEQ ID NO: 78, and a light chain variable region comprising the amino acid sequence set forth in SEQ ID NO: 79;
(j) a heavy chain variable region comprising the amino acid sequence set forth in SEQ ID NO: 86, and a light chain variable region comprising the amino acid sequence set forth in SEQ ID NO: 87;
(k) a heavy chain variable region comprising the amino acid sequence set forth in SEQ ID NO: 95, and a light chain variable region comprising the amino acid sequence set forth in SEQ ID NO: 96, or
(l) a heavy chain variable region comprising the amino acid sequence set forth in SEQ ID NO: 101, and a light chain variable region comprising the amino acid sequence set forth in SEQ ID NO: 102.

In certain embodiments, the extracellular antigen-binding domain comprises a linker between a heavy chain variable region and a light chain variable region of the extracellular antigen-binding domain. In certain embodiments, the linker comprises or consists of the amino acid sequence set forth in SEQ ID NO: 110, SEQ ID NO: 111, SEQ ID NO: 112, SEQ ID NO: 113, SEQ ID NO: 114, or SEQ ID NO: 115.

In certain embodiments, the extracellular antigen-binding domain comprises a signal peptide that is covalently joined to the 5' terminus of the extracellular antigen-binding domain. In certain embodiments, the transmembrane domain comprises a CD8 polypeptide, a CD28 polypeptide, a CD3ζ polypeptide, a CD4 polypeptide. a 4-1 BB polypeptide, an OX40 polypeptide, an ICOS polypeptide, a CTLA-4 polypeptide, a PD-1 polypeptide, a LAG-3 polypeptide, a 2B4 polypeptide, a BTLA polypeptide, or a combination thereof. In certain embodiments, the intracellular signaling domain comprises a CD3ζ polypeptide. In certain embodiments, the intracellular signaling domain further comprises at least one co-stimulatory signaling region. In certain embodiments, the at least one co-stimulatory signaling region comprises a CD28 polypeptide, a 4-1BB polypeptide, an OX40 polypeptide, an ICOS polypeptide, a DAP-10 polypeptide, or a combination thereof.

In certain embodiments, the antigen-recognizing receptor is a chimeric antigen receptor (CAR), a T-cell Receptor (TCR). or a T-cell like fusion protein. In certain embodiments, the antigen-recognizing receptor is a CAR.

In certain embodiments, the antigen-recognizing receptor is recombinantly expressed. In certain embodiments, the antigen-recognizing receptor is expressed from a vector. In certain embodiments, the vector is a γ-retroviral rector.

The presently disclosed subject matter provides cells comprises a presently disclosed antigen-recognizing receptor. In certain embodiments, the cell is transduced with the antigen-recognizing receptor. In certain embodiment, the antigen-recognizing receptor is constitutively expressed on the surface of the cell.

In certain embodiments, the cell is an immunoresponsive cell. In certain embodiments, the cell is a cell of the lymphoid lineage or a cell of the myeloid lineage. In certain embodiments, the cell is selected from the group consisting of a T cell, a Natural Killer (NK) cell, and a stem cell from which lymphoid cells may be differentiated. In certain embodiments, the cell is a T cell. In certain embodiments, the T cell is a cytotoxic T lymphocyte (CTL) or a regulatory T cell. In certain embodiments, the stem cell is a pluripotent stem cell. In certain embodiments, the pluripotent stem cell is an embryoid stem cell or an induced pluripotent stem cell.

The presently disclosed subject matter further provides nucleic acid that encode a presently disclosed antigen-recognizing receptor. The presently disclosed subject matter further provides vectors comprising the presently disclosed nucleic acid molecules. In certain embodiments, the vector is a viral vector. In certain embodiments, the vector is a γ-retroviral rector.

In addition, the presently disclosed subject matter provides host cells expressing the nucleic acid molecule disclosed herein. In certain embodiments, the host cell is a T cell.

The presently disclosed subject matter further provides compositions comprising the cells disclosed herein. In certain embodiments, the composition is a pharmaceutical composition further comprising a pharmaceutically acceptable carrier.

The presently disclosed subject matter further provides methods of treating or ameliorating a disease or disorder in a subject. In certain embodiments, the method comprises administering to the subject the presently disclosed cells, or the compositions. In certain embodiments, the disease or disorder is selected from the group consisting of tumors, senescence-associated pathologies, and tissue decline associated with aging. In certain embodiments, the disease or disorder is selected from the group consisting of lung fibrosis, cardiac fibrosis, liver fibrosis, atherosclerosis, osteoarthritis, diabetes. chronic kidney disease, Alzheimer's disease, and Parkinson disease.

In certain embodiments, the disease or disorder is a senescence-associated pathology. In certain embodiments, the senescence-associated pathology is selected from the group consisting of lung fibrosis, atherosclerosis, Alzheimer's disease, diabetes, osteoarthritis, liver fibrosis, chronic kidney disease, cardiac fibrosis, and Parkinson's disease.

In certain embodiments, the disease or disorder is a tumor. In certain embodiments, the tumor is selected from the group consisting of breast cancer (including triple negative breast cancer), endometrial cancer, ovarian cancer, colon cancer, rectal cancer, lung cancer (e.g., non-small cell lung cancer), stomach cancer, prostate cancer, gastric cancer, renal cancer, pancreatic cancer, blood cancer, cervical cancer, head and neck cancer, liver cancer (e.g., cholangiocarcinoma, hepatocellular carcinoma, and fibrolamaellar hepatocellular carcinoma), urotherial cancer, melanoma, and brain cancer (including glioblastoma multiforme). (In certain embodiments, the blood cancer is selected from the group consisting of acute lymphoblastic leukemia (ALL), chronic lymphocytic leukemia (CLL), acute myeloid leukemia (AML), myelofibrosis, polycythemia vera, myelodysplastic syndrome, erythroleukemia. In certain embodiments, the tumor is cancer.

The presently disclosed subject matter further provides methods of increasing production of an immune-activating cytokine in response to a tumor cell in a subject. In certain embodiments, the method comprises administering to the subject the presently disclosed cells or composition. In certain embodiments, the immune-activating cytokine is selected from the group consisting of granulocyte macrophage colony stimulating factor (GM-CSF), IFN-α, IFN-β, IFN-γ, TNF-α, IL-1, IL-2, IL-3, IL-6, IL-11, IL-7, IL-8, IL-12, IL-15, IL-21, interferon regulatory factor 7 (IRF7), CCL1, CCL2, CCL3, CCL5, CCL7, CCL8, CCL13, CCL16. CXCL1, CXCL3, CXCL5, CXCL9, CXCL10, and combinations thereof. In certain embodiments, the subject is a human.

The presently disclosed subject matter further provides kits for treating or ameliorating a disease or disorder in a subject, and/or increasing production of an immune-activating cytokine in response to a tumor cell in a subject, comprising the presently disclosed cells, the nucleic acid, or the composition. In certain embodiments, the kit further comprises written instructions for using the presently disclosed cell or composition for treating or ameliorating a disease or disorder in a subject, and/or increasing production of an immune-activating cytokine in response to a tumor cell in a subject.

In addition, the presently disclosed subject matter provides methods of producing a uPAR-targeted antigen-recognizing receptor, comprising introducing into the cell a nucleic acid that encodes the antigen-recognizing receptor.

### 4. DETAILED DESCRIPTION OF THE INVENTION

The presently disclosed subject matter provides antigen-recognizing receptors (e.g., chimeric antigen receptors (CARs)) that specifically target uPAR. The presently disclosed subject matter further provides cells comprising such receptors. The cells can be immunoresponsive cells, e.g.. genetically modified immunoresponsive cells (*e.g.*, T cells or NK cells). The presently disclosed subject matter also provides methods of using such cells for treatments. e.g., for treating and or ameliorating a disease or disorder.

Non-limiting embodiments of the present disclosure are described by the present specification and Examples.

For purposes of clarity of disclosure and not by way of limitation, the detailed description is divided into the following subsections:
- 4.1.: Definitions;
- 4.2.: uPAR;
- 4.3.: Antigen-Recognizing Receptors:
- 4.4.: Cells;
- 4.5.: Compositions and Vectors;
- 4.6.: Polypeptides;
- 4.7.: Formulations and Administration;
- 4.8.: Methods of Treatment:
- 4.9.: Kits; and
- 4.10.: Exemplary Embodiments.

### 4.1. Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

As used herein, the term "about" or "approximately" means within an acceptable error range for the particular value as determined by one of ordinary skill in the art, which will depend in part on how the value is measured or determined. *i.e.*, the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

By "immunoresponsive cell" is meant a cell that functions in an immune response or a progenitor, or progeny thereof. In certain embodiments, the immunoresponsive cell is a cell of lymphoid lineage. Non-limiting examples of cells of lymphoid lineage include T cells, Natural Killer (NK) cells, B cells, and stem cells from which lymphoid cells may be differentiated. In certain embodiments, the immunoresponsive cell is a cell of myeloid lineage.

By "activates an immunoresponsive cell" is meant induction of signal transduction or changes in protein expression in the cell resulting in initiation of an immune response. For example, when CD3 Chains cluster in response to ligand binding and immunoreceptor tyrosine-based inhibition motifs (ITAMs) a signal transduction cascade is produced. In certain embodiments, when an endogenous TCR or an exogenous CAR binds to an antigen, a formation of an immunological synapse occurs that includes clustering of many molecules near the bound receptor (e.g. CD4 or CD8, CD3γ/δ/ε/ζ, etc.). This clustering of membrane bound signaling molecules allows for ITAM motifs contained within the CD3 chains to become phosphorylated. This phosphorylation in turn initiates a T cell activation pathway ultimately activating transcription factors, such as NF-κB and AP-1. These transcription factors induce global gene expression of the T cell to increase IL-2 production for proliferation and expression of master regulator T cell proteins in order to initiate a T cell mediated immune response.

By "stimulates an immunoresponsive cell" is meant a signal that results in a robust and sustained immune response. In various embodiments, this occurs after immune cell (*e.g.,* T-cell) activation or concomitantly mediated through receptors including, but not limited to, CD28, CD137 (4-1BB), OX40, CD40 and ICOS. Receiving multiple stimulatory signals can be important to mount a robust and long-tenn T cell mediated immune response. T cells can quickly become inhibited and unresponsive to antigen. While the effects of these co-stimulatory signals may vary, they generally result in increased gene expression in order to generate long lived, proliferative, and anti-apoptotic T cells that robustly respond to antigen for complete and sustained eradication.

The term "antigen-recognizing receptor" as used herein refers to a receptor that is capable of recognizing a target antigen (e.g., uPAR). In certain embodiments, the antigen-recognizing receptor is capable of activating an immune or immunoresponsive cell (*e.g.,* a T cell) upon its binding to the target antigen.

As used herein, the term "antibody" means not only intact antibody molecules, but also fragments of antibody molecules that retain immunogen-binding ability. Such fragments are also well known in the art and are regularly employed both *in vitro* and *in vivo.* Accordingly, as used herein, the term "antibody" means not only intact immunoglobulin molecules but also the well-known active fragments F(ab')₂, and Fab. F(ab')₂, and Fab fragments that lack the Fe fragment of intact antibody, clear more rapidly from the circulation, and may have less non-specific tissue binding of an intact antibody (Wahl et al., Nuel Med (1983);24:216-325). As used herein, include whole native antibodies, bispecific antibodies; chimeric antibodies; Fab, Fab', single chain V region fragments (scFv), fusion polypeptides, and unconventional antibodies. In certain embodiments, an antibody is a glycoprotein comprising at least two heavy (H) chains and two light (L) chains inter-connected by disulfide bonds. Each heavy chain is comprised of a heavy chain variable region (abbreviated herein as V_{H}) and a heavy chain constant (C_{H}) region. The heavy chain constant region is comprised of three domains, CH1, CH2 and CH3. Each light chain is comprised of a light chain variable region (abbreviated herein as V_{L}) and a light chain constant C_{L} region. The light chain constant region is comprised of one domain, C_{L}, The V_{H} and V_{L} regions can be further sub-divided into regions of hypervariability, termed complementarity determining regions (CDR), interspersed with regions that are more conserved, termed framework regions (FR). Each V_{H} and V_{L} is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

As used herein, "CDRs" are defined as the complementarity determining region amino acid sequences of an antibody which are the hypervariable regions of immunoglobulin heavy and light chains. *See, e.g.,* Kabat et al., Sequences of Proteins of Immunological Interest, 4th U. S. Department of Health and Human Services, National Institutes of Health (1987), or IMGT numbering system (Lefranc, The Immunologist (1999);7:132-136; Lefranc et al., Dev. Comp. Immunol. (2003):27:55-77). Generally, antibodies comprise three heavy chain and three light chain CDRs or CDR regions in the variable region. CDRs provide the majority of contact residues for the binding of the antibody to the antigen or epitope. In certain embodiments, the CDRs regions are delineated using the IMGT numbering system. In certain embodiments, the CDR regions are delineated using the IMGT numbering system accessible at http://www.imgt.org/IMGT_vquest/input.

As used herein, the term "single-chain variable fragment" or "scFv" is a fusion protein of the variable regions of the heavy (V_{H}) and light chains (V_{L}) of an immunoglobulin (e.g., mouse or human) covalently linked to form a V_{H}::VL heterodimer. The heavy (V_{H}) and light chains (V_{L}) are either joined directly or joined by a peptide-encoding linker (e.g., 10, 15. 20, 25 amino acids), which connects the N-terminus of the V_{H} with the C-terminus of the V_{L}, or the C-terminus of the V_{H} with the N-terminus of the V_{L}, The linker is usually rich in glycine for flexibility, as well as serine or threonine for solubility. The linker can link the heavy chain variable region and the light chain variable region of the extracellular antigen-binding domain. Non-limiting examples of linkers are disclosed in Shen et al., Anal. Chem. 80(6):1910-1917 (2008) and WO 2014/087010, the contents of which are hereby incorporated by reference in their entireties. In certain embodiments, the linker is a G4S linker.

In certain embodiments, the linker comprises or consists of the amino acid sequence set forth in SEQ ID NO: 110, which is provided below:
GGGGSGGGGSGGGSGGGGS [SEQ ID NO: 110]

In certain embodiments, the linker comprise or consists of the amino acid sequence set forth in SEQ ID NO: 111, which is provided below:
GGGGSGGGGSGGGGS [SEQ ID NO: 111]

In certain embodiments, the linker comprises or consists of the amino acid sequence set forth in SEQ ID NO: 112, which is provided below:
GGGGSGGGGSGGGGSGGGSGGGGS [SEQ ID NO: 112]

In certain embodiments, the linker comprises or consists of the amino acid sequence set forth in SEQ ID NO: 113, which is provided below:
GGGGSGGGGSGGGGSGGGGSGGGSGGGGS [SEQ ID No: 113]

In certain embodiments, the linker comprises or consists of the amino acid sequence set forth in SEQ ID NO: 114, which is provided below:
GGGGS [SEQ ID NO: 114]

In certain embodiments, the linker comprises or consists of the amino acid sequence set forth in SEQ ID NO: 115, which is provided below:
GGGGSGGGGS [SEQ ID NO: 115]

Despite removal of the constant regions and the introduction of a linker, scFv proteins retain the specificity of the original immunoglobulin. Single chain Fv polypeptide antibodies can be expressed from a nucleic acid comprising V_{H} - and V_{L} -encoding sequences as described by Huston. et al. Proc. Nat. Acad. Sci. USA, (1988);85:5879-5883; U.S. Patent Nos. 5,091,513, 5,132,405 and 4,956,778; and U.S. Patent Publication Nos. 20050196754 and 20050196754. Antagonistic scFvs having inhibitory activity have been described (see, e.g., Zhao et al., Hyrbidoma (Larchmt) (2008);27(6):455-51; Peter et al., J Cachexia Sarcopenia Muscle (2012);August 12; Shieh et al., J Imunol (2009);183(4):2277-85; Giomarelli et al., Thromb Haemost (2007);97(6):955-63; Fife eta., J Clin Invst (2006);116(8):2252-61; Brocks et al., Immunotechnology 1997 3(3):173-84; Moosmayer et al., Ther Immunol 1995 2(10:31-40). Agonistic scFvs having stimulatory activity have been described (Peter et al., J Biol Chern (2003);25278(38):36740-7; Xie et al., Nat Biotech 1997 15(8):768-71; Ledbetter et al.. Crit Rev Immunol (1997);17(5-6):427-55; Ho et al., BioChim Biophys Acta (2003);1638(3):257-66).

The term "chimeric antigen receptor" or "CAR" as used herein refers to a molecule comprising an extracellular antigen-binding domain that is fused to an intracellular signaling domain that is capable of activating or stimulating an immunoresponsive cell, and a transmembrane domain. In certain embodiments, the extracellular antigen-binding domain of a CAR comprises a scFv. The scFv can be derived from fusing the variable heavy and light regions of an antibody. Alternatively or additionally. the scFv may be derived from Fab's (instead of from an antibody, e.g., obtained from Fab libraries). In certain embodiments, the scFv is fused to the transmembrane domain and then to the intracellular signaling domain. By "substantially identical" or "substantially homologous" is meant a polypeptide or nucleic acid molecule exhibiting at least about 50% homologous or identical to a reference amino acid sequence (for example, any of the amino acid sequences described herein) or a reference nucleic acid sequence (for example, any of the nucleic acid sequences described herein). In certain embodiments, such a sequence is at least about 60%, at least about 65%, at least about 70%, at least about 75%, at least about 80%, at least about 85%, at least about 90%, at least about 95%, at least about 99%, or at least about 100% homologous or identical to the sequence of the amino acid or nucleic acid used for comparison.

Sequence identity can be measured by using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid. glutamic acid, asparagine, glutamine; serine, threonine; lysine, arginine; and phenylalanine, tyrosine. In an exemplary approach to determining the degree of identity, a BLAST program may be used, with a probability score between e-3 and e-100 indicating a closely related sequence.

As used herein, the percent homology between two amino acid sequences is equivalent to the percent identity between the two sequences. The percent identity between the two sequences is a function of the number of identical positions shared by the sequences (*i.e.*, % homology = # of identical positions/total # of positions × 100), taking into account the number of gaps, and the length of each gap, which need to be introduced for optimal alignment of the two sequences. The comparison of sequences and determination of percent identity between two sequences can be accomplished using a mathematical algorithm.

The percent homology between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent homology between two amino acid sequences can be determined using the Needleman and Wunsch (J. Mol. Biol. 48:444-453 (1970)) algorithm which has been incorporated into the GAP program in the GCG software package (available at www.geg.com), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the amino acids sequences of the presently disclosed subject matter can further be used as a "query sequence" to perform a search against public databases to, for example, identify related sequences. Such searches can be performed using the XBLAST program (version 2.0) of Altschul, et al. (1990) J. Mol. Biol. 215:403-10. BLAST protein searches can be performed with the XBLAST program, score = 50, wordlength = 3 to obtain amino acid sequences homologous to the specified sequences (e.g., heavy and light chain variable region sequences of scFv m903, m904, m905, m906, and m900) disclosed herein. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used.

An "effective amount" is an amount sufficient to affect a beneficial or desired clinical result upon treatment. An effective amount can be administered to a subject in one or more doses. In certain embodiments, an effective amount can be an amount that is sufficient to palliate, ameliorate, stabilize, reverse or slow the progression of the disease, or otherwise reduce the pathological consequences of the disease. The effective amount can be determined by a physician on a case-by-case basis and is within the skill of one in the art. Several factors are typically taken into account when determining an appropriate dosage to achieve an effective amount. These factors include age, sex and weight of the subject, the condition being treated, the severity of the condition and the form and effective concentration of the cells administered.

As used herein, the term "endogenous" refers to a nucleic acid molecule or polypeptide that is normally expressed in a cell or tissue.

As used herein, the term "exogenous" refers to a nucleic acid molecule or polypeptide that is not endogenously present in a cell. The term "exogenous" would therefore encompass any recombinant nucleic acid molecule or polypeptide expressed in a cell, such as foreign. heterologous, and over-expressed nucleic acid molecules and polypeptides. By "exogenous" nucleic acid is meant a nucleic acid not present in a native wild-type cell; for example, an exogenous nucleic acid may vary from an endogenous counterpart by sequence, by position/location, or both. For clarity, an exogenous nucleic acid may have the same or different sequence relative to its native endogenous counterpart; it may be introduced by genetic engineering into the cell itself or a progenitor thereof, and may optionally be linked to alternative control sequences, such as a non-native promoter or secretory sequence.

By a "heterologous nucleic acid molecule or polypeptide" is meant a nucleic acid molecule (*e.g.*, a cDNA, DNA or RNA molecule) or polypeptide that is not normally present in a cell or sample obtained from a cell. This nucleic acid may be from another organism, or it may be, for example, an mRNA molecule that is not normally expressed in a cell or sample.

By "increase" is meant to after positively by at least about 5%. An alteration may be by about 5%, about 10%, about 25%, about 30%, about 50%, about 75%, about 100% or more.

By "reduce" is meant to alter negatively by at least about 5%. An alteration may be by about 5%, about 10%, about 25%, about 30%, about 50%, about 75%, or even by about 100%.

The terms "isolated," "purified," or "biologically pure" refer to material that is free to varying degrees from components which normally accompany it as found in its native state. "Isolate" denotes a degree of separation from original source or surroundings. "Purify" denotes a degree of separation that is higher than isolation. A "purified" or "biologically pure" protein is sufficiently free of other materials such that any impurities do not materially affect the biological properties of the protein or cause other adverse consequences. That is, a nucleic acid or peptide is purified if it is substantially free of cellular material, viral material, or culture medium when produced by recombinant DNA techniques, or chemical precursors or other chemicals when chemically synthesized. Purity and homogeneity are typically determined using analytical chemistry techniques, for example, polyacrylamide gel electrophoresis or high-performance liquid chromatography. The term "purified" can denote that a nucleic acid or protein gives rise to essentially one band in an electrophoretic gel. For a protein that can be subjected to modifications, for example. phosphorylation or glycosylation, different modifications may give rise to different isolated proteins, which can be separately purified.

By "isolated cell" is meant a cell that is separated from the molecular and or cellular components that naturally accompany the cell.

The term "antigen-binding domain" as used herein refers to a domain capable of specifically binding a particular antigenic determinant or set of antigenic determinants present on a cell.

By "recognize" is meant selectively binds to a target. A T cell that recognizes a tumor can expresses a receptor (e.g., a CAR) that binds to a tumor antigen.

By "signal sequence" or "leader sequence" is meant a peptide sequence (*e.g.*, 5, 10, 15, 20, 25 or 30 amino acids) present at the N-terminus of newly synthesized proteins that directs their entry to the secretory pathway

By "specifically binds" or "specifically binds to" or "specifically target" is meant a polypeptide or a fragment thereof that recognizes and or binds to a biological molecule of interest (*e.g.,* a polypeptide, e.g., a uPAR polypeptide), but which does not substantially recognize and/or bind other molecules in a sample, for example, a biological sample, which naturally includes a presently disclosed polypeptide (e.g., a uPAR polypeptide).

The terms "comprises", "comprising", and are intended to have the broad meaning ascribed to them in U.S. Patent Law and can mean "includes", "including" and the like.

As used herein, "treatment" refers to clinical intervention in an attempt to alter the disease course of the individual or cell being treated, and can be performed either for prophylaxis or during the course of clinical pathology. Therapeutic effects of treatment include, without limitation, preventing occurrence or recurrence of disease, alleviation of symptoms, diminishment of any direct or indirect pathological consequences of the disease, preventing metastases, decreasing the rate of disease progression, amelioration or palliation of the disease state, and remission or improved prognosis. By preventing progression of a disease or disorder, a treatment can prevent deterioration due to a disorder in an affected or diagnosed subject or a subject suspected of having the disorder, but also a treatment may prevent the onset of the disorder or a symptom of the disorder in a subject at risk for the disorder or suspected of having the disorder.

An "individual" or "subject" herein is a vertebrate, such as a human or non-human animal, for example, a mammal. Mammals include, but are not limited to, humans, primates, farm animals, sport animals, rodents and pets. Non-limiting examples of non-human animal subjects include rodents such as mice, rats, hamsters, and guinea pigs; rabbits; dogs; cats; sheep: pigs; goats; cattle; horses; and non-human primates such as apes and monkeys. The term "immunocompromised" as used herein refers to a subject who has an immunodeficiency. The subject is very vulnerable to opportunistic infections, infections caused by organisms that usually do not cause disease in a person with a healthy immune system but can affect people with a poorly functioning or suppressed immune system.

Other aspects of the presently disclosed subject matter are described in the following disclosure and are within the ambit of the presently disclosed subject matter.

### 4.2. uPAR

uPAR (urokinase-type plasminogen activator receptor), also known as CD87, is a glycosylphosphatidylinositol-anchored protein, uPAR is cysteine-rich and consists of three tandem LU domains, which bind urokinase-type plasminogen activator (uPA). (Kessler et al., J. Neurochem. (2017);142: 7-18; Llinas et al., EMBO J. (2005):24(9): 1655-63; Huai et al., Science (2006);311 (5761):656-9; Chelsea et al., Human Genomics (2016); 10:10), uPAR also interacts with several other proteins, including vitronectin, the uPAR associated protein (uPARAP) and the integrin family of membrane proteins.

uPAR is associated with tumor growth or metastasis in various different types of cancers, including breast cancer (including triple negative breast cancer), endometrial cancer, ovarian cancer, colon cancer, rectal cancer, lung cancer, stomach cancer, prostate cancer, renal cancer, pancreatic cancer, rectal cancer, cervical cancer, head and neck cancer, liver cancer, gastric cancer, urotherial cancer, melanoma, brain cancer (including glioblastoma multiforme), acute lymphoblastic leukemia (ALL), chronic lymphocytic leukemia (CLL), and acute myeloid leukemia (AML). It also plays a role in aging, such as its association with senescence-related diseases associated with aging. It can also regulate immune response and cell-matrix interaction and promote tumor cell proliferation and emergence from dormancy.

uPAR is induced during the process of cellular senescence, which can be elicited by certain cancer agents and accumulates in a range of age-related and tissue damage pathologies (LIST). Elimination of senescent cells can improve the response of therapy, and ameliorate symptoms of the tissue damage pathologies including fibrosis, etc.

Soluble urokinase plasminogen activator receptor (suPAR) is found upregulated in a number of pathologies noted above, also in chronic obstructive pulmonary disease, asthma, liver failure, heart failure, cardiovascular disease, and rheumatoid arthritis. (Desmedt et al., Crit. Rev. Clin. Lab. Sci. (2017);54(2): 117-133). uPAR is found to be highly expressed on senescent cells. (Wagner et al., Nature (2020);583 (7814): 37-38, Amor et al., Nature (2020 Jul),583(7814):127-132). Thus, uPAR (e.g., suPAR) can be used as a disease stage biomarker. Many oncogenic signaling pathways and tumor microenvironmental conditions such as hypoxia can activate transcription factors that in turn regulate uPAR. uPAR can regulate proteolysis by associating with the outer layer of the plasma membrane by a glycosyl phosphatidylinositol (GPI) anchor, but it can also be secreted or shed from the cell surface. (Harvey et al., Nat. Rev. Mol. Cell Biol (2010); 11, 23-36), uPAR expression directly correlates with the invasive potential of endometrial carcinomas. (Foca et al., Gynecol. Oncol. (2000);79(2):244-50). uPAR is implicated in several hematological malignancies, particularly acute leukemia and multiple myeloma. (Hata et al., Blood (1993); 81: 3357-3364; MC Bene et al., Leukemia (2004); 18, 394-400). uPAR is reported to be associated with poor prognosis in breast cancer patients. (Bo et al., Oncol. Rep. (2005); 14(1):105-12; Foekens et al., Cancer Rev. (2000); 60(3): 636-43).

In certain embodiments, uPAR is human uPAR comprising or consisting of the amino acid sequence with a UniProt Reference No: Q03405-1 (SEQ ID NO: 116) or a fragment thereof. SEQ ID NO: 116 is provided below. In certain embodiments, the uPAR comprises three domains: domain 1 (domain UPAR/Ly6 1), domain 2 (domain UPAR/Ly6 2), and domain 3 (domain UPAR/Ly6 3). In certain embodiments, domain 1 comprises or consists of amino acids 23 to 114 of SEQ ID NO: 116. In certain embodiments, domain 2 comprises or consists of amino acids 115 to 213 of SEQ ID NO: 116. In certain embodiments, domain 3 comprises or consists of amino acids 214 to 305 of SEQ ID NO: 116.

MGHFPLLPLL LLLHTCVFAS WGLRCMQCKT NGDCRVEECA LGQDLCRTTI VRLWEEGEEL
ELVEKSCTHS EKTNRTLSYR TGLKITSLTE VVCGLDLCNQ GNSGRAVTYS RSRYLECISC
GSSDMSCERG RHQSLQCRSP EEQCLDVVTH WIQEGEEGRP KDDRHLRGCG YLPGCPGSNG
FHNNDTFHFL KCCNTTKCNE GPILELENLP QNGRQCYSCK GNSTHGCSSE ETFLIDCRGP
MNQCLVATGT HEPKNQSYMV RGCATASMCQ HAHLGDAFSM NHIDVSCCTK SGCNHPDLDV
QYRSGAAPQP GPAHLSLTIT LLMTARLWGG TLLWT [SEQ ID NO: 116]

In certain embodiments, the uPAR comprises or consists of an amino acid sequence that is at least about 80%, at least about 85%, at least about 90%, at least about 95%, at least about 96%, at least about 97%, at least about 98%, or at least about 99%, at least about 100% identical to the amino acid sequence set forth in SEQ ID NO: 116 or a fragment thereof.

In certain embodiments, the antigen-recognizing receptor bind to a portion of human uPAR. In certain embodiments, the antigen-recognizing receptor bind to at least one of domain 1, domain 2, and domain 3. In certain embodiments, the antigen-recognizing receptor bind to domain 2. In certain embodiments, the antigen-recognizing receptor bind to domain 3. In certain embodiments, the antigen-recognizing receptor bind to both domain 2 and domain 3. In certain embodiments, the antigen-recognizing receptor bind to amino acids 115 to 303 of SEQ ID NO: 116. In certain embodiments, the antigen-recognizing receptor bind to amino acids 115 to 305 of SEQ ID NO: 116.

### 4.3. Antigen-Recognizing Receptors

The presently disclosed antigen-recognizing receptors specifically target or binds to uPAR. In certain embodiments, the antigen-recognizing receptor is a chimeric antigen receptor (CAR). In certain embodiments, the antigen-recognizing receptor is a T-cell receptor (TCR). In certain embodiments, the antigen-recognizing receptor is a TCR like fusion molecule.

The presently disclosed subject matter also provides nucleic acid molecules that encode the presently disclosed antigen-recognizing receptors. In certain embodiments, the nucleic acid molecule comprises a nucleotide sequence that encodes a polypeptide of a uPAR-targeted antigen recognizing receptor disclosed herein.

### 4.3.1. T-Cell Receptor (TCR)

In certain embodiments, the antigen-recognizing receptor is a TCR. A TCR is a disulfide-linked heterodimeric protein consisting of two variable chains expressed as part of a complex with the invariant CD3 chain molecules. A TCR found on the surface of T cells is responsible for recognizing antigens as peptides bound to major histocompatibility complex (MHC) molecules. In certain embodiments, a TCR comprises an alpha chain and a beta chain (encoded by TRA and TRB, respectively). In certain embodiments, a TCR comprises a gamma chain and a delta chain (encoded by TRG and TRD, respectively).

Each chain of a TCR is composed of two extracellular domains: Variable (V) region and a Constant (C) region. The Constant region is proximal to the cell membrane, followed by a transmembrane region and a short cytoplasmic tail. The Variable region binds to the peptide/MHC complex. The variable domain of both chains each has three complementarity determining regions (CDRs).

In certain embodiments, a TCR can form a receptor complex with three dimeric signaling modules CD3δ/ε, CD3γ/ε and CD247 ζ/ζ or ζ/η. When a TCR complex engages with its antigen and MHC (peptide/MHC), the T cell expressing the TCR complex is activated.

In certain embodiments, the TCR is an endogenous TCR. In certain embodiments, the antigen-recognizing receptor is naturally occurring TCR.

In certain embodiments, the antigen-recognizing receptor is an exogenous TCR. In certain embodiments, the antigen-recognizing receptor is a recombinant TCR. In certain embodiments, the antigen-recognizing receptor is a recombinant TCR. In certain embodiments, the recombinant TCR differs from any recombinant TCR by at least one amino acid residue. In certain embodiments, the recombinant TCR differs from any naturally occurring TCR by at least about 2, about 3, about 4. about 5, about 6, about 7, about 8. about 9. about 10, about 11, about 12, about 13, about 14, about 15, about 20, about 25, about 30, about 40, about 50, about 60, about 70, about 80, about 90, about 100 or more amino acid residues. In certain embodiments, the recombinant TCR is moditied from a naturally occurring TCR by at least one amino acid residue. In certain embodiments, the recombinant TCR is modified from a naturally occurring TCR by at least about 2, about 3, about 4, about 5, about 6, about 7, about 8, about 9, about 10, about 11, about 12, about 13, about 14, about 15, about 20, about 25, about 30, about 40, about 50, about 60, about 70, about 80, about 90, about 100 or more amino acid residues.

### 4.3.2. Chimeric Antigen Receptor (CAR)

In certain embodiments, the antigen-recognizing receptor is a CAR. CARs are engineered receptors, which graft or confer a specificity of interest onto an immune effector cell. CARs can be used to graft the specificity of a monoclonal antibody onto a T cell; with transfer of their coding sequence facilitated by retroviral vectors.

There are three generations of CARs. "First generation" CARs are typically composed of an extracellular antigen-binding domain (e.g., an scFv), which is fused to a transmembrane domain, which is fused to cytoplasmic/intracellular signaling domain. "First generation" CARs can provide *de novo* antigen recognition and cause activation of both CD4⁺ and CD8⁺ T cells through their CD3ζ chain signaling domain in a single fusion molecule, independent of HLA-mediated antigen presentation. "Second generation" CARs add intracellular signaling domains from various co-stimulatory molecules (e.g., CD28, 4-1BB, ICOS, OX40) to the cytoplasmic tail of the CAR to provide additional signals to the T cell. "Second generation" CARs comprise those that provide both co-stimulation (e.g., CD28 or 4-1BB) and activation (CD3ζ). "Third generation" CARs comprise those that provide multiple co-stimulation (*e.g.*, CD28 and 4-1BB) and activation (CD3ζ). In certain embodiments, the antigen-recognizing receptor is a first-generation CAR. In certain embodiments, the antigen-recognizing receptor is a CAR that does not comprise an intracellular signaling domain of a co-stimulatory molecule or a fragment thereof. In certain embodiments, the antigen-recognizing receptor is a second-generation CAR.

In certain embodiments, the CAR comprises an extracellular antigen-binding domain that specifically binds to uPAR, a transmembrane domain, and an intracellular signaling domain.

### 4.3.2.1. Extracellular Antigen-Binding Domain of A CAR

In certain embodiments, the extracellular antigen-binding domain is an scFv. In certain embodiments, the scFv is a human scFv. In certain embodiments, the scFv is a humanized scFv. In certain embodiments, the scFv is a murine scFv. In certain embodiments, the scFv is identified by screening scFv phage library with an antigen-Fc fusion protein.

In certain embodiments, the extracellular antigen-binding domain is a Fab. In certain embodiments, the Fab is crosslinked. In certain embodiments, the extracellular antigen-binding domain is a F(ab)₂.

Any of the foregoing molecules may be comprised in a fusion protein with a heterologous sequence to form the extracellular antigen-binding domain. In certain non-limiting embodiments, the extracellular antigen-binding domain of the CAR (embodied, for example, an scFv or an analog thereof) binds to uPAR (e.g., human uPAR) with a binding affinity, for example with a dissociation constant (K_{D}) of about 1 × 10⁻⁶ M or less, e.g., about 1 × 10⁻⁷ M or less, about 1 × 10⁻⁸ M or less, about 1 × 10⁻⁹ M or less, about 1 × 10⁻¹⁰ M or less, or about 1 × 10⁻¹¹ M or less.

Binding of the extracellular antigen-binding domain of the CAR can be confirmed by, for example, enzyme-linked immunosorbent assay (ELISA), radioimmunoassay (RIA), FACS analysis, bioassay (*e.g..* growth inhibition), or Western Blot assay. Each of these assays generally detect the presence of protein-antibody complexes of particular interest by employing a labeled reagent (*e.g.*, an antibody, or a scFv) specific for the complex of interest. For example, the scFv can be radioactively labeled and used in a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by such means as the use of a γ counter or a scintillation counter or by autoradiography. In certain embodiments, the uPAR-targeted extracellular antigen-binding domain is labeled with a fluorescent marker. Non-limiting examples of fluorescent markers include green fluorescent protein (GFP), blue fluorescent protein (e.g., EBFP, EBFP2, Azurite, and mKalama1), cyan fluorescent protein (e.g., ECFP, Cerulean, and CyPet), and yellow fluorescent protein (e.g., YFP, Citrine, Venus, and YPet). In certain embodiments, the uPAR-targeted human scFv is labeled with GFP.

In certain embodiments, the CDRs are identified according to the IMGT numbering system.

In certain embodiments, the extracellular antigen-binding domain of the CAR (e.g., an scFv) comprises a heavy chain variable region (V_{H}) comprising a CDR1 comprising the amino acid sequence set forth in SEQ ID NO: 1 or a conservative modification thereof, a CDR2 comprising the amino acid sequence set forth in SEQ ID NO: 2 or a conservative modification thereof, and a CDR3 comprising the amino acid sequence set forth in SEQ ID NO: 3 or a conservative modification thereof, SEQ ID NOs: 1-3 are provided in Table 1.

In certain embodiments, the extracellular antigen-binding domain of the CAR (e.g., an scFv) comprises a light chain variable region (V_{L}) comprising a CDR1 comprising the amino acid sequence set forth in SEQ ID NO: 4 or a conservative modification thereof, a CDR2 comprising the amino acid sequence set forth in SEQ ID NO: 5 or a conservative modification thereof, and a CDR3 comprising the amino acid sequence set forth in SEQ ID NO: 6 or a conservative modification thereof. SEQ ID NOs: 4-6 are provided in Table 1.

In certain embodiments, the extracellular antigen-binding domain of the CAR (e.g., an scFv) comprises a V_{H} comprising a CDR1 comprising the amino acid sequence set forth in SEQ ID NO: 1 or a conservative modification thereof, a CDR2 comprising the amino acid sequence set forth in SEQ ID NO: 2 or a conservative modification thereof, and a CDR3 comprising the amino acid sequence set forth in SEQ ID NO: 3 or a conservative modification thereof: and a V_{L} comprising a CDR1 comprising the amino acid sequence set forth in SEQ ID NO: 4 or a conservative modification thereof, a CDR2 comprising the amino acid sequence set forth in SEQ ID NO: 5 or a conservative modification, and a CDR3 comprising the amino acid sequence set forth in SEQ ID NO: 6 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; and a V_{L} comprising a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6.

In certain embodiments, the extracellular antigen-binding domain of the CAR (e.g., an scFv) comprises a V_{H} comprising an amino acid sequence that is at least about 80% (*e.g.*, at least about 85%, at least about 90%, or at least about 95%) homologous or identical to the amino sequence set forth in SEQ ID NO: 7. For example, the extracellular antigen-binding domain of the CAR (e.g., an scFv) comprises a V_{H} comprising an amino acid sequence that is about 80%, about 81%, about 82%, about 83%, about 84% about 85%, about 86%, about 87%, about 88%, about 89%, about 90% about 91%, about 92%, about 93%, about 94%, about 95%, about 96%, about 97%, about 98%, about 99% or about 100% homologous or identical to SEQ ID NO: 7. In certain embodiments, the extracellular antigen-binding domain comprises a V_{H} comprising the amino sequence set forth in SEQ ID NO: 7. SEQ ID NO: 7 is provided in Table 1 below.

In certain embodiments, the extracellular antigen-binding domain of the CAR (e.g., an scFv) comprises a V_{L} comprising an amino acid sequence that is at least about 80% (*e.g.,* at least about 85%, at least about 90%, or at least about 95%) homologous or identical to the amino sequence set forth in SEQ ID NO: 8. For example, the extracellular antigen-binding domain of the CAR (e.g., an scFv) comprises a V_{L} comprising an amino acid sequence that is about 80%, about 81%, about 82%, about 83% about 84% about 85%, about 86%, about 87%, about 88%, about 89%, about 90%, about 91%, about 92%, about 93%, about 94%. about 95%. about 96%, about 97%, about 98%, about 99% or about 100% homologous or identical to SEQ ID NO: 8. In certain embodiments, the extracellular antigen-binding domain comprises a V_{L} comprising the amino sequence set forth in SEQ ID NO: 8. SEQ ID NO: 8 is provided in Table 1 below.

In certain embodiments, the extracellular antigen-binding domain of the CAR (e.g., an scFv) comprises a V_{H} comprising the amino acid sequence set forth in SEQ ID NO: 7, and a V_{L} comprising the amino acid sequence set forth in SEQ ID NO: 8. In certain embodiments, the V_{H} and V_{L} are linked via a linker. In certain embodiments, the linker comprises the amino acid sequence set forth in SEQ ID NO: 110.

In certain embodiments. the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 9. In certain embodiments, the scFv is designated as "3-C3-A". An exemplary nucleotide sequence encoding the amino acid sequence of SEQ ID NO: 9 is set forth in SEQ ID NO: 10. SEQ ID NOS: 9 and 10 are provided in Table 1 below.

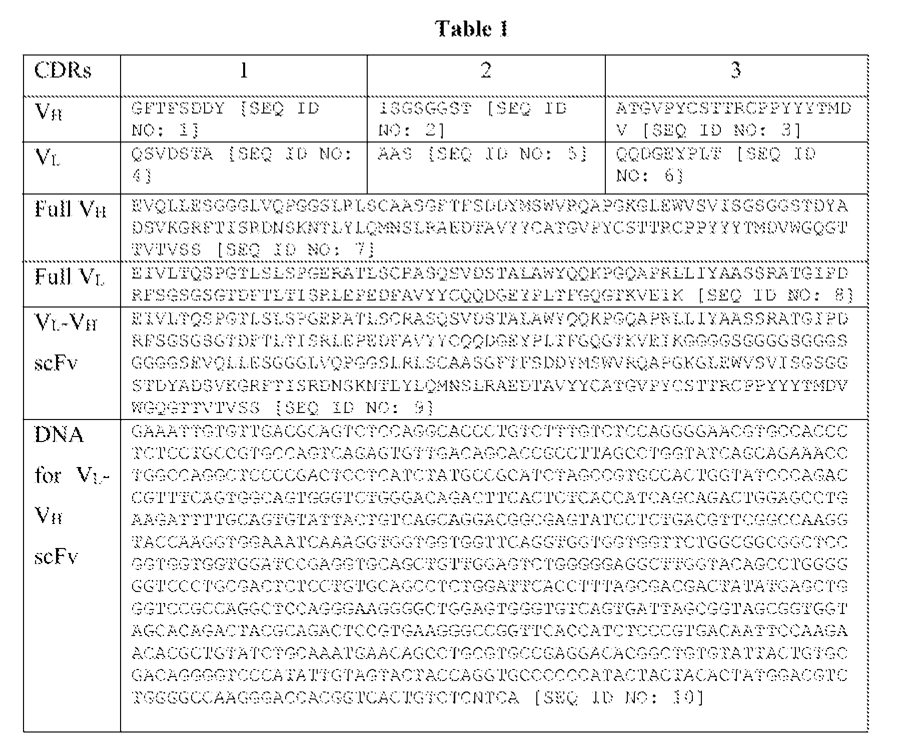

In certain embodiments, the extracellular antigen-binding domain of the CAR (e.g., an scFv) comprises a V_{H} comprising a CDR1 comprising the amino acid sequence set forth in SEQ ID NO: 11 or a conservative modification thereof, a CDR2 comprising the amino acid sequence set forth in SEQ ID NO: 2 or a conservative modification thereof, and a CDR3 comprising the amino acid sequence set forth in SEQ ID NO: 12 or a conservative modification thereof. SEQ ID NOs: 2, 11, and 12 are provided in Table 2.

In certain embodiments, the extracellular antigen-binding domain of the CAR (e.g., an scFv) comprises a V_{L} comprising a CDR1 comprising the amino acid sequence set forth in SEQ ID NO: 13 or a conservative modification thereof, a CDR2 comprising the amino acid sequence set forth in SEQ ID NO: 5 or a conservative modification thereof, and a CDR3 comprising the amino acid sequence set forth in SEQ ID NO: 14 or a conservative modification thereof. SEQ ID NOs: 5, 13, and 14 are provided in Table 2.

In certain embodiments, the extracellular antigen-binding domain of the CAR (e.g., an scFv) comprises a V_{H} comprising a CDR1 comprising the amino acid sequence set forth in SEQ ID NO: 11 or a conservative modification thereof, a CDR2 comprising the amino acid sequence set forth in SEQ ID NO: 2 or a conservative modification thereof, and a CDR3 comprising the amino acid sequence set forth in SEQ ID NO: 12 or a conservative modification thereof; and a V_{L} comprising a CDR1 comprising the amino acid sequence set forth in SEQ ID NO: 13 or a conservative modification thereof, a CDR2 comprising the amino acid sequence set forth in SEQ ID NO: 5 or a conservative modification thereof, and a CDR3 comprising the amino acid sequence set forth in SEQ ID NO: 14 or a conservative modification thereof.

In certain embodiments, the extracellular antigen-binding domain of the CAR (e.g., a scFv) comprises a V_{H} comprising a CDR1 comprising the amino acid sequence set forth in SEQ ID NO: 11, a CDR2 comprising the amino acid sequence set forth in SEQ ID NO: 2, and a CDR3 comprising the amino acid sequence set forth in SEQ ID NO: 12; and a V_{L} comprising a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14.

In certain embodiments, the extracellular antigen-binding domain of the CAR (e.g., an scFv) comprises a V_{H} comprising an amino acid sequence that is at least about 80% (*e.g.*, at least about 85%, at least about 90%, or at least about 95%) homologous or identical to the amino sequence set forth in SEQ ID NO: 15. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 15. In certain embodiments, the extracellular antigen-binding domain comprises a V_{H} comprising the amino sequence set forth in SEQ ID NO: 15. SEQ ID NO: 15 is provided in Table 2 below.

In certain embodiments, the extracellular antigen-binding domain of the CAR (e.g., an scFv) comprises a V_{L} comprising an amino acid sequence that is at least about 80% (*e.g.*, at least about 85%, at least about 90%, or at least about 95%) homologous or identical to the amino sequence set forth in SEQ ID NO: 16. For example, the extracellular antigen-binding domain of the CAR (e.g., an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 16. In certain embodiments, the extracellular antigen-binding domain comprises a V_{L} comprising the amino sequence set forth in SEQ ID NO: 16. SEQ ID NO: 16 is provided in Table 2 below.

In certain embodiments, the extracellular antigen-binding domain of the CAR (e.g., an scFv) comprises a V_{H} comprising the amino acid sequence set forth in SEQ ID NO: 15, and a V_{L} comprising the amino acid sequence set forth in SEQ ID NO: 16. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 17. In certain embodiments, the scFv is designated as "3-DS-A". An exemplary nucleotide sequence encoding the amino acid sequence of SEQ ID NO: 17 is set forth in SEQ ID NO: 18. SEQ ID NOS: 17 and 18 are provided in Table 2 below.

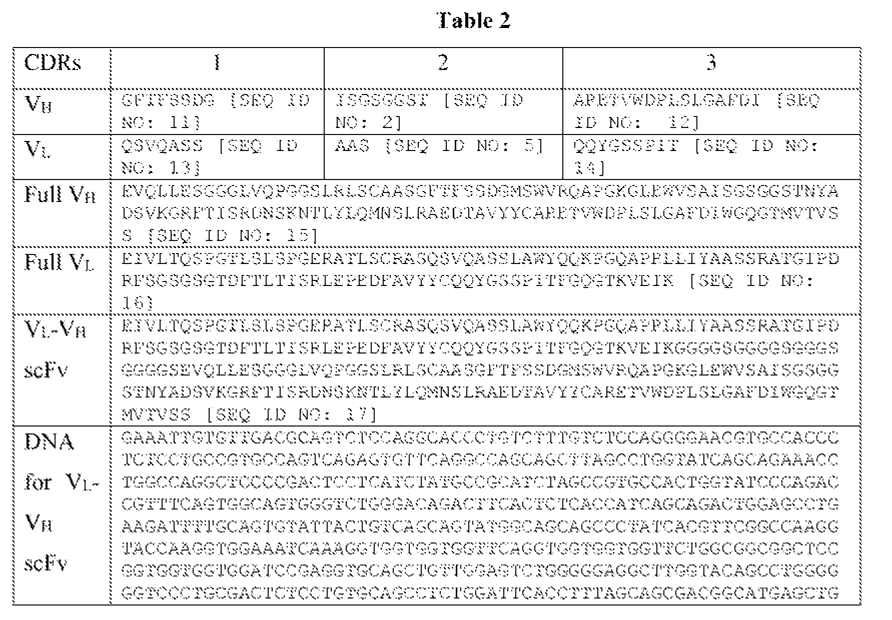

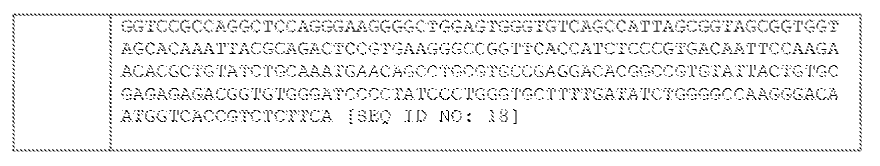

In certain embodiments, the extracellular antigen-binding domain of the CAR (e.g., an scFv) comprises a V_{H} comprising a CDR1 comprising the amino acid sequence set forth in SEQ ID NO: 19 or a conservative modification thereof, a CDR2 comprising the amino acid sequence set forth in SEQ ID NO: 2 or a conservative modification thereof, and a CDR3 comprising the amino acid sequence set forth in SEQ ID NO: 20 or a conservative modification thereof. SEQ ID NOS: 2, 19, and 10 are provided in Table 3.

In certain embodiments, the extracellular antigen-binding domain of the CAR (e.g., an scFv) comprises a V_{L} comprising a CDR1 comprising the amino acid sequence set forth in SEQ ID NO: 21 or a conservative modification thereof, a CDR2 comprising the amino acid sequence set forth in SEQ ID NO: 5 or a conservative modification thereof, and a CDR3 comprising the amino acid sequence set forth in SEQ ID NO: 22 or a conservative modification thereof. SEQ ID NOs: 5, 21, and 22 are provided in Table 3.

In certain embodiments, the extracellular antigen-binding domain of the CAR (e.g., an scFv) comprises a V_{H} comprising a CDR1 comprising the amino acid sequence set forth in SEQ ID NO: 19 or a conservative modification thereof, a CDR2 comprising the amino acid sequence set forth in SEQ ID NO: 2 or a conservative modification thereof, and a CDR3 comprising the amino acid sequence set forth in SEQ ID NO: 20 or a conservative modification thereof; and a V_{L} comprising a CDR1 comprising the amino acid sequence set forth in SEQ ID NO: 21 or a conservative modification thereof, a CDR2 comprising the amino acid sequence set forth in SEQ ID NO: 5 or a conservative modification thereof, and a CDR3 comprising the amino acid sequence set forth in SEQ ID NO: 22 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; and a V_{L} comprising a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22.

In certain embodiments, the extracellular antigen-binding domain of the CAR (e.g., an scFv) comprises a V_{H} comprising an amino acid sequence that is at least about 80% (*e.g.,* at least about 85%, at least about 90%, or at least about 95%) homologous or identical to the amino sequence set forth in SEQ ID NO: 23. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 23. In certain embodiments, the extracellular antigen-binding domain comprises a V_{H} comprising the amino sequence set forth in SEQ ID NO: 23. SEQ ID NO: 23 is provided in Table 3 below.

In certain embodiments, the extracellular antigen-binding domain of the CAR (e.g., an scFv) comprises a V_{L} comprising an amino acid sequence that is at least about 80% (*e.g.*, at least about 85%, at least about 90%, or at least about 95%) homologous or identical to the amino sequence set forth in SEQ ID NO: 24. For example, the extracellular antigen-binding domain of the CAR (e.g.. an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 24. In certain embodiments, the extracellular antigen-binding domain comprises a V_{L} comprising the amino sequence set forth in SEQ ID NO: 24. SEQ ID NO: 24 is provided in Table 3 below.

In certain embodiments, the extracellular antigen-binding domain of the CAR (e.g., an scFv) comprises a V_{H} comprising the amino acid sequence set forth in SEQ ID NO: 23, and a V_{L} comprising the amino acid sequence set forth in SEQ ID NO: 24. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 25. In certain embodiments, the scFv is designated as "3-G1-A". An exemplary nucleotide sequence encoding the amino acid sequence of SEQ ID NO: 25 is set forth in SEQ ID NO: 26. SEQ ID NOS: 25 and 26 are provided in Table 3 below.

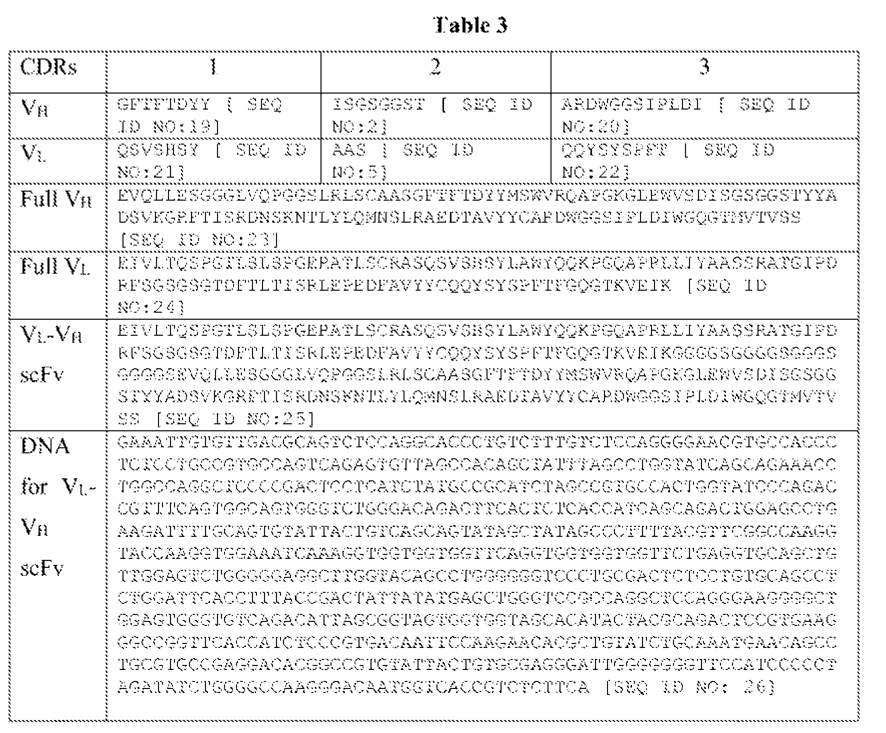

In certain embodiments, the extracellular antigen-binding domain of the CAR (e.g., an scFv) comprises a V_{H} comprising a CDR1 comprising the amino acid sequence set forth in SEQ ID NO: 19 or a conservative modification thereof, a CDR2 comprising the amino acid sequence set forth in SEQ ID NO: 27 or a conservative modification thereof, and a CDR3 comprising the amino acid sequence set forth in SEQ ID NO: 28 or a conservative modification thereof. SEQ ID NOs: 19, 27, and 28 are provided in Table 4.

In certain embodiments, the extracellular antigen-binding domain of the CAR (e.g., an scFv) comprises a V_{L} comprising a CDR1 comprising the amino acid sequence set forth in SEQ ID NO: 29 or a conservative modification thereof, a CDR2 comprising the amino acid sequence set forth in SEQ ID NO: 5 or a conservative modification thereof, and a CDR3 comprising the amino acid sequence set forth in SEQ ID NO: 30 or a conservative modification thereof. SEQ ID NOs: 5, 29, and 30 are provided in Table 4.

In certain embodiments, the extracellular antigen-binding domain of the CAR (e.g., an scFv) comprises a V_{H} comprising a CDR1 comprising the amino acid sequence set forth in SEQ ID NO: 19 or a conservative modification thereof, a CDR2 comprising the amino acid sequence set forth in SEQ ID NO: 27 or a conservative modification thereof, and a CDR3 comprising the amino acid sequence set forth in SEQ ID NO: 28 or a conservative modification thereof; and a V_{L} comprising a CDR1 comprising the amino acid sequence set forth in SEQ ID NO: 29 or a conservative modification thereof, a CDR2 comprising the amino acid sequence set forth in SEQ ID NO: 5 or a conservative modification thereof, and a CDR3 comprising the amino acid sequence set forth in SEQ ID NO: 30 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28; and a V_{L} comprising a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30.

In certain embodiments, the extracellular antigen-binding domain of the CAR (e.g., an scFv) comprises a V_{H} comprising an amino acid sequence that is at least about 80% (*e.g.,* at least about 85%, at least about 90%, or at least about 95%) homologous or identical to the amino sequence set forth in SEQ ID NO: 31. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 31. In certain embodiments, the extracellular antigen-binding domain comprises a V_{H} comprising the amino sequence set forth in SEQ ID NO: 31. SEQ ID NO: 31 is provided in Table 4 below.

In certain embodiments, the extracellular antigen-binding domain of the CAR (e.g., an scFv) comprises a V_{L} comprising an amino acid sequence that is at least about 80% (e.g., at least about 85%, at least about 90%, or at least about 95%) homologous or identical to the amino sequence set forth in SEQ ID NO: 32. For example, the extracellular antigen-binding domain of the CAR (e.g., an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 32. In certain embodiments, the extracellular antigen-binding domain comprises a V_{L} comprising the amino sequence set forth in SEQ ID NO: 32. SEQ ID NO: 32 is provided in Table 4 below.

In certain embodiments, the extracellular antigen-binding domain of the CAR (e.g., an scFv) comprises a V_{H} comprising the amino acid sequence set forth in SEQ ID NO: 31, and a V_{L} comprising the amino acid sequence set forth in SEQ ID NO: 32. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 33. In certain embodiments, the scFv is designated as "3-H4-A". An exemplary nucleotide sequence encoding the amino acid sequence of SEQ ID NO: 33 is set forth in SEQ ID NO: 34. SEQ ID NOS: 33 and 34 are provided in Table 4 below.

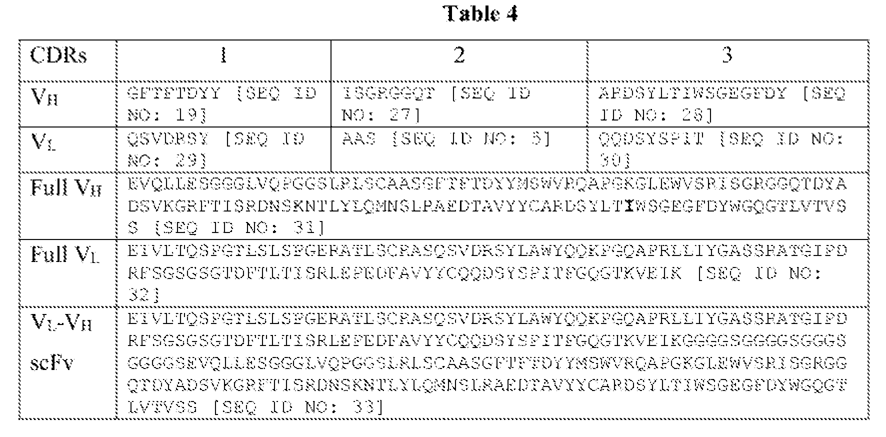

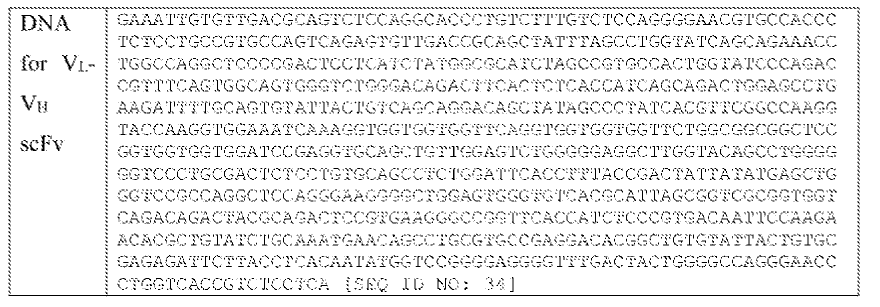

In certain embodiments, the extracellular antigen-binding domain of the CAR (e.g., an scFv) comprises a V_{H} comprising a CDR1 comprising the amino acid sequence set forth in SEQ ID NO: 35 or a conservative modification thereof, a CDR2 comprising the amino acid sequence set forth in SEQ ID NO: 36 or a conservative modification thereof, and a CDR3 comprising the amino acid sequence set forth in SEQ ID NO: 37 or a conservative modification thereof. SEQ ID NOs: 35-37 are provided in Table 5.

In certain embodiments, the extracellular antigen-binding domain of the CAR (e.g., an scFv) comprises a V_{L} comprising a CDR1 comprising the amino acid sequence set forth in SEQ ID NO: 38 or a conservative modification thereof, a CDR2 comprising the amino acid sequence set forth in SEQ ID NO: 39 or a conservative modification thereof, and a CDR3 comprising the amino acid sequence set forth in SEQ ID NO: 40 or a conservative modification thereof. SEQ ID NOs: 38-40 are provided in Table 5.

In certain embodiments, the extracellular antigen-binding domain of the CAR (e.g., an scFv) comprises a V_{H} comprising a CDR1 comprising the amino acid sequence set forth in SEQ ID NO: 35 or a conservative modification thereof, a CDR2 comprising the amino acid sequence set forth in SEQ ID NO: 36 or a conservative modification thereof, and a CDR3 comprising the amino acid sequence set forth in SEQ ID NO: 37 or a conservative modification thereof; and a V_{L} comprising a CDR1 comprising the amino acid sequence set forth in SEQ ID NO: 38 or a conservative modification thereof, a CDR2 comprising the amino acid sequence set forth in SEQ ID NO: 39 or a conservative modification thereof, and a CDR3 comprising the amino acid sequence set forth in SEQ ID NO: 40 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; and a V_{L} comprising a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40.

In certain embodiments, the extracellular antigen-binding domain of the CAR (e.g., an scFv) comprises a V_{H} comprising an amino acid sequence that is at least about 80% (*e.g.*, at least about 85%, at least about 90%, or at least about 95%) homologous or identical to the amino sequence set forth in SEQ ID NO: 41. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 41. In certain embodiments, the extracellular antigen-binding domain comprises a V_{H} comprising the amino sequence set forth in SEQ ID NO: 41. SEQ ID NO: 41 is provided in Table 5 below.

In certain embodiments, the extracellular antigen-binding domain of the CAR (e.g., an scFv) comprises a V_{L} comprising an amino acid sequence that is at least about 80% (*e.g.*, at least about 85%, at least about 90%, or at least about 95%) homologous or identical to the amino sequence set forth in SEQ ID NO: 42. For example, the extracellular antigen-binding domain of the CAR (e.g., an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 42. In certain embodiments, the extracellular antigen-binding domain comprises a V_{L} comprising the amino sequence set forth in SEQ ID NO: 42. SEQ ID NO: 42 is provided in Table 5 below.

In certain embodiments, the extracellular antigen-binding domain of the CAR (e.g., an scFv) comprises a V_{H} comprising the amino acid sequence set forth in SEQ ID NO: 41, and a V_{L} comprising the amino acid sequence set forth in SEQ ID NO: 42. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 43. In certain embodiments, the scFv is designated as "4-F5-A". An exemplary nucleotide sequence encoding the amino acid sequence of SEQ ID NO: 43 is set forth in SEQ ID NO: 44. SEQ ID NOS: 43 and 44 are provided in Table 5 below.

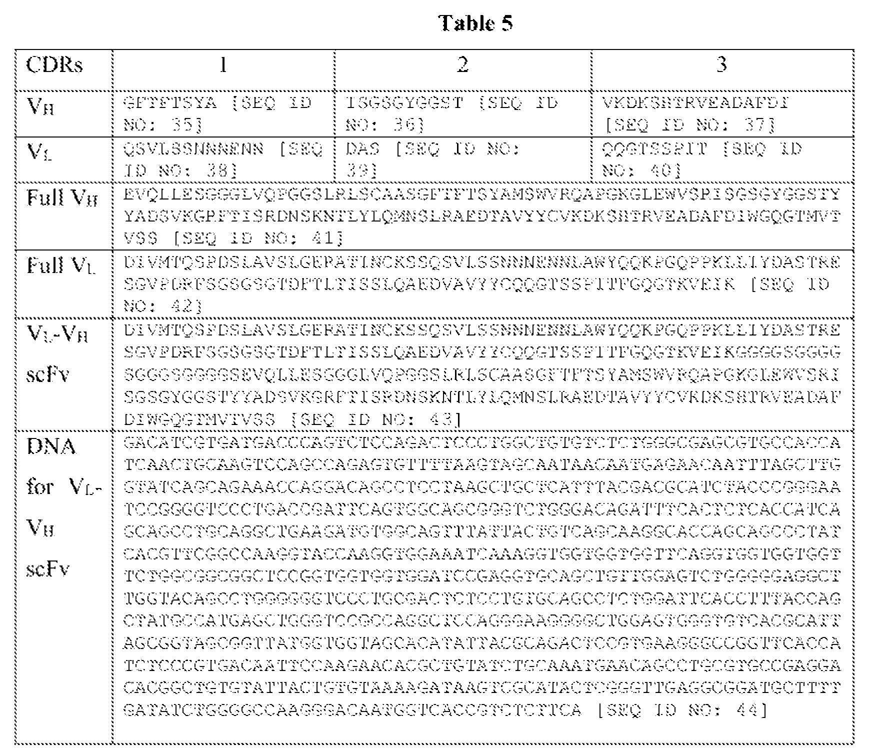

In certain embodiments, the extracellular antigen-binding domain of the CAR (e.g., an scFv) comprises a V_{H} comprising a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 47 or a conservative modification thereof. SEQ ID NOs: 45-47 are provided in Table 6.

In certain embodiments, the extracellular antigen-binding domain of the CAR (e.g., an scFv) comprises a V_{L} comprising a CDR1 comprising the amino acid sequence set forth in SEQ ID NO: 48 or a conservative modification thereof, a CDR2 comprising the amino acid sequence set forth in SEQ ID NO: 5 or a conservative modification thereof, and a CDR3 comprising the amino acid sequence set forth in SEQ ID NO: 49 or a conservative modification thereof. SEQ ID NOs: 5, 48, and 49 are provided in Table 6.

In certain embodiments, the extracellular antigen-binding domain of the CAR (e.g., an scFv) comprises a V_{H} comprising a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 47 or a conservative modification thereof; and a V_{L} comprising a CDR1 comprising the amino acid sequence set forth in SEQ ID NO: 48 or a conservative modification thereof, a CDR2 comprising the amino acid sequence set forth in SEQ ID NO: 5 or a conservative modification thereof, and a CDR3 comprising the amino acid sequence set forth in SEQ ID NO: 49 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; and a V_{L} comprising a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49.

In certain embodiments, the extracellular antigen-binding domain of the CAR (e.g., an scFv) comprises a V_{H} comprising an amino acid sequence that is at least about 80% (*e.g.,* at least about 85%, at least about 90%, or at least about 95%) homologous or identical to the amino sequence set forth in SEQ ID NO: 50. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 50. In certain embodiments, the extracellular antigen-binding domain comprises a V_{H} comprising the amino sequence set forth in SEQ ID NO: 50. SEQ ID NO: 50 is provided in Table 6 below.

In certain embodiments, the extracellular antigen-binding domain of the CAR (e.g., an scFv) comprises a V_{L} comprising an amino acid sequence that is at least about 80% (*e.g.,* at least about 85%, at least about 90%, or at least about 95%) homologous or identical to the amino sequence set forth in SEQ ID NO: 51. For example, the extracellular antigen-binding domain of the CAR (e.g., an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 51. In certain embodiments, the extracellular antigen-binding domain comprises a V_{L} comprising the amino sequence set forth in SEQ ID NO: 51. SEQ ID NO: 51 is provided in Table 6 below.

In certain embodiments, the extracellular antigen-binding domain of the CAR (e.g., an scFv) comprises a V_{H} comprising the amino acid sequence set forth in SEQ ID NO: 50, and a V_{L} comprising the amino acid sequence set forth in SEQ ID NO: 51. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 52. In certain embodiments, the scFv is designated as "4-F12-A". An exemplary nucleotide sequence encoding the amino acid sequence of SEQ ID NO: 52 is set forth in SEQ ID NO: 53. SEQ ID NOS: 52 and 53 are provided in Table 6 below.

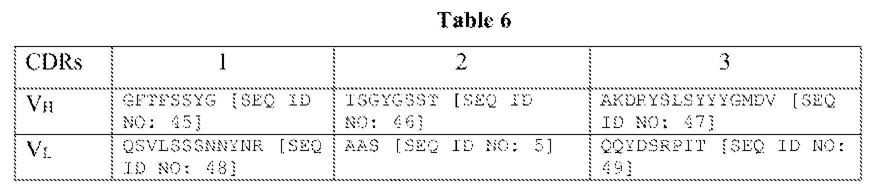

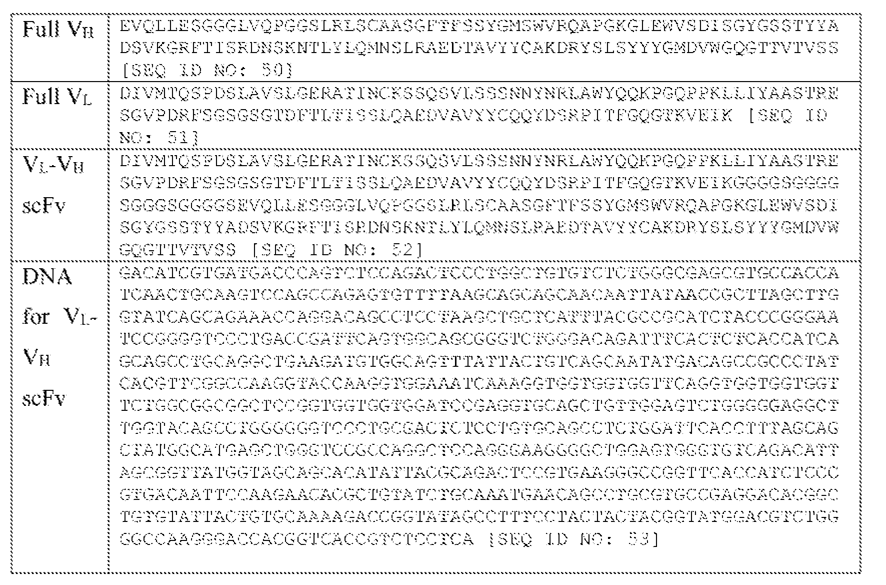

In certain embodiments, the extracellular antigen-binding domain of the CAR (e.g., an scFv) comprises a V_{H} comprising a CDR1 comprising the amino acid sequence set forth in SEQ ID NO: 54 or a conservative modification thereof, a CDR2 comprising the amino acid sequence set forth in SEQ ID NO: 55 or a conservative modification thereof, and a CDR3 comprising the amino acid sequence set forth in SEQ ID NO: 56 or a conservative modification thereof. SEQ ID NOs: 54-56 are provided in Table 7.

In certain embodiments, the extracellular antigen-binding domain of the CAR (e.g., an scFv) comprises a V_{L} comprising a CDR1 comprising the amino acid sequence set forth in SEQ ID NO: 57 or a conservative modification thereof, a CDR2 comprising the amino acid sequence set forth in SEQ ID NO: 5 or a conservative modification thereof, and a CDR3 comprising the amino acid sequence set forth in SEQ ID NO: 58 or a conservative modification thereof. SEQ ID NOs: 5, 57, and 58 are provided in Table 7.

In certain embodiments, the extracellular antigen-binding domain of the CAR (e.g., an scFv) comprises a V_{H} comprising a CDR1 comprising the amino acid sequence set forth in SEQ ID NO: 54 or a conservative modification thereof, a CDR2 comprising the amino acid sequence set forth in SEQ ID NO: 55 or a conservative modification thereof, and a CDR3 comprising the amino acid sequence set forth in SEQ ID NO: 56 or a conservative modification thereof; and a V_{L} comprising a CDR1 comprising the amino acid sequence set forth in SEQ ID NO: 57 or a conservative modification thereof, a CDR2 comprising the amino acid sequence set forth in SEQ ID NO: 5 or a conservative modification thereof, and a CDR3 comprising the amino acid sequence set forth in SEQ ID NO: 58 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 54, a CDR2 comprising the amino acid sequence set forth in SEQ ID NO: 55, and a CDR3 comprising the amino acid sequence set forth in SEQ ID NO: 56; and a V_{L} comprising a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58.

In certain embodiments, the extracellular antigen-binding domain of the CAR (e.g., an scFv) comprises a V_{H} comprising an amino acid sequence that is at least about 80% (*e.g.,* at least about 85%, at least about 90%, or at least about 95%) homologous or identical to the amino sequence set forth in SEQ ID NO: 59. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 59. In certain embodiments, the extracellular antigen-binding domain comprises a V_{H} comprising the amino sequence set forth in SEQ ID NO: 59. SEQ ID NO: 59 is provided in Table 7 below.

In certain embodiments, the extracellular antigen-binding domain of the CAR (e.g., an scFv) comprises a V_{L} comprising an amino acid sequence that is at least about 80% (*e.g.*, at least about 85%, at least about 90%, or at least about 95%) homologous or identical to the amino sequence set forth in SEQ ID NO: 60. For example, the extracellular antigen-binding domain of the CAR (e.g., an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 60. In certain embodiments, the extracellular antigen-binding domain comprises a V_{L} comprising the amino sequence set forth in SEQ ID NO: 60. SEQ ID NO: 60 is provided in Table 7 below.

In certain embodiments, the extracellular antigen-binding domain of the CAR (e.g., an scFv) comprises a V_{H} comprising the amino acid sequence set forth in SEQ ID NO: 59, and a V_{L} comprising the amino acid sequence set forth in SEQ ID NO: 60. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 61. In certain embodiments, the scFv is designated as "4-A5-B". An exemplary nucleotide sequence encoding the amino acid sequence of SEQ ID NO: 61 is set forth in SEQ ID NO: 62. SEQ ID NOS: 61 and 62 are provided in Table 7 below.

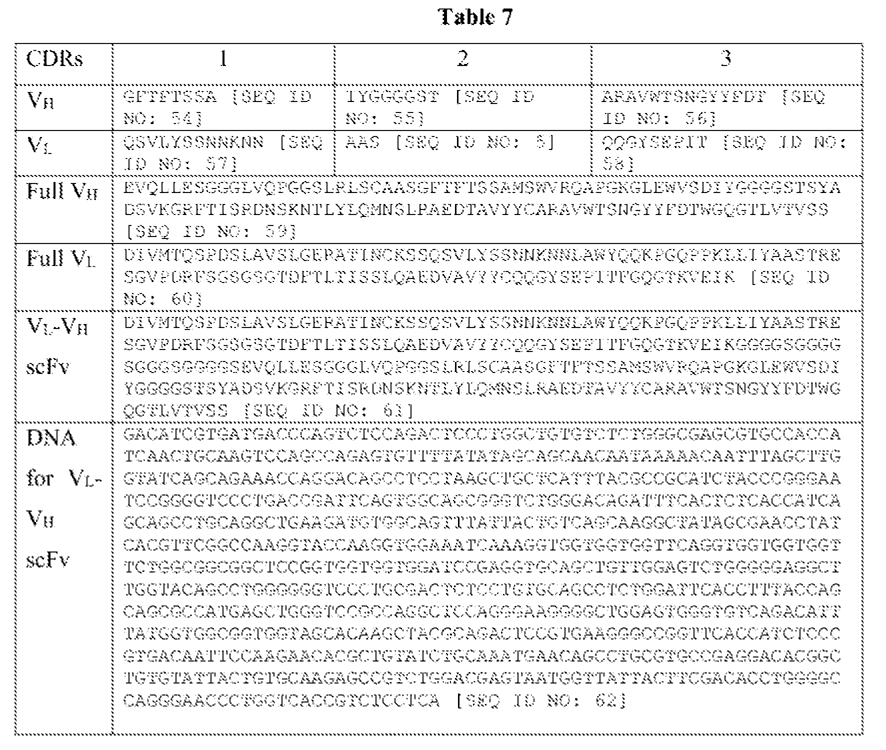

In certain embodiments, the extracellular antigen-binding domain of the CAR (e.g., an scFv) comprises a V_{H} comprising a CDR1 comprising the amino acid sequence set forth in SEQ ID NO: 63 or a conservative modification thereof, a CDR2 comprising the amino acid sequence set forth in SEQ ID NO: 64 or a conservative modification thereof, and a CDR3 comprising the amino acid sequence set forth in SEQ ID NO: 65 or a conservative modification thereof. SEQ ID NOs: 63-65 are provided in Table 8.

In certain embodiments, the extracellular antigen-binding domain of the CAR (e.g., an scFv) comprises a V_{L} comprising a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 68 or a conservative modification thereof. SEQ ID NOs: 66-68 are provided in Table 8.

In certain embodiments, the extracellular antigen-binding domain of the CAR (e.g., an scFv) comprises a V_{H} comprising a CDR1 comprising the amino acid sequence set forth in SEQ ID NO: 63 or a conservative modification thereof, a CDR2 comprising the amino acid sequence set forth in SEQ ID NO: 64 or a conservative modification thereof, and a CDR3 comprising the amino acid sequence set forth in SEQ ID NO: 65 or a conservative modification thereof; and a V_{L} comprising a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 68 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65; and a V_{L} comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68.

In certain embodiments, the extracellular antigen-binding domain of the CAR (e.g., an scFv) comprises a V_{H} comprising an amino acid sequence that is at least about 80% (*e.g.*, at least about 85%, at least about 90%, or at least about 95%) homologous or identical to the amino sequence set forth in SEQ ID NO: 69. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 69. In certain embodiments, the extracellular antigen-binding domain comprises a V_{H} comprising the amino sequence set forth in SEQ ID NO: 69. SEQ ID NO: 69 is provided in Table 8 below.

In certain embodiments, the extracellular antigen-binding domain of the CAR (e.g., an scFv) comprises a V_{L} comprising an amino acid sequence that is at least about 80% (e.g., at least about 85%, at least about 90%, or at least about 95%) homologous or identical to the amino sequence set forth in SEQ ID NO: 70. For example, the extracellular antigen-binding domain of the CAR (e.g.. an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 70. In certain embodiments, the extracellular antigen-binding domain comprises a V_{L} comprising the amino sequence set forth in SEQ ID NO: 70. SEQ ID NO: 70 is provided in Table 8 below.

In certain embodiments, the extracellular antigen-binding domain of the CAR (e.g.. an scFv) comprises a V_{H} comprising the amino acid sequence set forth in SEQ ID NO: 69, and a V_{L} comprising the amino acid sequence set forth in SEQ ID NO: 70. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 71. In certain embodiments, the scFv is designated as "05G9". An exemplary nucleotide sequence encoding the amino acid sequence of SEQ ID NO: 71 is set forth in SEQ ID NO: 72. SEQ ID NOS: 71 and 72 are provided in Table 8 below.

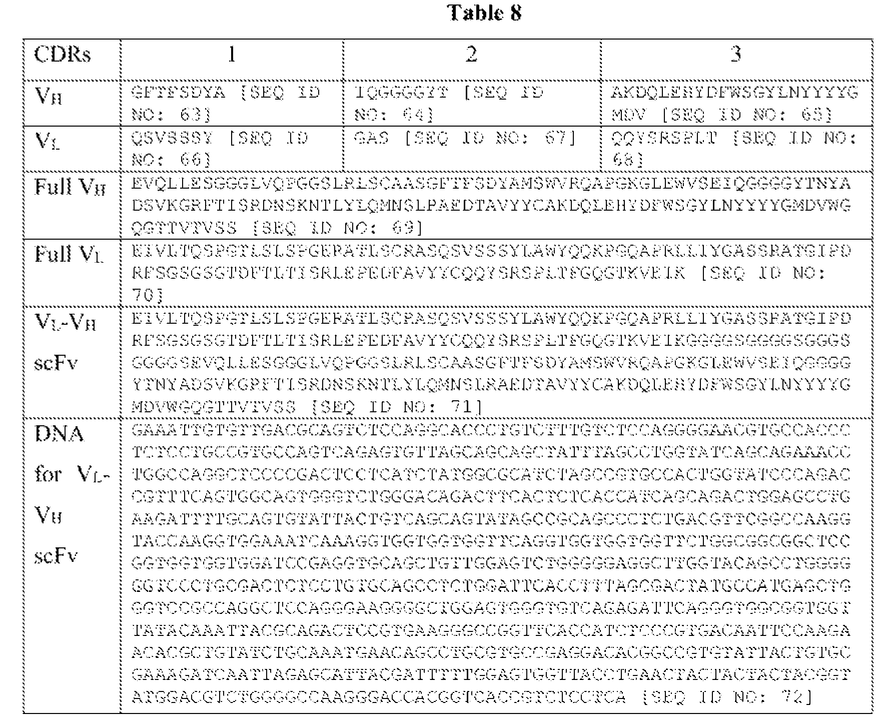

In certain embodiments, the extracellular antigen-binding domain of the CAR (e.g., an scFv) comprises a V_{H} comprising a CDR1 comprising the amino acid sequence set forth in SEQ ID NO: 73 or a conservative modification thereof, a CDR2 comprising the amino acid sequence set forth in SEQ ID NO: 74 or a conservative modification thereof, and a CDR3 comprising the amino acid sequence set forth in SEQ ID NO: 75 or a conservative modification thereof. SEQ ID NOs: 73-75 are provided in Table 9.

In certain embodiments, the extracellular antigen-binding domain of the CAR (e.g., an scFv) comprises a V_{L} comprising a CDR1 comprising the amino acid sequence set forth in SEQ ID NO: 76 or a conservative modification thereof, a CDR2 comprising the amino acid sequence set forth in SEQ ID NO: 67 or a conservative modification thereof, and a CDR3 comprising the amino acid sequence set forth in SEQ ID NO: 77 or a conservative modification thereof. SEQ ID NOs: 67, 76, and 77 are provided in Table 9.

In certain embodiments, the extracellular antigen-binding domain of the CAR (e.g., an scFv) comprises a V_{H} comprising a CDR1 comprising the amino acid sequence set forth in SEQ ID NO: 73 or a conservative modification thereof, a CDR2 comprising the amino acid sequence set forth in SEQ ID NO: 74 or a conservative modification thereof, and a CDR3 comprising the amino acid sequence set forth in SEQ ID NO: 75 or a conservative modification thereof; and a V_{L} comprising a CDR1 comprising the amino acid sequence set forth in SEQ ID NO: 76 or a conservative modification thereof, a CDR2 comprising the amino acid sequence set forth in SEQ ID NO: 67 or a conservative modification thereof, and a CDR3 comprising the amino acid sequence set forth in SEQ ID NO: 77 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75: and a V_{L} comprising a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77.

In certain embodiments, the extracellular antigen-binding domain of the CAR (e.g., an scFv) comprises a V_{H} comprising an amino acid sequence that is at least about 80% *(e.g.,* at least about 85%, at least about 90%, or at least about 95%) homologous or identical to the amino sequence set forth in SEQ ID NO: 78. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 78. In certain embodiments, the extracellular antigen-binding domain comprises a V_{H} comprising the amino sequence set forth in SEQ ID NO: 78. SEQ ID NO: 78 is provided in Table 9 below.

In certain embodiments, the extracellular antigen-binding domain of the CAR (e.g., an scFv) comprises a V_{L} comprising an amino acid sequence that is at least about 80% (*e.g.,* at least about 85%, at least about 90%, or at least about 95%) homologous or identical to the amino sequence set forth in SEQ ID NO: 79. For example, the extracellular antigen-binding domain of the CAR (e.g., an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 79. In certain embodiments, the extracellular antigen-binding domain comprises a V_{L} comprising the amino sequence set forth in SEQ ID NO: 79. SEQ ID NO: 79 is provided in Table 9 below.

In certain embodiments, the extracellular antigen-binding domain of the CAR (e.g., an scFv) comprises a V_{H} comprising the amino acid sequence set forth in SEQ ID NO: 78, and a V_{L} comprising the amino acid sequence set forth in SEQ ID NO: 79. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 116. In certain embodiments, the scFv is designated as "05A6". An exemplary nucleotide sequence encoding the amino acid sequence of SEQ ID NO: 80 is set forth in SEQ ID NO: 81. SEQ ID NOS: 80 and 81 are provided in Table 9 below.

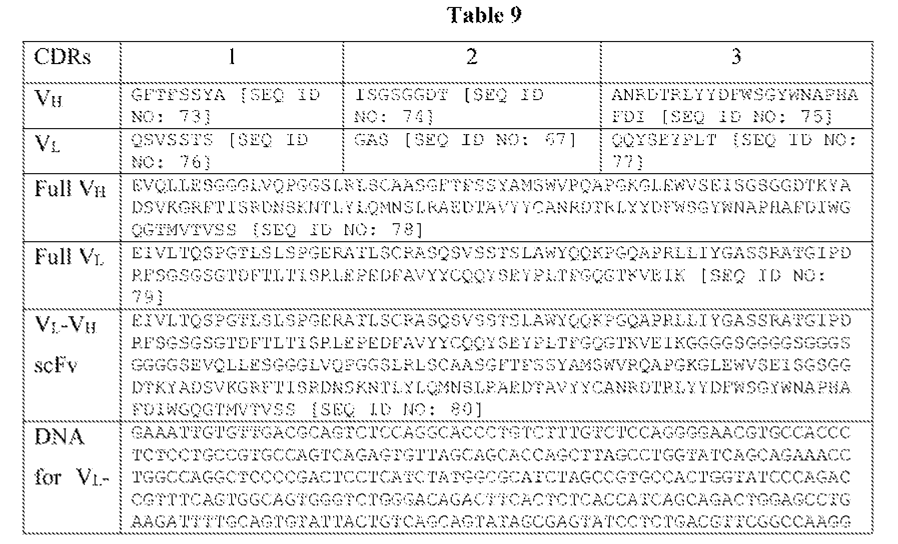

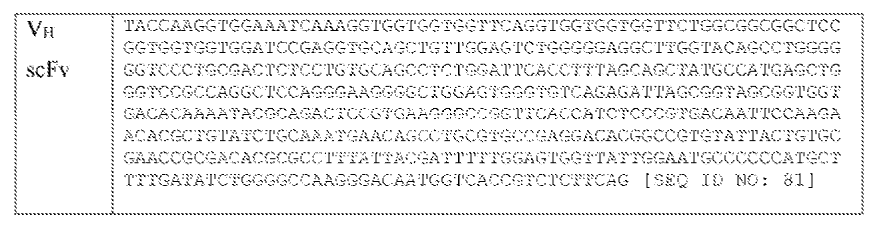

In certain embodiments, the extracellular antigen-binding domain of the CAR (e.g., an scFv) comprises a V_{H} comprising a CDR1 comprising the amino acid sequence set forth in SEQ ID NO: 45 or a conservative modification thereof, a CDR2 comprising the amino acid sequence set forth in SEQ ID NO: 82 or a conservative modification thereof, and a CDR3 comprising the amino acid sequence set forth in SEQ ID NO: 83 or a conservative modification thereof. SEQ ID NOs: 45, 82, and 83 are provided in Table 10.

In certain embodiments, the extracellular antigen-binding domain of the CAR (e.g., an scFv) comprises a V_{L} comprising a CDR1 comprising the amino acid sequence set forth in SEQ ID NO: 84 or a conservative modification thereof, a CDR2 comprising the amino acid sequence set forth in SEQ ID NO: 67 or a conservative modification thereof, and a CDR3 comprising the amino acid sequence set forth in SEQ ID NO: 85 or a conservative modification thereof. SEQ ID NOs: 67, 84, and 85 are provided in Table 10.

In certain embodiments, the extracellular antigen-binding domain of the CAR (e.g., an scFv) comprises a V_{H} comprising a CDR1 comprising the amino acid sequence set forth in SEQ ID NO: 45 or a conservative modification thereof, a CDR2 comprising the amino acid sequence set forth in SEQ ID NO: 82 or a conservative modification thereof, and a CDR3 comprising the amino acid sequence set forth in SEQ ID NO: 83 or a conservative modification thereof; and a V_{L} comprising a CDR1 comprising the amino acid sequence set forth in SEQ ID NO: 84 or a conservative modification thereof, a CDR2 comprising the amino acid sequence set forth in SEQ ID NO: 67 or a conservative modification thereof, and a CDR3 comprising the amino acid sequence set forth in SEQ ID NO: 85 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83; and a V_{L} comprising a CDR1 comprising the amino acid sequence set forth in SEQ ID NO: 84, a CDR2 comprising the amino acid sequence set forth in SEQ ID NO: 67, and a CDR3 comprising the amino acid sequence set forth in SEQ ID NO: 85.

In certain embodiments, the extracellular antigen-binding domain of the CAR (e.g., an scFv) comprises a V_{H} comprising an amino acid sequence that is at least about 80% *(e.g.,* at least about 85%, at least about 90%, or at least about 95%) homologous or identical to the amino sequence set forth in SEQ ID NO: 86. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 86. In certain embodiments, the extracellular antigen-binding domain comprises a V_{H} comprising the amino sequence set forth in SEQ ID NO: 86. SEQ ID NO: 86 is provided in Table 10 below.

In certain embodiments, the extracellular antigen-binding domain of the CAR (e.g., an scFv) comprises a V_{L} comprising an amino acid sequence that is at least about 80% (e.g., at least about 85%, at least about 90%, or at least about 95%) homologous or identical to the amino sequence set forth in SEQ ID NO: 87. For example, the extracellular antigen-binding domain of the CAR (e.g.. an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 87. In certain embodiments, the extracellular antigen-binding domain comprises a V_{L} comprising the amino sequence set forth in SEQ ID NO: 87. SEQ ID NO: 87 is provided in Table 10 below.

In certain embodiments, the extracellular antigen-binding domain of the CAR (e.g., an scFv) comprises a V_{H} comprising the amino acid sequence set forth in SEQ ID NO: 86, and a V_{L} comprising the amino acid sequence set forth in SEQ ID NO: 87. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 88. In certain embodiments, the scFv is designated as "05B2". An exemplary nucleotide sequence encoding the amino acid sequence of SEQ ID NO: 88 is set forth in SEQ ID NO: 89. SEQ ID NOS: 88 and 89 are provided in Table 10 below.

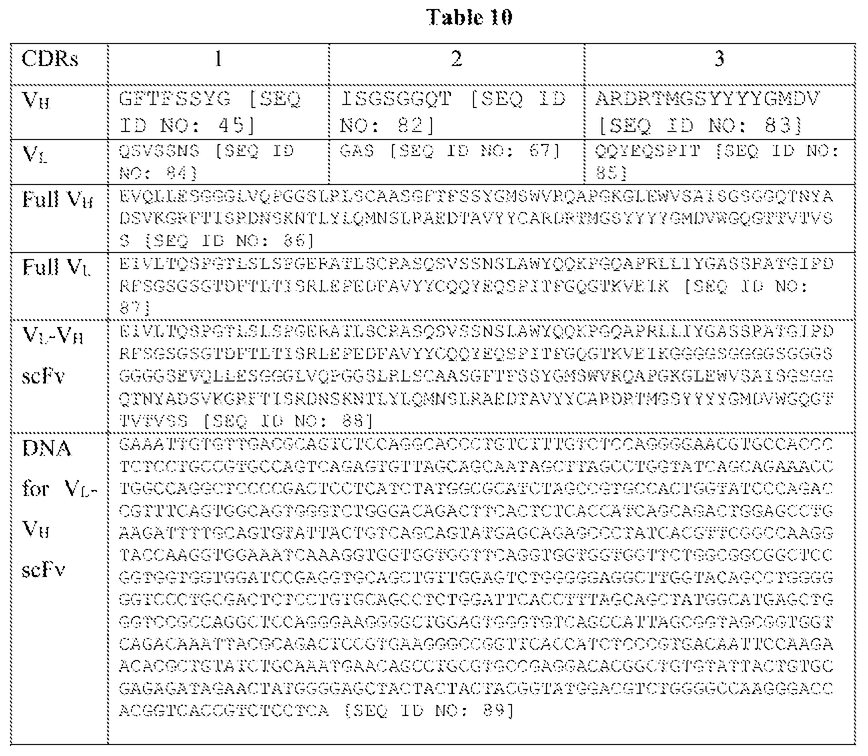

In certain embodiments, the extracellular antigen-binding domain of the CAR (e.g., an scFv) comprises a V_{H} comprising a CDR1 comprising the amino acid sequence set forth in SEQ ID NO: 90 or a conservative modification thereof, a CDR2 comprising the amino acid sequence set forth in SEQ ID NO: 91 or a conservative modification thereof, and a CDR3 comprising the amino acid sequence set forth in SEQ ID NO: 92 or a conservative modification thereof. SEQ ID NOs: 90-92 are provided in Table 11.

In certain embodiments, the extracellular antigen-binding domain of the CAR (e.g., an scFv) comprises a V_{L} comprising a CDR1 comprising the amino acid sequence set forth in SEQ ID NO: 93 or a conservative modification thereof, a CDR2 comprising the amino acid sequence set forth in SEQ ID NO: 67 or a conservative modification thereof, and a CDR3 comprising the amino acid sequence set forth in SEQ ID NO: 94 or a conservative modification thereof. SEQ ID NOs: 67, 93, and 94 are provided in Table 11.

In certain embodiments, the extracellular antigen-binding domain of the CAR (e.g., an scFv) comprises a V_{H} comprising a CDR1 comprising the amino acid sequence set forth in SEQ ID NO: 90 or a conservative modification thereof, a CDR2 comprising the amino acid sequence set forth in SEQ ID NO: 91 or a conservative modification thereof, and a CDR3 comprising the amino acid sequence set forth in SEQ ID NO: 92 or a conservative modification thereof; and a V_{L} comprising a CDR1 comprising the amino acid sequence set forth in SEQ ID NO: 93 or a conservative modification thereof, a CDR2 comprising the amino acid sequence set forth in SEQ ID NO: 67 or a conservative modification thereof, and a CDR3 comprising the amino acid sequence set forth in SEQ ID NO: 94 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 90. a CDR2 comprising the amino acid sequence set forth in SEQ ID NO: 91, and a CDR3 comprising the amino acid sequence set forth in SEQ ID NO: 92; and a V_{L} comprising a CDR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94.

In certain embodiments, the extracellular antigen-binding domain of the CAR (e.g., an scFv) comprises a V_{H} comprising an amino acid sequence that is at least about 80% (*e.g.,* at least about 85%, at least about 90%, or at least about 95%) homologous or identical to the amino sequence set forth in SEQ ID NO: 95. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 95. In certain embodiments, the extracellular antigen-binding domain comprises a V_{H} comprising the amino sequence set forth in SEQ ID NO: 95. SEQ ID NO: 95 is provided in Table 11 below.

In certain embodiments, the extracellular antigen-binding domain of the CAR (e.g., an scFv) comprises a V_{L} comprising an amino acid sequence that is at least about 80% *(e.g.,* at least about 85%, at least about 90%, or at least about 95%) homologous or identical to the amino sequence set forth in SEQ ID NO: 96. For example, the extracellular antigen-binding domain of the CAR (e.g., an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 96. In certain embodiments, the extracellular antigen-binding domain comprises a V_{L} comprising the amino sequence set forth in SEQ ID NO: 96. SEQ ID NO: 96 is provided in Table 11 below.

In certain embodiments, the extracellular antigen-binding domain of the CAR (e.g., an scFv) comprises a V_{H} comprising the amino acid sequence set forth in SEQ ID NO: 95, and a V_{L} comprising the amino acid sequence set forth in SEQ ID NO: 96. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 97. In certain embodiments, the scFv is designated as "05F5". An exemplary nucleotide sequence encoding the amino acid sequence of SEQ ID NO: 97 is set forth in SEQ ID NO: 98. SEQ ID NOS: 97 and 98 are provided in Table 11 below.

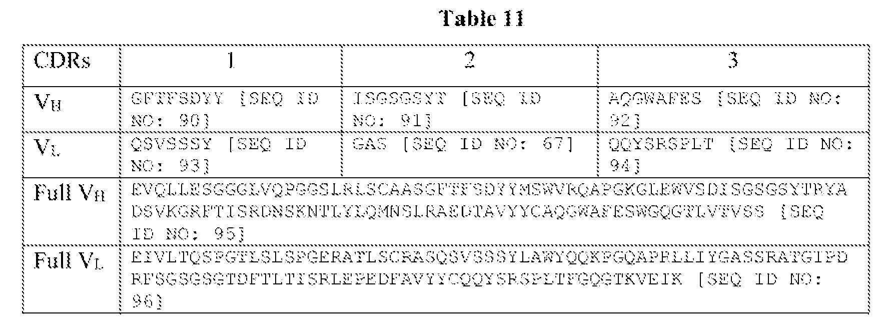

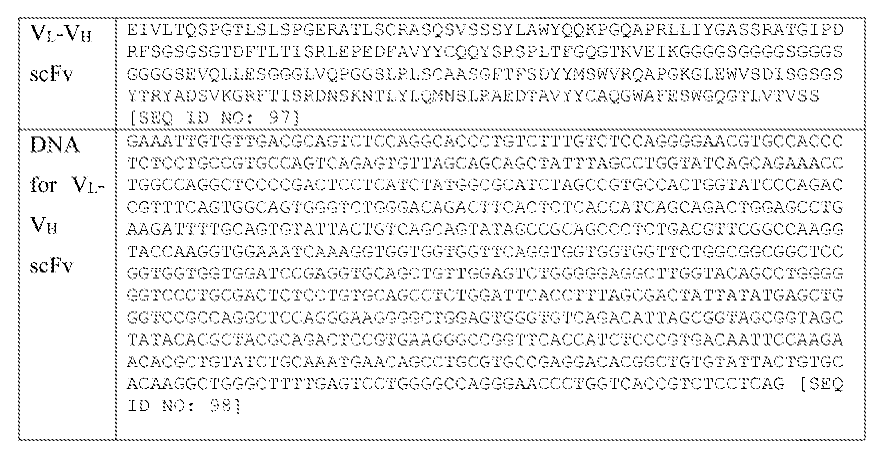

In certain embodiments, the extracellular antigen-binding domain of the CAR (e.g., an scFv) comprises a V_{H} comprising a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 99 or a conservative modification thereof. SEQ ID NOs: 45, 46 and 99 are provided in Table 12.

In certain embodiments, the extracellular antigen-binding domain of the CAR (e.g., an scFv) comprises a V_{L} comprising a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 100 or a conservative modification thereof. SEQ ID NOs: 66, 67, and 100 are provided in Table 12.

In certain embodiments, the extracellular antigen-binding domain of the CAR (e.g., an scFv) comprises a V_{H} comprising a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 99 or a conservative modification thereof; and a V_{L} comprising a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 100 or a conservative modification thereof.

In certain embodiments, the extracellular antigen-binding domain of the CAR (e.g., an scFv) comprises a V_{H} comprising a CDR1 comprising the amino acid sequence set forth in SEQ ID NO: 45, a CDR2 comprising the amino acid sequence set forth in SEQ ID NO: 46, a CDR3 comprising the amino acid sequence set forth in SEQ ID NO: 99; and a V_{L} comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.

In certain embodiments, the extracellular antigen-binding domain of the CAR (e.g., an scFv) comprises a V_{H} comprising an amino acid sequence that is at least about 80% *(e.g.,* at least about 85%, at least about 90%, or at least about 95%) homologous or identical to the amino sequence set forth in SEQ ID NO: 101. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 101. In certain embodiments, the extracellular antigen-binding domain comprises a V_{H} comprising the amino sequence set forth in SEQ ID NO: 101. SEQ ID NO: 101 is provided in Table 12 below.

In certain embodiments, the extracellular antigen-binding domain of the CAR (e.g., an scFv) comprises a V_{L} comprising an amino acid sequence that is at least about 80% *(e.g.,* at least about 85%, at least about 90%, or at least about 95%) homologous or identical to the amino sequence set forth in SEQ ID NO: 102. For example, the extracellular antigen-binding domain of the CAR (e.g., an scFv) comprises a V_{L} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 102. In certain embodiments, the extracellular antigen-binding domain comprises a V_{L} comprising the amino sequence set forth in SEQ ID NO: 102. SEQ ID NO: 102 is provided in Table 12 below.

In certain embodiments, the extracellular antigen-binding domain of the CAR (e.g., an scFv) comprises a V_{H} comprising the amino acid sequence set forth in SEQ ID NO: 101, and a V_{L} comprising the amino acid sequence set forth in SEQ ID NO: 102. In certain embodiments, the V_{H} and V_{L} are linked via a linker. In certain embodiments, the linker comprises the amino acid sequence set forth in SEQ ID NO: 110.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, scFv comprises the amino acid sequence set forth in SEQ ID NO: 103. In certain embodiments, the scFv is designated as "05G5". An exemplary nucleotide sequence encoding the amino acid sequence of SEQ ID NO: 103 is set forth in SEQ ID NO: 104. SEQ ID NOS: 103 and 104 are provided in Table 12 below.

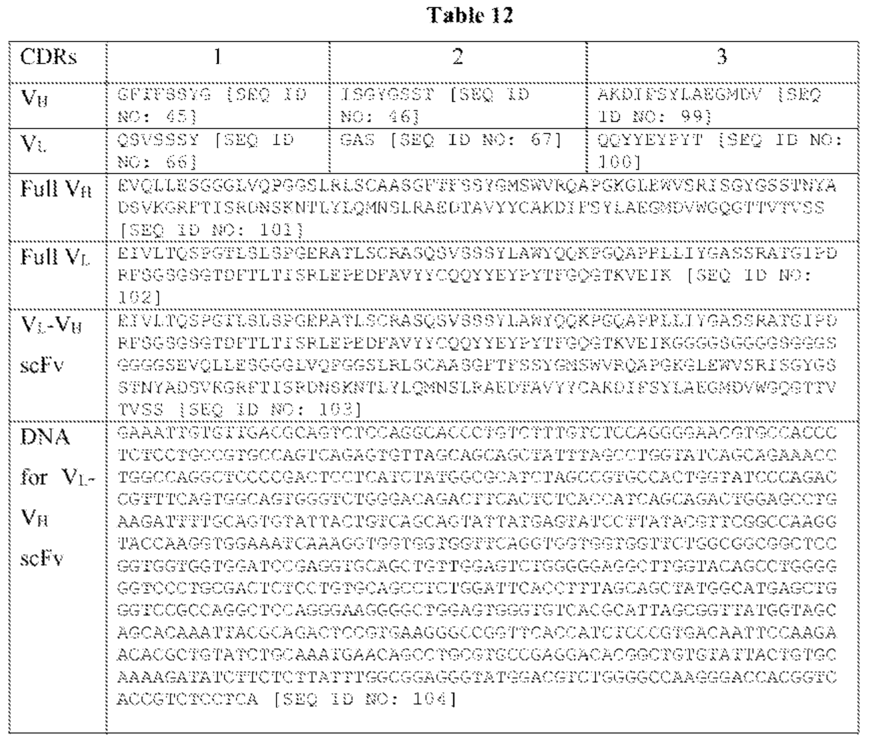

In certain embodiments, the extracellular antigen-binding domain of the CAR (e.g., an scFv) comprises a V_{H} comprising a CDR1 comprising the amino acid sequence set forth in SEQ ID NO: 105 or a conservative modification thereof, a CDR2 comprising the amino acid sequence set forth in SEQ ID NO: 106 or a conservative modification thereof, and a CDR3 comprising the amino acid sequence set forth in SEQ ID NO: 107 or a conservative modification thereof. SEQ ID NOs: 105-107 are provided in Table 13.

In certain embodiments, the extracellular antigen-binding domain of the CAR (e.g., an scFv) comprises a V_{H} comprising an amino acid sequence that is at least about 80% (*e.g.,* at least about 85%, at least about 90%, or at least about 95%) homologous or identical to the amino sequence set forth in SEQ ID NO: 108. For example, the extracellular antigen-binding domain of the CAR (e.g., an scFv) comprises a V_{H} comprising an amino acid sequence that is about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% homologous or identical to the amino sequence set forth in SEQ ID NO: 108. In certain embodiments, the extracellular antigen-binding domain comprises a V_{H} comprising the amino sequence set forth in SEQ ID NO: 108. SEQ ID NO: 108 is provided in Table 13 below. An exemplary nucleotide sequence encoding the amino acid sequence of SEQ ID NO: 108 is set forth in SEQ ID NO: 109. SEQ ID NOS: 108 and 109 are provided in Table 13 below.

In certain embodiments, the V_{H} and V_{L} are linked via a linker.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}. In certain embodiments, the scFv is designated as "07B3".

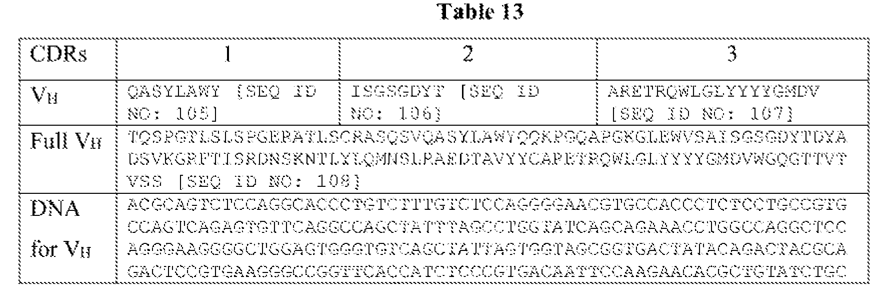

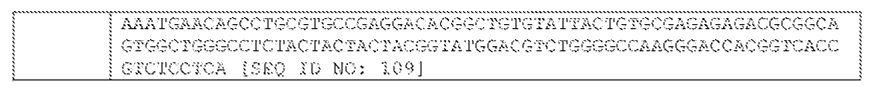

As used herein, the term "a conservative sequence modification" refers to an amino acid modification that does not significantly affect or alter the binding characteristics of the presently disclosed uPAR-targeted CAR (*e*.*g*., the extracellular antigen-binding domain of the CAR) comprising the amino acid sequence. Conservative modifications can include amino acid substitutions, additions and deletions. Modifications can be introduced into the extracellular antigen-binding domain of the presently disclosed CAR by standard techniques known in the art, such as site-directed mutagenesis and PCR-mediated mutagenesis. Amino acids can be classified into groups according to their physicochemical properties such as charge and polarity. Conservative amino acid substitutions are ones in which the amino acid residue is replaced with an amino acid within the same group. For example, amino acids can be classified by charge: positively-charged amino acids include lysine, arginine, histidine, negatively-charged amino acids include aspartic acid, glutamic acid, neutral charge amino acids include alanine, asparagine, cysteine, glutamine, glycine, isoleucine, leucine, methionine, phenylalanine, proline, serine, threonine, tryptophan, tyrosine, and valine. In addition, amino acids can be classified by polarity: polar amino acids include arginine (basic polar), asparagine, aspartic acid (acidic polar), glutamic acid (acidic polar), glutamine, histidine (basic polar), lysine (basic polar), serine, threonine, and tyrosine; non-polar amino acids include alanine, cysteine, glycine, isoleucine, leucine, methionine, phenylalanine, proline, tryptophan, and valine. Thus, one or more amino acid residues within a CDR region can be replaced with other amino acid residues from the same group and the altered antibody can be tested for retained function (*i.e*., the functions set forth in (c) through (l) above) using the functional assays described herein. In certain embodiments, no more than one, no more than two, no more than three, no more than four, no more than five residues within a specified sequence or a CDR region are altered.

The V_{H} and/or V_{L} amino acid sequences having at least about 80%, at least about 80%, at least about 85%, at least about 90%, or at least about 95% (*e.g*., about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99%) homology or identity to a specific sequence (*e.g*., SEQ ID NO: 7, SEQ ID NO: 8, SEQ ID NO: 15, SEQ ID NO: 16, SEQ ID NO: 23, SEQ ID NO: 24, SEQ ID NO: 31, SEQ ID NO: 32, SEQ ID NO: 41, SEQ ID NO: 42, SEQ ID NO: 50, SEQ ID NO: 51, SEQ ID NO: 59, SEQ ID NO: 60, SEQ ID NO: 69, SEQ ID NO: 70, SEQ ID NO: 78, SEQ ID NO: 79, SEQ ID NO: 86, SEQ ID NO: 87, SEQ ID NO: 95, SEQ ID NO: 96, SEQ ID NO: 101. SEQ ID NO: 102, or SEQ ID NO: 108) may contain substitutions (*e.g*., conservative substitutions), insertions, or deletions relative to the specified sequence(s), but retain the ability to bind to uPAR. In certain embodiments, a total of 1 to 10 amino acids are substituted, inserted and/or deleted in a specific sequence (*e.g.,* SEQ ID NO: 7, SEQ ID NO: 8, SEQ ID NO: 15, SEQ ID NO: 16. SEQ ID NO: 23, SEQ ID NO: 24, SEQ ID NO: 31, SEQ ID NO: 32, SEQ ID NO: 41, SEQ ID NO: 42, SEQ ID NO: 50, SEQ ID NO: 51, SEQ ID NO: 59, SEQ ID NO: 60, SEQ ID NO: 69, SEQ ID NO: 70, SEQ ID NO: 78, SEQ ID NO: 79, SEQ ID NO: 86, SEQ ID NO: 87, SEQ ID NO: 95, SEQ ID NO: 96, SEQ ID NO: 101, SEQ ID NO: 102, or SEQ ID NO: 108). In certain embodiments, substitutions, insertions, or deletions occur in regions outside the CDRs (*e*.*g*., in the FRs) of the extracellular antigen-binding domain. In certain embodiments, the extracellular antigen-binding domain comprises V_{H} and/or V_{L} sequence selected from SEQ ID NOs: 7, 8, 15, 16, 23, 24, 31, 32, 41, 42, 50, 51, 59, 60, 69, 70, 78, 79, 86, 87, 95, 96, 101, 102 or 108 including post-translational modifications of that sequence (SEQ ID NO: 7. 8, 15, 16, 23, 24, 31, 32, 41, 42, 50, 51, 59, 60, 69, 70, 78, 79, 86, 87, 95, 96, 101, 102 or 108).

In certain embodiments, the extracellular antigen-binding domain of a presently disclosed CAR cross-competes for binding to uPAR (*e.g.,* human uPAR) with a reference antibody or an antigen-binding fragment thereof comprising the V_{H} CDR1, CDR2, and CDR3 sequences and the V_{L} CDR1, CDR2, and CDR3 sequences of, for example, any one of the presently disclosed scFvs (*e.g.,* 3-C3-A, 3-D8-A, 3-G1-A, 3-H4-A, 4-F5-A, 4-F12-A, 4-A5-B. 05G9, 05A6, 05B2, 05F5. 05G5, and 07B3). In certain embodiments, the extracellular antigen-binding domain of a presently disclosed CAR cross-competes for binding to uPAR (*e.g..* human uPAR) with a reference antibody or an antigen-binding portion thereof comprising the V_{H} and V_{L} sequences of, for example, any one of the presently disclosed scFvs (*e.g.,* 3-C3-A. 3-D8-A, 3-G1-A, 3-H4-A, 4-F5-A, 4-F12-A, 4-A5-B, 0509, 05A6, 05B2, 05F5, 05G5, and 07B3).

In certain embodiments, the extracellular antigen-binding domain of a presently disclosed CAR cross-competes for binding to uPAR (*e.g..* human uPAR) with a reference antibody or an antigen-binding portion thereof comprising the V_{H} CDR1, CDR2, and CDR3 sequences and the V_{L} CDR1, CDR2, and CDR3 sequences of scFv 3-C3-A. For example, the extracellular antigen-binding domain of a presently disclosed CAR cross-competes for binding to uPAR (*e*.*g*., human uPAR) with a reference antibody or an antigen-binding portion thereof comprising a V_{H} compising a CDR1 comprising the amino acid sequence set forth in SEQ ID NO: 1, a CDR2 comprising the amino acid sequence set forth in SEQ ID NO: 2. and a CDR3 comprising the amino acid sequence set forth in SEQ ID NO: 3; and a V_{L} comprising a CDR1 comprising the amino acid sequence set forth in SEQ ID NO: 4, a CDR2 comprising amino acids having the sequence set forth in SEQ ID NO: 5, and a CDR3 comprising amino acids having the sequence set forth in SEQ ID NO: 6. In certain embodiments, the extracellular antigen-binding domain of a presently disclosed CAR cross-competes for binding to uPAR (*e.g.,* human uPAR) with a reference antibody or an antigen-binding portion thereof comprising the V_{H} and V_{L} sequences of scFv 3-C3-A . For example, the extracellular antigen-binding domain of a presently disclosed CAR cross-competes for binding to uPAR (*e.g.,* human uPAR) with a reference antibody or an antigen-binding portion thereof comprising a V_{H} comprising amino acids having the sequence set forth in SEQ ID NO: 7, and a V_{L} comprising amino acids having the sequence set forth in SEQ ID NO: 8.

In certain embodiments, the extracellular antigen-binding domain binds to the same epitope region on uPAR (*e*.*g*., human uPAR) as the reference antibody or antigen-binding portion thereof. For example, the extracellular antigen-binding domain of a presently disclosed CAR binds to the same epitope region on uPAR (*e.g.,* human uPAR) as a reference antibody or an antigen-binding portion thereof comprising the V_{H} comprising CDR1, CDR2, and CDR3 sequences and the V_{L} comprising CDR1, CDR2, and CDR3 sequences of, for example, any one of the presently disclosed scFvs (*e*.*g*.. 3-C3-A, 3-D8-A, 3-G1-A, 3-H4-A, 4-F5-A, 4-F12-A, 4-A5-B, 05G9, 05A6, 05B2, 05F5, 05G5, and 07B3). In certain embodiments, the extracellular antigen-binding domain of a presently disclosed CAR binds to the same epitope region on uPAR (*e.g.,* human uPAR) as a reference antibody or an antigen-binding portion thereof comprising the V_{H} and V_{L} sequences of, for example, any one of the presently disclosed scFvs (*e.g.,* 3-C3-A, 3-D8-A, 3-G1-A, 3-H4-A, 4-F5-A, 4-F12-A, 4-A5-B, 05G9, 05A6, 05B2, 05F5, 05G5, and 07B3).

Extracellular antigen-binding domains that cross-compete or compete with the reference antibody or antigen-binding portions thereof for binding to uPAR (*e.g.,* human uPAR) can be identified by using routine methods known in the art, including, but not limited to, ELISAs, radioimmunoassays (RIAs), Biacore, flow cytometry, Western blotting, and any other suitable quantitative or qualitative antibody-binding assays. Competition ELISA is described in Morris, "Epitope Mapping of Protein Antigens by Competition ELISA", The Protein Protocols Handbook (1996), pp 595-600, edited by J. Walker, which is incorporated by reference in its entirety. In certain embodiments, the antibody-binding assay comprises measuring an initial binding of a reference antibody to a uPAR polypeptide, admixing the reference antibody with a test extracellular antigen-binding domain, measuring a second binding of the reference antibody to the uPAR polypeptide in the presence of the test extracellular antigen-binding domain, and comparing the initial binding with the second binding of the reference antibody, wherein a decreased second binding of the reference antibody to the uPAR polypeptide in comparison to the initial binding indicates that the test extracellular antigen-binding domain cross-competes with the reference antibody for binding to uPAR, *e.g.,* one that recognizes the same or substantially the same epitope, an overlapping epitope, or an adjacent epitope. In certain embodiments, the reference antibody is labeled, *e.g.,* with a fluorochrome, biotin, or peroxidase. In certain embodiments, the uPAR polypeptide is expressed in cells, *e.g*., in a flow cytometry test. In certain embodiments, the uPAR polypeptide is immobilized onto a surface, including a Biacore ship (*e.g.,* in a Biacore test), or other media suitable for surface plasmon resonance analysis. The binding of the reference antibody in the presence of a completely irrelevant antibody (that does not bind to uPAR) can serve as the control high value. The control low value can be obtained by incubating a labeled reference antibody with an unlabeled reference antibody, where competition and reduced binding of the labeled reference antibody would occur. In certain embodiments, a test extracellular antigen-binding domain that reduces the binding of the reference antibody to a uPAR polypeptide by at least about 20%, at least about 30%, at least about 40%, at least about 50%, at least about 60%, at least about 70%, at least about 80%, at least about 90%, or at least about 95% is considered to be an extracellular antigen-binding domain that cross-competes with the reference antibody for binding to uPAR. In certain embodiments, the assays are performed at room temperature.

In certain embodiments, the antibody-binding assay comprises measuring an initial binding of a test extracellular antigen-binding domain to a uPAR polypeptide, admixing the test extracellular antigen-binding domain with a reference antibody, measuring a second binding of the test extracellular antigen-binding domain to the uPAR polypeptide in the presence of the reference antibody, and comparing the initial binding with the second binding of the test extracellular antigen-binding domain, where a decreased second binding of the test extracellular antigen-binding domain to the uPAR polypeptide in comparison to the initial binding indicates that the test extracellular antigen-binding domain cross-competes with the reference antibody for binding to uPAR, *e.g.,* one that recognizes the same or substantially the same epitope, an overlapping epitope, or an adjacent epitope. In certain embodiments, the test extracellular antigen-binding domain is labeled, *e.g.,* with a fluorochrome, biotin, or peroxidase. In certain embodiments, the uPAR polypeptide is expressed in cells, *e.g.,* in a flow cytometry test. In certain embodiments, the uPAR polypeptide is immobilized onto a surface, including a Biacore ship (*e.g.,* in a Biacore test), or other media suitable for surface plasmon resonance analysis. The binding of the test extracellular antigen-binding domain in the presence of a completely irrelevant antibody (that does not bind to uPAR) can serve as the control high value. The control low value can be obtained by incubating a labeled test extracellular antigen-binding domain with an unlabeled test extracellular antigen-binding domain, where competition and reduced binding of the labeled test extracellular antigen-binding domain would occur. In certain embodiments, a test extracellular antigen-binding domain, whose binding to a uPAR polypeptide is decreased by at least about 20%, at least about 30%, at least about 40%, at least about 50%, at least about 60%, at least about 70%, at least about 80%, at least about 90%, or at least about 95% in the presence of a reference antibody, is considered to be an extracellular antigen-binding domain that cross-competes with the reference antibody for binding to uPAR. In certain embodiments, the assays are performed at room temperature.

In certain non-limiting embodiments, the extracellular antigen-binding domain of the presently disclosed CAR comprises a linker connecting the heavy chain variable region and light chain variable region of the extracellular antigen-binding domain. In certain embodiments, the linker comprises or consists of the amino acid sequence set forth in SEQ ID NO: 110. In certain embodiments, the linker comprises or consists of the amino acid sequence set forth in SEQ ID NO: 111. In certain embodiments, the linker comprises or consists of the amino acid sequence set forth in SEQ ID NO: 112. In certain embodiments, the linker comprises or consists of the amino acid sequence set forth in SEQ ID NO: 113. In certain embodiments, the linker comprises or consists of the amino acid sequence set forth in SEQ ID NO: 114. In certain embodiments, the linker comprises or consists of the amino acid sequence set forth in SEQ ID NO: 115.

In certain embodiments, the variable regions within the extracellular antigen-binding domain of the CAR have to be linked one after another such that at the N-terminus of the extracellular antigen-binding domain, a heavy chain variable region (V_{H}) is positioned. In certain embodiments, if the extracellular antigen-binding domain of the CAR is an scFv, the variable regions are positioned from the N- to the C-terminus: V_{H}-V_{L}.

In certain embodiments, the variable regions within the extracellular antigen-binding domain of the CAR have to be linked one after another such that at the N-terminus of the extracellular antigen-binding domain, a light chain variable region (V_{L}) is positioned. In certain embodiments, if the extracellular antigen-binding domain of the CAR is an scFv, the variable regions are positioned from the N- to the C-terminus: V_{L}-V_{H}.

In addition, the extracellular antigen-binding domain can comprise a leader or a signal peptide that directs the nascent protein into the endoplasmic reticulum. Signal peptide or leader can be essential if the CAR is to be glycosylated and anchored in the cell membrane. The signal sequence or leader can be a peptide sequence (about 5. about 10, about 15, about 20, about 25, or about 30 amino acids long) present at the N-terminus of newly synthesized proteins that directs their entry to the secretory pathway. In certain embodiments, the signal peptide is covalently joined to the 5' terminus of the extracellular antigen-binding domain. In certain embodiments, the signal peptide comprises a CD8 polypeptide, e.g., the CAR comprises a truncated CD8 signal peptide.

### 4.3.2.2. Transmembrane Domain of a CAR

In certain non-limiting embodiments, the transmembrane domain of the CAR comprises a hydrophobic alpha helix that spans at least a portion of the membrane. Different transmembrane domains result in different receptor stability. After antigen recognition, receptors cluster and a signal are transmitted to the cell. In accordance with the presently disclosed subject matter, the transmembrane domain of the CAR can comprise a native or modified transmembrane domain of CD8 or a fragment thereof, a native or modified transmembrane domain of CD28 or a fragment thereof, a native or modified transmembrane domain of CD3ζ or a fragment thereof, a native or modified transmembrane domain of CD4 or a fragment thereof, a native or modified transmembrane domain of 4-1BB or a fragment thereof, a native or modified transmembrane domain of OX40 or a fragment thereof, a native or modified transmembrane domain of ICOS or a fragment thereof, a native or modified transmembrane domain of CD84 or a fragment thereof, a native or modified transmembrane domain of CD166 or a fragment thereof, a native or modified transmembrane domain of CD8a or a fragment thereof, a native or modified transmembrane domain of CD8b or a fragment thereof, a native or modified transmembrane domain of ICAM-1 or a fragment thereof, a native or modified transmembrane domain of CTLA-4 or a fragment thereof, a native or modified transmembrane domain of CD27 or a fragment thereof, a native or modified transmembrane domain of CD40 or a fragment thereof, NKGD2 or a fragment thereof, or a combination thereof.

In certain embodiments, the transmembrane domain of the CAR comprises a CD8 polypeptide (e.g., a transmembrane domain of CD8 or a fragment thereof). In certain embodiments, the transmembrane domain of the CAR comprises a transmembrane domain of human CD8 or a fragment thereof. In certain embodiments, the CD8 polypeptide comprises or consists of an amino acid sequence that is at least about 85%, about 90%, about 95%, about 96%, about 97%, about 98%, about 99% or about 100% homologous or identical to the amino acid sequence having a NCBI Reference No: NP_001139345.1 (SEQ ID NO: 117) or a fragments thereof, and/or may optionally comprise up to one or up to two or up to three conservative amino acid substitutions. In certain embodiments, the CD8 polypeptide comprises or consists of an amino acid sequence that is a consecutive portion of SEQ ID NO: 117, which is at least 20, or at least 30, or at least 40, or at least 50, and up to 235 amino acids in length. Alternatively or additionally, in non-limiting various embodiments, the CD8 polypeptide comprises or consists of an amino acid sequence of amino acids 1 to 235, 1 to 50, 50 to 100, 100 to 150, 150 to 200, 137 to 209 or 200 to 235 of SEQ ID NO: 117. In certain embodiments, the transmembrane domain of the CAR comprises a CD8 polypeptide comprising or consisting of amino acids 137 to 209 of SEQ ID NO: 117. SEQ ID NO: 117 is provided below.




 (SEQ ID NO: 117)

In certain embodiments, the transmembrane domain of the CAR comprises a transmembrane domain of mouse CD8 or a fragment thereof. In certain embodiments, the CD8 polypeptide comprises or consists of an amino acid sequence that is at least about 85%, about 90%, about 95%, about 96%, about 97%, about 98%, about 99% or about 100% homologous or identical to the amino acid sequence having a NCBI Reference No: AAA92533.1 (SEQ ID NO: 118) or a fragment thereof, and/or may optionally comprise up to one or up to two or up to three conservative amino acid substitutions. In certain embodiments, the CD8 polypeptide comprises or consists of an amino acid sequence that is a consecutive portion of SEQ ID NO: 118, which is at least about 20, or at least about 30, or at least about 40, or at least about 50, or at least about 60, or at least about 70, or at least about 100, or at least about 200, and up to 247 amino acids in length. Alternatively or additionally, in non-limiting various embodiments, the CD8 polypeptide comprises or consists of an amino acid sequence of amino acids 1 to 247, 1 to 50, 50 to 100, 100 to 150, 150 to 200, 151 to 219, or 200 to 247 of SEQ ID NO: 118. In certain embodiments, the transmembrane domain of the CAR comprises a CD8 polypeptide comprising or consisting of amino acids 151 to 219 of SEQ ID NO: 118. SEQ ID NO: 118 is provided below.

1 MASPLTRFLS LNLLLMGESI ILGSGEAKPQ APELRIFPKK MDAELGQKVD LVCEVLGSVS
61 QGCSWLFQNS SSKLPQPTFV VYMASSHNKI TWDEKLNSSK LFSAVRDTNN KYVLTLNKFS
121 KENEGYYFCS VISNSVMYFS SVVPVLQKVN STTTKPVLRT PSPVHPTGTS QPQRPEDCRP
181 RGSVKGTGLD FACDIYIWAP LAGICVAPLL SLIITLICYH RSRKRVCKCP RPLVRQEGKP
241 RPSEKIV [SEQ ID NO: 118]

In certain embodiments, the transmembrane domain of a presently disclosed CAR comprises a CD28 polypeptide (e.g., a transmembrane domain of CD28 or a fragment thereof).

In certain embodiments, the transmembrane domain of the CAR comprises a transmembrane domain of human CD28 or a fragment thereof. In certain embodiments, the CD28 polypeptide comprises or consists of an amino acid sequence that is at least about 85%, about 90%, about 95%, about 96%, about 97%, about 98%, about 99% or 100% homologous or identical to the amino acid sequence having a NCBI Reference No: NP_000130 (SEQ ID No: 119) or a fragment thereof, and/or may optionally comprise up to one or up to two or up to three conservative amino acid substitutions. In non-limiting certain embodiments, the CD28 polypeptide comprises or consists of an amino acid sequence that is a consecutive portion of SEQ ID NO: 119 which is at least 20, or at least 30, or at least 40, or at least 50. and up to 220 amino acids in length. Alternatively or additionally, in non-limiting various embodiments, the CD28 polypeptide comprises or consists of an amino acid sequence of amino acids 1 to 220, 1 to 50, 50 to 100, 100 to 150, 150 to 200, 153 to 179, or 200 to 220 of SEQ ID NO: 119. In certain embodiments, the transmembrane domain of the CAR comprises a CD28 polypeptide comprising or consisting of amino acids 153 to 179 of SEQ ID NO: 119. SEQ ID NO: 119 is provided below:

1     MLRLLLALNL FPSIQVTGNK ILVKQSPMLV AYDNAVNLSC KYSYNLFSRE FRASLHKGLD
61    SAVEVCVVYG NYSQQLQVYS KTGFNCDGKL GNESVTFYLQ NLYVNQTDIY FCKIEVMYPP
121   PYLDNEKSNG TIIHVKGKHL CPSPLFPGPS KPFWVLVVVG GVLACYSLLV TVAFIIFWVR
181   SKRSRLLHSD YMNMTPRRPG PTRKHYQPYA PPRDFAAYRS [SEQ ID NO: 119]

In certain embodiments, the transmembrane domain of the CAR comprises a CD28 polypeptide (e.g., a transmembrane domain of mouse CD28 or a fragment thereof). In certain embodiments, the CD28 polypeptide comprises or consists of an amino acid sequence that is at least about 85%, about 90% about 95%, about 96%, about 97%, about 98%, about 99% or 100% homologous or identical to the amino acid sequence having a NCBI Reference No: NP_031668.3 (SEQ ID No: 120) or a fragment thereof, and/or may optionally comprise up to one or up to two or up to three conservative amino acid substitutions. In non-limiting certain embodiments, the CD28 polypeptide comprises or consists of an amino acid sequence that is a consecutive portion of SEQ ID NO: 120, which is at least 20, or at least 30. or at least 40, or at least 50, and up to 218 amino acids in length. Alternatively or additionally, in non-limiting various embodiments, the CD28 polypeptide comprises or consists of an amino acid sequence of amino acids 1 to 220, 1 to 50. 50 to 100, 100 to 150, 150 to 200, 151 to 177, or 200 to 218 of SEQ ID NO: 120. In certain embodiments, the transmembrane domain of the CAR comprises a CD28 polypeptide comprising or consisting of amino acids 151 to 177 of SEQ ID NO: 120. SEQ ID NO: 120 is provided below:

1 MTLRLLFLAL NFFSVQVTEN KILVKQSPLL VVDSNEVSLS CRYSYNLLAK EFRASLYKGV
61 NSDVEVCVGN GNFTYQPQFR SNAEFNCDGD FDNETVTFRL WNLHVNHTDI YFCKIEFMYP
121 PPYLDNERSN GTIIHIKEKH LCHTQSSPKL FWALVVVAGV LFCYGLLVTV ALCVIWTNSR
181 RNRLLQSDYM NMTPRRPGLT RKPYQPYAPA RDFAAYRP [SEQ ID NO: 120]

In certain non-limiting embodiments, the CAR further comprises a spacer region that links the extracellular antigen-binding domain to the transmembrane domain. The spacer region can be flexible enough to allow the antigen binding domain to orient in different directions to facilitate antigen recognition while preserving the activating activity of the CAR.

In certain embodiments, the hinge spacer region of the CAR comprises a native or modified hinge region of CD8 or a fragment thereof, a native or modified hinge region of CD28 or a fragment thereof, a native or modified hinge region of CD3ζ or a fragment thereof, a native or modified hinge region of CD40 or a fragment thereof, a native or modified hinge region of 4-1BB or a fragment thereof, a native or modified hinge region of OX40 or a fragment thereof, a native or modified hinge region of CD84 or a fragment thereof, a native or modified hinge region of CD166 or a fragment thereof, a native or modified hinge region of CD8a or a fragment thereof, a native or modified hinge region of CD8b or a fragment thereof, a native or modified hinge region of ICOS or a fragment thereof, a native or modified hinge region of ICAM-1 or a fragment thereof, a native or modified hinge region of CTLA-4 or a fragment thereof, a native or modified hinge region of CD27 or a fragment thereof, a native or modified hinge region of CD40 or a fragment thereof, a native or modified hinge region of NKGD2 or a fragment thereof, a synthetic polypeptide (not based on a protein associated with the immune response), or a combination thereof. The hinge/spacer region can be the hinge region from IgG1, or the CH₂CH₃ region of immunoglobulin and portions of CD3, a portion of a CD28 polypeptide (e.g., a portion of SEQ ID NO: 119 or 120), a portion of a CD8 polypeptide (e.g., a portion of SEQ ID NO: 117 or 118), a variation of any of the foregoing which is at least about 80%, at least about 85%, at least about 90%, at least about 95%, or at least about 100% homologous or identical thereto, or a synthetic spacer sequence.

### 4.3.2.3. Intracellular Signaling Domain of a CAR

In certain embodiments, the CAR comprises an intracellular signaling domain. In certain non-limiting embodiments, the intracellular signaling domain of the CAR comprises a CD3ζ polypeptide. CD3ζ can activate or stimulate a cell (e.g., a cell of the lymphoid lineage, *e.g.,* a T cell). Wild type ("native") CD3ζ comprises three functional immunoreceptor tyrosine-based activation motifs (ITAMs), three functional basic-rich stretch (BRS) regions (BRS1, BRS2 and BRS3). CD3ζ transmits an activation signal to the cell (*e.g.,* a cell of the lymphoid lineage, *e.g.,* a T cell) after antigen is bound. The intracellular signaling domain of the CD3ζ-chain is the primary transmitter of signals from endogenous TCRs.

In certain embodiments, the intracellular signaling domain of the CAR comprises a native CD3ζ polypeptide. In certain embodiments, the CD3ζ polypeptide comprises or consists of an amino acid sequence that is at least about 85%, about 90%, about 95%, about 96%, about 97%, about 98%, about 99% or about 100% homologous or identical to the amino acid sequence having a NCBI Reference No: NP_932170 (SEQ ID NO: 121) or a fragment thereof, and/ or may optionally comprise up to one or up to two or up to three conservative amino acid substitutions. In certain non-limiting embodiments, the CD3ζ polypeptide comprises or consists of an amino acid sequence that is a consecutive portion of SEQ ID NO: 121, which is at least 20, or at least 30, or at least 40, or at least 50, and up to 164 amino acids in length. Alternatively or additionally, in non-limiting various embodiments, the CD3ζ polypeptide comprises or consists of an amino acid sequence of amino acids 1 to 164, 1 to 50. 50 to 100, 52 to 164, 100 to 150. or 150 to 164 of SEQ ID NO: 121. In certain embodiments, the intracellular signaling domain of the CAR comprises a CD3ζ polypeptide comprising or consisting of amino acids 52 to 164 of SEQ ID NO: 121. SEQ ID NO: 121 is provided below:

1 MKWKALFTAA ILQAQLPITE AQSFGLLDPK LCYLLDGILF IYGVILTALF LRVKFSRSAD

61 APAYQQGQNQ LYNELNLGRR EEYDVLDKRR GRDPEMGGKP QRRKNPQEGL YNELQKDKMA

121 EAYSEIGMKG ERRRGKGHDG LYQGLSTATK DTYDALHMQA LPPR [SEQ ID NO: 121]

In certain embodiments, the intracellular signaling domain of the CAR comprises a modified CD3ζ polypeptide. In certain embodiments, the modified CD3ζ polypeptide comprises one, two, or three ITAMs. In certain embodiments, the modified CD3ζ polypeptide comprises a native ITAMI. In certain embodiments, the native ITAM1 comprises or consists of the amino acid sequence set forth in SEQ ID NO: 123.
QNQLYNELNLGRREEYDVLDKR (SEQ ID NO: 123]

An exemplary nucleic acid sequence encoding the amino acid sequence of SEQ ID NO: 123 is set forth in SEQ ID NO: 124, which is provided below.
CAGAACCAGCTCTATAACGAGCTCAATCAGGACGAAGAGGAGTACGATGTTTTGGACAAGAGA [SEQ ID NO: 124]

In certain embodiments, the modified CD3ζ polypeptide comprises an ITAM1 variant comprising one or more loss-of-function mutations. In certain embodiments, the ITAM1 variant comprises or consists of two loss-of-function mutations. In certain embodiments, each of the one or more (e.g., two) loss of function mutations comprises a mutation of a tyrosine residue in ITAM1. In certain embodiments, the ITAMI variant consists of two loss-of-function mutations. In certain embodiments, the ITAM1 variant comprises or consists of the amino acid sequence set forth in SEQ ID NO: 125, which is provided below.
QNQLPNELNLGPPEEPDVLDRR [SEQ ID NO: 125]

An exemplary nucleic acid sequence encoding the amino acid sequence of SEQ ID NO: 125 is set forth in SEQ ID NO: 126, which is provided below.
CAGAACCAGCTCTTTAACGAGCTCAATCTAGGACGAAGAGAGGAGTTCGATGTTTTGGACAAGAGA [SEQ ID NO:126]

In certain embodiments, the modified CD3ζ polypeptide comprises a native ITAM2. In certain embodiments, the native ITAM2 comprises or consists of the amino acid sequence set forth in SEQ ID NO: 127, which is provided below.
QEGLYNELQKDKMAEAYSEIGMK (SEQ ID NO: 127]

An exemplary nucleic acid sequence encoding the amino acid sequence of SEQ ID NO: 127 is set forth in SEQ ID NO: 128, which is provided below.
CAGGAAGGCCTGTACAATGAACTGCAGAAAGATAAGATGGCGGAGGCCTACAGTGAGATTGGGATGAAA [SEQ ID NO: 128]

In certain embodiments, the modified CD3ζ polypeptide comprises an ITAM2 variant. In certain embodiments, the ITAM2 variant comprises or consists of one or more loss-of-function mutations. In certain embodiments, the HAM2 variant comprises or consists of two loss-of-function mutations. In certain embodiments, each of the one or more (e.g., two) the loss of function mutations comprises a mutation of a tyrosine residue in ITAM2. In certain embodiments, the ITAMI variant consists of two loss-of-function mutations. In certain embodiments, the ITAM2 variant comprises or consists of the amino acid sequence set forth in SEQ ID NO: 129, which is provided below.
QEGLFNELQKDKMAEAFSEIGMK (SEQ ID NO: 129]

An exemplary nucleic acid sequence encoding the amino acid sequence of SEQ ID NO: 129 is set forth in SEQ ID NO: 130, which is provided below.
CAGGAAGGCCTGTTCAATGAACTGCAGAAAGATAAGATGGCGGAGGCCTTCAGTGAGATTGGGATGAAA [SEQ ID NO: 130]

In certain embodiments, the modified CD3ζ polypeptide comprises a native ITAM3. In certain embodiments, the native ITAM3 comprises or consists of the amino acid sequence set forth in SEQ ID NO: 131, which is provided below.
HDGLYQGLSTATKDTYDALHMQ (SEQ ID NO: 131]

An exemplary nucleic acid sequence encoding the amino acid sequence of SEQ ID NO: 131 is set forth in SEQ ID NO: 132, which is provided below.
CACGATGGCCTTTACCAGGGTCTCAGTACAGCCACCAAGGACACCTACGACGCCCTTCACATGCAG [SEQ ID NO: 132]

In certain embodiments, the modified CD3ζ polypeptide comprises an ITAM3 variant. In certain embodiments, the ITAM3 variant comprises or consists of two loss-of-function mutations. In certain embodiments, each of the one or more (e.g., two) the loss of function mutations comprises a mutation of a tyrosine residue in ITAM3. In certain embodiments, the ITAM3 variant comprises or consists of two loss-of- function mutations. In certain embodiments, the ITAM3 variant comprises or consists of the amino acid sequence set forth in SEQ ID NO: 133, which is provided below.
HDGLFQGLSTATKDTFDALHMQ [SEQ ID NO: 133]

An exemplary nucleic acid sequence encoding the amino acid sequence of SEQ ID NO: 133 is set forth in SEQ ID NO: 134, which is provided below.
CACGATGGCCTTTTCCAGGGGCTCAGTACAGCCACCAAGGACACCTTCGACGCCCTTCACATGCAG [SEQ ID NO: 134]

Various modified CD3ζ polypeptides and CARs comprising modified CD3ζ polypeptides are disclosed in International Patent Application Publication No. WO2019/133969, which is incorporated by reference hereby in its entirety.

In certain embodiments, the intracellular signaling domain of the CAR comprises a modified CD3ζ polypeptide comprising a native ITAM1, an ITAM2 variant comprising or consisting of one or more (e.g., two) loss-of-function mutations, and an ITAM3 variant comprising or consisting of one or more (e.g., two) loss-of-function mutations. In certain embodiments, the intracellular signaling domain of the CAR comprises a modified CD3ζ polypeptide comprising a native ITAM1, an ITAM2 variant consisting of two loss-of-function mutations, and an ITAM3 variant consisting of two loss-of-function mutations. In certain embodiments, the intracellular signaling domain of the CAR comprises a modified CD3ζ polypeptide comprising a native ITAM1 consisting of the amino acid sequence set forth in SEQ ID NO: 125, an ITAM2 variant consisting of the amino acid sequence set forth in SEQ ID NO: 129, and an ITAM3 variant consisting of the amino acid sequence set forth in SEQ ID NO: 133. In certain embodiments, the CAR is designated as "1XX". In certain embodiments, the modified CD3ζ polypeptide comprises or consists of the amino acid sequence set forth in SEQ ID NO: 135. SEQ ID NO: 135 is provided below:

RVKFSRSADA PAYQQGQNQL YNELNLGRRE EYDVLDKRRG RDPEMGGKPR RKNPQEGLFN ELQKDKMAEA
FSEIGMKGER RRGKGHDGLF QGLSTATKDT FDALHMQALP PR [SEQ ID NO: 135]

In certain embodiments, the intracellular signaling domain of the CAR comprises a modified CD3ζ polypeptide comprising or consisting of an amino acid sequence that is at least about 80%, at least about 85%, at least about 90%, at least about 95%, at least about 96%, at least about 97%, at least about 98%, or at least about 99%, at least about 100% identical to SEQ ID NO: 135 or a fragment thereof, and/or may optionally comprise up to one or up to two or up to three conservative amino acid substitutions.

An exemplary nucleic acid sequence encoding the amino acid sequence of SEQ ID NO: 135 is set forth in SEQ ID NO: 136, which is provided below.

AGAGTGAAGTTCAGCAGGAGCGCAGACGCCCCCGCGTACCAGCAGGGCCAGAACCAGCTCTATAACGAGCTCAATCTA
GGACGAAGAGAGGAGTACGATGTTTTGGACAAGAGACGTGGCCGGGACCCTGAGATGGGGGGAAAGCCGAGAAGGAAG
AACCCTCAGGAAGGCCTGTTCAATGAACTGCAGAAAGATAAGATGGCGGAGGCCTTCAGTGAGATTGGGATGAAAGGC
GAGCGCCGGAGGGGCAAGGGGCACGATGGCCTTTTCCAGGGGCTCAGTACAGCCACCAAGGACACCTTCGACGCCCTT
CACATGCAGGCCCTGCCCCCTCGC [SEQ ID NO: 136]

In certain non-limiting embodiments, the intracellular signaling domain of the CAR further comprises at least a co-stimulatory signaling region. In certain embodiments, the co-stimulatory signaling region comprises at least one co-stimulatory molecule or a fragment thereof. In certain embodiments, the co-stimulatory signaling region comprises an intracellular domain of at least one co-stimulatory molecule or a fragment thereof.

As used herein, a "co-stimulatory molecule" refers to a cell surface molecule other than antigen receptor or its ligand that can provide an efficient response of lymphocytes to an antigen. In certain embodiments, a co-stimulatory molecule can provide optimal lymphocyte activation. Non-limiting examples of co-stimulatory molecules include CD28, 4-1BB, OX40, ICOS, DAP-10, CD27, CD40, NKGD2, CD2, FN14, HVEM, LTBR, CD28H, TNFR1, TNFR2, BAFF-R, BCMA, TAC1, TROY, RANK, CD40, CD27, CD30, EDAR. XEDAR, GITR, DR6, and NGFR, and combinations thereof. The co-stimulatory molecule can bind to a co-stimulatory ligand, which is a protein expressed on cell surface that upon binding to its receptor produces a co-stimulatory response, *i.e.,* an intracellular response that effects the stimulation provided when an antigen-recognizing receptor (e.g., a chimeric antigen receptor (CAR)) binds to its target antigen. As one example, a 4-IBB ligand (i.e., 4-1BBL) may bind to 4-IBB for providing an intracellular signal that in combination with a CAR signal induces an effector cell function of the CAR⁺ T cell.

In certain embodiments, the intracellular signaling domain of the CAR comprises a co-stimulatory signaling region that comprises a CD28 polypeptide, e.g., an intracellular domain of CD28 or a fragment thereof. The CD28 polypeptide can comprise or have an amino acid sequence that is at least about 80%, at least about 85%, at least about 90%, at least about 95%, at least about 96%, at least about 97%, at least about 98%, or at least about 99%, at least about 100% homologous or identical to the amino acid sequence set forth in SEQ ID NO: 119 or a fragment thereof, and/or may optionally comprise up to one or up to two or up to three conservative amino acid substitutions. In non-limiting certain embodiments, the CD28 polypeptide comprises or consists of an amino acid sequence that is a consecutive portion of SEQ ID NO: 119, which is at least 20, or at least 30, or at least 40, or at least 50, and up to 220 amino acids in length. Alternatively or additionally, in non-limiting various embodiments, the CD28 polypeptide comprises or consists of an amino acid sequence of amino acids 1 to 220, 1 to 50, 50 to 100, 100 to 150, 114 to 220. 150 to 200, 180 to 220, or 200 to 220 of SEQ ID NO: 119. In certain embodiments, the intracellular signaling domain of the CAR comprises a co-stimulatory signaling region that comprises a CD28 polypeptide comprising or consisting of an amino acid sequence of amino acids 180 to 220 of SEQ ID NO: 119.

In certain embodiments, the CD28 polypeptide comprises or consists of an amino acid sequence that is at least about 80%, at least about 85%, at least about 90%, at least about 95%, at least about 96%, at least about 97%, at least about 98%, or at least about 99%, at least about 100% homologous or identical to the amino acid sequence set forth in SEQ ID NO: 120 or a fragment thereof, and/or may optionally comprise up to one or up to two or up to three conservative amino acid substitutions. In non-limiting certain embodiments, the CD28 polypeptide comprises or consists of an amino acid sequence that is a consecutive portion of SEQ ID NO: 120, which is at least about 20, or at least about 30, or at least about 40, or at least about 50, and up to 218 amino acids in length. Alternatively or additionally, in non-limiting various embodiments, the CD28 polypeptide comprises or consists of an amino acid sequence of amino acids 1 to 218, 1 to 50, 50 to 100, 100 to 150, 150 to 218, 178 to 218, or 200 to 218 of SEQ ID NO: 120. In certain embodiments, the co-stimulatory signaling region of a presently disclosed CAR comprises a CD28 polypeptide that comprises or consists of the amino acids 178 to 218 of SEQ ID NO: 120.

In certain embodiments, the intracellular signaling domain of the CAR comprises a co-stimulatory signaling region that comprises a 4-IBB polypeptide, e.g., an intracellular domain of 4-1BB or a fragment thereof. The 4-IBB polypeptide can comprise or consists of an amino acid sequence that is at least about 80%, at least about 85%, at least about 90%, at least about 95%, at least about 96%, at least about 97%, at least about 98%, or at least about 99%, at least about 100% homologous or identical to the amino acid sequence having a NCBI Ref. No.: NP_001552 (SEQ ID NO: 122) or a fragment thereof, and/or may optionally comprise up to one or up to two or up to three conservative amino acid substitutions. In non-limiting certain embodiments, the 4-IBB polypeptide comprises or consists of an amino acid sequence that is a consecutive portion of SEQ ID NO: 122, which is at least 20, or at least 30, or at least 40, or at least 50, or at least 100, or at least 150, or at least 150, and up to 255 amino acids in length. Alternatively or additionally, in non-limiting various embodiments, the 4-1BB polypeptide comprises or consists of an amino acid sequence of amino acids 1 to 255, 1 to 50, 50 to 100, 100 to 150, 150 to 200, or 200 to 255 of SEQ ID NO: 122. In certain embodiments, the intracellular signaling domain of the CAR comprises a co-stimulatory signaling region that comprises a 4-IBB polypeptide comprising or consisting of an amino acid sequence of amino acids 214 to 255 of SEQ ID NO: 122. SEQ ID NO: 122 is provided below.

1 MGNSCYNIVA TLLLVLNFER TRSLQDPCSN CPAGTFCDNN RNQICSPCPP NSFSSAGGQR
61 TCDICRQCKG VFRTRKECSS TSNAECDCTP GFHCLGAGCS MCEQDCKQGQ ELTKKGCKDC
121 CFGTFNDQKR GICRPWTNCS LDGKSVLVNG TKERDVVCGP SPADLSPGAS SVTPPAPARE
181 PGHSPQIISF FLALTSTALL FLLFFLTLRF SVVKRGRKKL LYIFKQPFMR PVQTTQEEDG
241 CSCRFPEEEE GGCEL [SEQ ID NO: 122]

In certain embodiments, the intracellular signaling domain of the CAR comprises a co-stimulatory signaling region that comprises intracellular domains of two or more co-stimulatory molecules or portions thereof, e.g., an intracellular domain of CD28 or a fragment thereof and an intracellular domain of 4-1BB or a fragment thereof, or an intracellular domain of CD28 or a fragment thereof and an intracellular domain of OX40 or a fragment thereof.

In certain embodiments, a presently disclosed CAR further comprises an inducible promoter, for expressing nucleic acid sequences in human cells. Promoters for use in expressing CAR genes can be a constitutive promoter, such as ubiquitin C (UbiC) promoter.

### 4.3.3. TCR like Fusion Molecules

In certain embodiments, the antigen-recognizing receptor is a TCR like fusion molecule. Non-limiting examples of TCR fusion molecules include HLA-Independent TCR-based Chimeric Antigen Receptor (also known as "HIT-CAR", e.g., those disclosed in International Patent Application No. PCT/US19/017525, which is incorporated by reference in its entirety), and T cell receptor fusion constructs (TRuCs) (e.g., those disclosed in Baeuerle et al., "Synthetic TRuC receptors engaging the complete T cell receptor for potent anti-tumor response," Nature Communications volume 10, Article number: 2087 (2019), which is incorporated by reference in its entirety).

In certain embodiments, the TCR like fusion molecule comprises an antigen binding chain that comprises an extracellular antigen-binding domain and a constant domain, wherein the TCR like fusion molecule binds to an antigen in an HLA-independent manner. In certain embodiments, the constant domain comprises a T cell receptor constant region selected from the group consisting of a native or modified TRAC peptide, a native or modified TRBC peptide, a native or modified TRDC peptide, a native or modified TRGC peptide and any variants or functional fragments thereof. In certain embodiments, the constant domain comprises a native or modified TRAC peptide. In certain embodiments, the constant domain comprises a native or modified TRBC peptide. In certain embodiments, the constant domain is capable of forming a homodimer or a heterodimer with another constant domain. In certain embodiments, the antigen binding chain is capable of associating with a CD3ζ polypeptide. In certain embodiments, the antigen binding chain, upon binding to an antigen, is capable of activating the CD3ζ polypeptide associated to the antigen binding chain. In certain embodiments, the activation of the CD3ζ polypeptide is capable of activating an immunoresponsive cell. In certain embodiments, the TCR like fusion molecule is capable of integrating with a CD3 complex and providing HLA-independent antigen recognition. In certain embodiments, the TCR like fusion molecule replaces an endogenous TCR in a CD3/TCR complex. In certain embodiments, the extracellular antigen-binding domain of the TCR like fusion molecule is capable of dimerizing with another extracellular antigen-binding domain. In certain embodiments, the extracellular antigen-binding domain of the TCR like fusion molecule comprises a ligand for a cell-surface receptor, a receptor for a cell surface ligand, an antigen binding portion of an antibody or a fragment thereof or an antigen binding portion of a TCR. In certain embodiments, the extracellular antigen-binding domain of the TCR like fusion molecule comprises one or two immunoglobulin variable region(s). In certain embodiments, the extracellular antigen-binding domain of the TCR like fusion molecule comprises a heavy chain variable region (V_{H}) of an antibody. In certain embodiments, the extracellular antigen-binding domain of the TCR like fusion molecule comprises a light chain variable region (V_{L}) of an antibody. In certain embodiments, the extracellular antigen-binding domain of the TCR like fusion molecule is capable of dimerizing with another extracellular antigen-binding domain. In certain embodiments, the extracellular antigen-binding domain of the TCR like fusion molecule comprises a V_{H} of an antibody, wherein the V_{H} is capable of dimerizing with another extracellular antigen-binding domain comprising a V_{L} of the antibody and form a fragment variable (Fv). In certain embodiments, the extracellular antigen-binding domain of the TCR like fusion molecule comprises a V_{L} of an antibody, wherein the V_{L} is capable of dimerizing with another extracellular antigen-binding domain comprising a V_{H} of the antibody and form a fragment variable (Fv).

### 4.4. Cells

The presently disclosed subject matter provides cells comprising a presently disclosed uPAR-targeted antigen-recognizing receptor (e.g., one disclosed in Section 4.3). In certain embodiments, the cell is selected from the group consisting of cells of lymphoid lineage, cells of myeloid lineage, stem cells from which cells of lymphoid lineage can be derived, and stem cells from which cells of myeloid lineage can be derived. In certain embodiments, the cell is an immunoresponsive cell. In certain embodiments, the immunoresponsive cell is a cell of lymphoid lineage.

In certain embodiments, the cell is a cell of the lymphoid lineage. Cells of the lymphoid lineage can provide production of antibodies, regulation of cellular immune system, detection of foreign agents in the blood, detection of cells foreign to the host, and the like. Non-limiting examples of cells of the lymphoid lineage include T cells, Natural Killer (NK) cells, B cells, dendritic cells, stem cells from which lymphoid cells may be differentiated. In certain embodiments, the stem cell is a pluripotent stem cell (e.g.. embryonic stem cell).

In certain embodiments, the cell is a T cell. T cells can be lymphocytes that mature in the thymus and are chiefly responsible for cell-mediated immunity. T cells are involved in the adaptive immune system. The T cells of the presently disclosed subject matter can be any type of T cells, including, but not limited to, helper T cells, cytotoxic T cells, memory T cells (including central memory T cells, stem-cell-like memory T cells (or stem-like memory T cells), and two types of effector memory T cells: *e.g.,* TEM cells and TEMRA cells, Regulatory T cells (also known as suppressor T cells), tumor-infiltrating lymphocyte (TIL), Natural killer T cells, Mucosal associated invariant T cells, and γδ T cells. Cytotoxic T cells (CTL or killer T cells) are a subset of T lymphocytes capable of inducing the death of infected somatic or tumor cells. A patient's own T cells may be genetically modified to target specific antigens through the introduction of an antigen-recognizing receptor, *e.g.,* a CAR. In certain embodiments, the immunoresponsive cell is a T cell. The T cell can be a CD4⁺ T cell or a CD8⁺ T cell. In certain embodiments, the T cell is a CD4⁺ T cell. In certain embodiments, the T cell is a CD8⁺ T cell.

In certain embodiments, the cell is a NK cell. Natural killer (NK) cells can be lymphocytes that are part of cell-mediated immunity and act during the innate immune response. NK cells do not require prior activation in order to perform their cytotoxic effect on target cells.

Types of human lymphocytes of the presently disclosed subject matter include, without limitation, peripheral donor lymphocytes. *e.g*., those disclosed in Sadelain et al., Nat Rev Cancer (2003); 3:35-45 (disclosing peripheral donor lymphocytes genetically modified to express CARs), in Morgan, R.A., et al. 2006 Science 314:126-129 (disclosing peripheral donor lymphocytes genetically modified to express a full-length tumor antigen-recognizing T cell receptor complex comprising the α and β heterodimer), in Panelli et al., J Immunol (2000);164:495-504; Panelli et al., J lmmunol (2000);164:4382-4392 (disclosing lymphocyte cultures derived from tumor infiltrating lymphocytes (TILs) in tumor biopsies), and in Dupont et al., Cancer Res (2005);65:5417-5427; Papanicolaou et al., Blood (2003);102:2498-2505 (disclosing selectively *in* vitro-expanded antigen-specific peripheral blood leukocytes employing artificial antigen-presenting cells (AAPCs) or pulsed dendritic cells).

The cells (*e.g*., T cells) can be autologous, non-autologous (*e.g.,* allogeneic), or derived *in vitro* from engineered progenitor or stem cells.

The cells of the presently disclosed subject matter can be cells of the myeloid lineage. Non-limiting examples of cells of the myeloid lineage include monocytes, macrophages, neutrophils, dendritic cells, basophils, neutrophils, eosinophils, megakaryocytes, mast cell, erythrocyte, thrombocytes, and stem cells from which myeloid cells may be differentiated. In certain embodiments, the stem cell is a pluripotent stem cell (e.g., an embryonic stem cell or an induced pluripotent stem cell).

In certain embodiments, the presently disclosed cells are capable of modulating the tumor microenvironment. Tumors have a microenvironment that is hostile to the host immune response involving a series of mechanisms by malignant cells to protect themselves from immune recognition and elimination. This "hostile tumor microenvironment" comprises a variety of immune suppressive factors including infiltrating regulatory CD4⁺ T cells (Tregs), myeloid derived suppressor cells (MDSCs), tumor associated macrophages (TAMs), immune suppressive cytokines including TGF-β, and expression of ligands targeted to immune suppressive receptors expressed by activated T cells (CTLA-4 and PD-1). These mechanisms of immune suppression play a role in the maintenance of tolerance and suppressing inappropriate immune responses, however within the tumor microenvironment these mechanisms prevent an effective anti-tumor immune response. Collectively these immune suppressive factors can induce either marked anergy or apoptosis of adoptively transferred CAR modified T cells upon encounter with targeted tumor cells.

In certain embodiments, the cells can be transduced with the presently disclosed uPAR-targeted antigen-recognizing receptor such that the cells express the antigen-recognizing receptor.

### 4.5. Nucleic Acid Compositions and Vectors

The present discloses subject matter provides a nucleic acid encoding a presently disclosed uPAR-targeted antigen-recognizing receptor (e.g., one disclosed in Section 4.3). Further provided are nucleic acid compositions comprising the nucleic acids disclosed herein. Also provided are cells comprising such nucleic acid compositions.

In certain embodiments, the nucleic acid composition further comprises a promoter that is operably linked to the presently disclosed uPAR-targeted antigen-recognizing receptor.

In certain embodiments, the promoter is endogenous or exogenous. In certain embodiments, the exogenous promoter is selected from an elongation factor (EF)-1 promoter, a cytomegalovirus immediate-early promoter (CMV) promoter, a simian virus 40 early promoter (SV40) promoter, a phosphoglycerate kinase (PGK) promoter, and a metallothionein promoter. In certain embodiments, the promoter is an inducible promoter. In certain embodiment, the inducible promoter is selected from a NFAT transcriptional response element (TRE) promoter, a CD69 promoter, a CD25 promoter, and an IL-2 promoter.

The compositions and nucleic acid compositions can be administered to subjects or and/delivered into cells by art-known methods or as described herein. Genetic modification of a cell (*e.g.,* a T cell or a NK cell) can be accomplished by transducing a substantially homogeneous cell composition with a recombinant DNA construct. In certain embodiments, a retroviral vector (e.g.. gamma-retroviral vector or lentiviral vector) is employed for the introduction of the DNA construct into the cell. For example, a polynucleotide encoding an antigen-recognizing receptor can be cloned into a retroviral vector and expression can be driven from its endogenous promoter, from the retroviral long terminal repeat, or from a promoter specific for a target cell type of interest. Non-viral vectors may be used as well.

For initial genetic modification of a cell to include a presently disclosed uPAR-targeted antigen-recognizing receptor (*e.g*., a CAR), a retroviral vector can be employed for transduction, however any other suitable viral vector or non-viral delivery system can be used. The antigen-recognizing receptor can be constructed in a single, multicistronic expression cassette, in multiple expression cassettes of a single vector, or in multiple vectors. Examples of elements that create polycistronic expression cassette include, but is not limited to, various viral and non-viral Internal Ribosome Entry Sites (IRES, *e.g.,* FGF-1 IRES, FGF-2 IRES, VEGF IRES, IGF-II IRES, NF-κB IRES, RUNX1 IRES, p53 IRES, hepatitis A IRES, hepatitis C IRES, pestivirus IRES, aphthovirus IRES, picornavirus IRES, poliovirus IRES and encephalomyocarditis virus IRES) and cleavable linkers (*e.g..* 2A peptides , *e.g.*, P2A. T2A, E2A and F2A peptides). Combinations of retroviral vector and an appropriate packaging line are also suitable, where the capsid proteins will be functional for infecting human cells. Various amphotropic virus-producing cell lines are known, including, but not limited to, PA12 (Miller et al., (1985) Mol Cell Biol (1985);5:431-437); PA317 (Miller., et al., Mol Cell Biol (1986); 6:2895-2902); and CRIP (Danos et al., Proc Natl Acad Sci USA (1988);85:6460-6464). Non-amphotropic particles are suitable too, *e.g*., particles pseudotyped with VSVG, RD114 or GALV envelope and any other known in the art.

Possible methods of transduction also include direct co-culture of the cells with producer cells (Bregni et al., Blood (1992);80:1418-1422), or culturing with viral supernatant alone or concentrated vector stocks with or without appropriate growth factors and polycations (Xu et al., Exp Hemat (1994); 22:223-230; and Hughes et al. J Clin Invest (1992); 89:1817).

Other transducing viral vectors can be used to modify a cell. In certain embodiments, the chosen vector exhibits high efficiency of infection and stable integration and expression (*see, e.g.,* Cayouette et al., Human Gene Therapy 8:423-430, 1997; Kido et al., Current Eye Research 15:833-844, 1996; Bloomer et al.. Journal of Virology 71:6641-6649, 1997; Naldini et al., Science 272:263-267. 1996; and Miyoshi et al., Proc. Natl. Acad. Sci. U.S.A. 94:10319, 1997). Other viral vectors that can be used include, for example, adenoviral, lentiviral, and adena-associated viral vectors, vaccinia virus, a bovine papilloma virus, or a herpes virus, such as Epstein-Barr Virus (also see, for example, the vectors of M iller, Human Gene Thera (1990);15-14; Friedman, Science 244:1275-1281, 1989; Eglitis et al., BioTechniques (1988);6:608-614; Tolstoshev et al., Cur Opin Biotechnol (1990); 1:55-61; Sharp, The Lancer (1991);337:1277-78; Cornetta et al., Nucleic Acid Research and Molecular Biology 36:311-22, 1987; Anderson. Science (1984);226:401-409; Moen, Blood Cells 17:407-16. 1991; Miller et al., Biotechnol (1989);7:980-90; LeGal La Salle et al., Science (1993);259:988-90; and Johnson, Chest (1995)107:77S- 83S). Retroviral vectors are particularly well developed and have been used in clinical settings (Rosenberg et al., N Engl J Med (1990);323:370, 1990; Anderson et al., U.S. Patent. No. 5,399,346).

Non-viral approaches can also be employed for genetic modification of a cell. For example, a nucleic acid molecule can be introduced into a cell by administering the nucleic acid in the presence of lipofection (Feigner et al., Proc Natl Acad Sci U.S.A. (1987);84:7413; Ono et al., Neurosci Lett (1990);17:259; Brigham et al., Am J Med Sci (1989);298:278; Staubinger et al., Methods in Enzymol (1983);101:512, Wu et al., J Biol Chem (1988);263:14621; Wu et al., J Biol Chem (1989);264:16985), or by micro-injection under surgical conditions (Wolff et al., Science (1990);247:1465). Other non-viral means for gene transfer include transfection *in vitro* using calcium phosphate, DEAE dextran, electroporation, and protoplast fusion. Liposomes can also be potentially beneficial for delivery of DNA into a cell. Transplantation of normal genes into the affected tissues of a subject can also be accomplished by transferring a normal nucleic acid into a cultivatable cell type *ex vivo* (e.g., an autologous or heterologous primary cell or progeny thereof), after which the cell (or its descendants) are injected into a targeted tissue or are injected systemically. Recombinant receptors can also be derived or obtained using transposases or targeted nucleases (e.g. Zinc finger nucleases, meganucleases, or TALE nucleases, CRISPR). Transient expression may be obtained by RNA electroporation.

Any targeted genome editing methods can also be used to deliver a presently disclosed antigen-recognizing receptor to a cell or a subject. In certain embodiments, a CRISPR system is used to deliver a presently disclosed antigen-recognizing receptor disclosed herein. In certain embodiments, zinc-finger nucleases are used to deliver the antigen-recognizing receptor. In certain embodiments, a TALEN system is used to deliver a presently disclosed antigen-recognizing receptor.

Clustered regularly-interspaced short palindromic repeats (CRISPR) system is a genome editing tool discovered in prokaryotic cells. When utilized for genome editing, the system includes Cas9 (a protein able to modify DNA utilizing crRNA as its guide), CRISPR RNA (crRNA, contains the RNA used by Cas9 to guide it to the correct section of host DNA along with a region that binds to tracrRNA (generally in a hairpin loop form) forming an active complex with Cas9), transactivating crRNA (tracrRNA, binds to crRNA and forms an active complex with Cas9), and an optional section of DNA repair template (DNA that guides the cellular repair process allowing insertion of a specific DNA sequence). CRISPR/Cas9 often employs a plasmid to transfect the target cells. The crRNA needs to be designed for each application as this is the sequence that Cas9 uses to identify and directly bind to the target DNA in a cell. The repair template carrying CAR expression cassette need also be designed for each application, as it must overlap with the sequences on either side of the cut and code for the insertion sequence. Multiple crRNA's and the tractRNA can be packaged together to form a single-guide RNA (sgRNA). This sgRNA can be joined together with the Cas9 gene and made into a plasmid in order to be transfected into cells.

A zinc-finger nuclease (ZFN) is an artificial restriction enzyme, which is generated by combining a zinc finger DNA-binding domain with a DNA-cleavage domain. A zinc finger domain can be engineered to target specific DNA sequences which allows a zinc-finger nuclease to target desired sequences within genomes. The DNA-binding domains of individual ZFNs typically contain a plurality of individual zinc finger repeats and can each recognize a plurality of basepairs. The most common method to generate new zinc-finger domain is to combine smaller zinc-finger "modules" of known specificity. The most common cleavage domain in ZFNs is the non-specific cleavage domain from the type IIs restriction endonuclease FokI. Using the endogenous homologous recombination (HR) machinery and a homologous DNA template carrying CAR expression cassette, ZFNs can be used to insert the CAR expression cassette into genome. When the targeted sequence is cleaved by ZFNs, the HR machinery searches for homology between the damaged chromosome and the homologous DNA template, and then copies the sequence of the template between the two broken ends of the chromosome, whereby the homologous DNA template is integrated into the genome.

Transcription activator-like effector nucleases (TALEN) are restriction enzymes that can be engineered to cut specific sequences of DNA. TALEN system operates on almost the same principle as ZFNs. They are generated by combining a transcription activator-like effectors DNA-binding domain with a DNA cleavage domain. Transcription activator-like effectors (TALEs) are composed of 33-34 amino acid repeating motifs with two variable positions that have a strong recognition for specific nucleotides. By assembling arrays of these TALEs, the TALE DNA-binding domain can be engineered to bind desired DNA sequence, and thereby guide the nuclease to cut at specific locations in genome, cDNA expression for use in polynucleotide therapy methods can be directed from any suitable promoter (e.g.. the human cytomegalovirus (CMV), simian virus 40 (SV40), or metallothionein promoters), and regulated by any appropriate mammalian regulatory element or intron (e.g. the elongation factor 1a enhancer/promoter/intron structure). For example, if desired, enhancers known to preferentially direct gene expression in specific cell types can be used to direct the expression of a nucleic acid. The enhancers used can include, without limitation, those that are characterized as tissue- or cell-specific enhancers. Alternatively, if a genomic clone is used as a therapeutic construct, regulation can be mediated by the cognate regulatory sequences or, if desired, by regulatory sequences derived from a heterologous source, including any of the promoters or regulatory elements described above.

Methods for delivering the genome editing agents/systems can vary depending on the need. In certain embodiments, the components of a selected genome editing method are delivered as DNA constructs in one or more plasmids. In certain embodiments, the components are delivered via viral vectors. Common delivery methods include but is not limited to, electroporation, microinjection, gene gun, impalefection, hydrostatic pressure, continuous infusion, sonication, magnetofection, adeno-associated viruses, envelope protein pseudotyping of viral vectors, replication-competent vectors cis and trans-acting elements, herpes simplex virus, and chemical vehicles (e.g., oligonucleotides, lipoplexes, polymersomes, polyplexes, dendrimers, inorganic Nanoparticles, and cell-penetrating peptides).

### 4.6. Polypeptides

The presently disclosed subject matter provides methods for optimizing an amino acid sequence or a nucleic acid sequence by producing an alteration in the sequence. Such alterations may include certain mutations, deletions, insertions, or post-translational modifications. The presently disclosed subject matter further includes analogs of any naturally-occurring polypeptides disclosed herein (including, but not limited to, uPAR, CD8, CD28, 4-1BB, and CD3ζ,). Analogs can differ from a naturally-occurring polypeptide disclosed herein by amino acid sequence differences, by post-translational modifications, or by both. Analogs can exhibit at least about 85%, about 90%, about 91%, about 92%, about 93%, about 94%, about 95%, about 96%, about 97%, about 98%, about 99% or more homologous or identical to all or part of a naturally-occurring amino, acid sequence of the presently disclosed subject matter. The length of sequence comparison is at least 5, 10, 15 or 20 amino acid residues, e.g., at least 25, 50, or 75 amino acid residues, or more than 100 amino acid residues. Again, in an exemplary approach to determining the degree of identity, a BLAST program may be used, with a probability score between e⁻³ and e⁻¹⁰⁰ indicating a closely related sequence. Modifications include *in vivo* and *in vitro* chemical derivatization of polypeptides, *e.g*., acetylation, carboxylation, phosphorylation, or glycosylation; such modifications may occur during polypeptide synthesis or processing or following treatment with isolated modifying enzymes. Analogs can also differ from the naturally-occurring polypeptides by alterations in primary sequence. These include genetic variants, both natural and induced (for example, resulting from random mutagenesis by irradiation or exposure to ethanemethylsulfate or by site-specific mutagenesis as described in Sambrook. Fritsch and Maniatis, Molecular Cloning: A Laboratory Manual (2d ed.), CSH Press, 1989, or Ausubel et al., supra). Also included are cyclized peptides, molecules, and analogs which contain residues other than L-amino acids, e.g., D-amino acids or non-naturally occurring or synthetic amino acids, *e.g*., β or γ amino acids.

In addition to full-length polypeptides, the presently disclosed subject matter also provides fragments of any of the polypeptides disclosed herein. As used herein, the term "a fragment" means at least 5, 10, 13, or 15 amino acids. In certain embodiments, a fragment comprises at least 20 contiguous amino acids, at least 30 contiguous amino acids, or at least 50 contiguous amino acids. In certain embodiments, a fragment comprises at least 60 to 80, 100, 200, 300 or more contiguous amino acids. Fragments can be generated by methods known to those skilled in the art or may result from normal protein processing (*e.g*., removal of amino acids from the nascent polypeptide that are not required for biological activity or removal of amino acids by alternative mRNA splicing or alternative protein processing events).

### 4.7. Formulations and Administration

The presently disclosed subject matter provides compositions comprising the presently disclosed cells. In certain embodiments, the compositions are pharmaceutical compositions further comprising a pharmaceutically acceptable carrier. Compositions comprising the presently disclosed cells can be conveniently provided as sterile liquid preparations, e.g., isotonic aqueous solutions, suspensions, emulsions, dispersions, or viscous compositions, which may be buffered to a selected pH. Liquid preparations are normally casier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol, and the like) and suitable mixtures thereof.

Sterile injectable solutions can be prepared by incorporating the genetically modified cells in the required amount of the appropriate solvent with various amounts of the other ingredients, as desired. Such compositions may be in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buttering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17th edition. 1985, incorporated herein by reference, may be consulted to prepare suitable preparations, without undue experimentation.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin. According to the presently disclosed subject matter, however, any vehicle, diluent, or additive used would have to be compatible with the genetically modified cells.

The compositions can be isotonic, i.e., they can have the same osmotic pressure as blood and lacrimal fluid. The desired isotonicity of the compositions may be accomplished using sodium chloride, or other pharmaceutically acceptable agents such as dextrose, boric acid, sodium tartrate, propylene glycol or other inorganic or organic solutes. Sodium chloride can be particularly for buffers containing sodium ions.

Viscosity of the compositions, if desired, can be maintained at the selected level using a pharmaceutically acceptable thickening agent. For example, methylcellulose is readily and economically available and is easy to work with. Other suitable thickening agents include, for example, xanthan gum, carboxymethyl cellulose, hydroxypropyl cellulose, carbomer, and the like. The concentration of the thickener can depend upon the agent selected. The important point is to use an amount that will achieve the selected viscosity. Obviously, the choice of suitable carriers and other additives will depend on the exact route of administration and the nature of the particular dosage form, *e.g*., liquid dosage form (*e.g.*, whether the composition is to be formulated into a solution, a suspension, gel or another liquid form, such as a time release form or liquid-filled form).

Compositions comprising the presently disclosed cells can be provided systemically or directly to a subject for treating or ameliorating a disease or disorder. In certain embodiments, the presently disclosed cells or compositions comprising thereof are directly injected into an organ of interest (e.g., an organ affected by a neoplasia). Alternatively, the presently disclosed cells or compositions comprising thereof are provided indirectly to the organ of interest, for example, by administration into the circulatory system (*e.g*., the tumor vasculature). Expansion and differentiation agents can be provided prior to, during or after administration of the cells or compositions to increase production of cells (e.g., T cells or NK cells) *in vitro* or *in vivo.*

The presently disclosed cells can be administered in any physiologically acceptable vehicle, normally intravascularly, although they may also be introduced into bone or other convenient site where the cells may find an appropriate site for regeneration and differentiation (*e.g.*, thymus).

The quantity of cells to be administered can vary for the subject being treated. In certain embodiments, between about 10⁴ and about 10¹⁰, between about 10⁴ and about 10⁷, between about 10⁵ and about 10⁷, between about 10⁵ and about 10⁹, or between about 10⁶ and about 10⁸ of the presently disclosed cells are administered to a subject. More effective cells may be administered in even smaller numbers. Usually, at least about 1 × 10⁵ cells will be administered, eventually reaching about 1 × 10¹⁰ or more. In certain embodiments, at least about 1×10⁵, 5×10⁵, 1×10⁶, about 5×10⁶, about 1×10⁷, about 5×10⁷, about 1×10⁸, or about 5×10⁸ of the presently disclosed cells are administered to a subject. In certain embodiments, about 1×10⁶ of the presently disclosed cells are administered to a subject. The precise determination of what would be considered an effective dose can be based on factors individual to each subject, including their size, age, sex, weight, and condition of the particular subject. Dosages can be readily ascertained by those skilled in the art from this disclosure and the knowledge in the art.

The presently disclosed cells can comprise a purified population of cells. Those skilled in the art can readily determine the percentage of the presently disclosed cells in a population using various well-known methods, such as fluorescence activated cell sorting (FACS). Suitable ranges of purity in populations comprising the presently disclosed immunoresponsive cells are about 50% to about 55%, about 5% to about 60%, and about 65% to about 70%. In certain embodiments, the purity is about 70% to about 75%, about 75% to about 80%, or about 80% to about 85%. In certain embodiments, the purity is about 85% to about 90%, about 90% to about 95%, and about 95% to about 100%. Dosages can be readily adjusted by those skilled in the art (*e.g.*, a decrease in purity may require an increase in dosage). The cells can be introduced by injection, catheter, or the like.

The skilled artisan can readily determine the amount of cells and optional additives, vehicles, and/or carrier in compositions and to be administered in methods. Typically, any additives (in addition to the active cell(s) and/or agent(s)) are present in an amount of 0.001 to 50% (weight) solution in phosphate buffered saline, and the active ingredient is present in the order of micrograms to milligrams, such as about 0.0001 to about 5 wt %, about 0.0001 to about 1 wt %, about 0.0001 to about 0.05 wt% or about 0.001 to about 20 wt %, about 0.01 to about 10 wt %, or about 0.05 to about 5 wt %. For any composition to be administered to an animal or human, the followings can be determined: toxicity such as by determining the lethal dose (LD) and LD50 in a suitable animal model *e.g.*, rodent such as mouse; the dosage of the composition(s), concentration of components therein and timing of administering the composition(s), which elicit a suitable response. Such determinations do not require undue experimentation from the knowledge of the skilled artisan, this disclosure and the documents cited herein. And, the time for sequential administrations can be ascertained without undue experimentation.

In certain embodiments, the composition is a pharmaceutical composition comprising the presently disclosed cells and a pharmaceutically acceptable carrier.

Administration of the compositions can be autologous or heterologous. For example, cells can be obtained from one subject, and administered to the same subject or a different, compatible subject. Peripheral blood derived cells or their progeny (*e.g., in vivo, ex vivo* or *in vitro* derived) can be administered. When administering a presently disclosed composition (*e.g.*, a pharmaceutical composition comprising presently disclosed cells), it can be formulated in a unit dosage injectable form (solution, suspension, emulsion).

The presently disclosed cells and compositions can be administered by any method known in the art including, but not limited to, oral administration, intravenous administration, subcutaneous administration, intranodal administration, intratumoral administration, intrathecal administration, intravitreal administration , intrapleural administration, intraosseous administration, intraperitoneal administration, pleural administration, and direct administration to the subject.

### 4.8. Methods of Treatment

The presently disclosed cells and compositions comprising thereof can be used for treating or ameliorating a disease or disorder in a subject. In certain embodiments, the disease or disorder is associated with uPAR. In certain embodiments, the disease or disorder is associated with overexpression of uPAR. In certain embodiments, the disease or disorder is selected from the group consisting of tumors, senescence-associated pathologies, and tissue decline associated with aging. Non-limiting examples of senescence-associated pathologies include lung fibrosis, atherosclerosis, Alzheimer's disease, diabetes, osteoarthritis, liver fibrosis, chronic kidney disease, cardiac fibrosis, and Parkinson's disease.

In certain embodiments, the method comprises administering to a subject in need thereof the presently disclosed cells or compositions comprising thereof. In certain embodiments, the cell is a T cell. The T cell can be a CD4⁺ T cell or a CD8⁺ T cell. In certain embodiments, the T cell is a CD4⁺ T cell.

For treatment, the amount administered is an amount effective in producing the desired effect. An effective amount can be provided in one or a series of administrations. An effective amount can be provided in a bolus or by continuous perfusion.

In certain embodiments, the disease or disorder is a tumor. In certain embodiments, the presently disclosed cells and compositions can reduce tumor burden, reduce the number of tumor cells, reduce tumor size, and/or eradicate the tumor in the subject, and/or increase or lengthen survival of the subject.

Non-limiting examples of tumors include breast cancer (including triple negative breast cancer), endometrial cancer, ovarian cancer, colon cancer, rectal cancer, lung cancer (e.g., non-small cell lung cancer), stomach cancer, prostate cancer, gastric cancer, renal cancer, pancreatic cancer, blood cancer, cervical cancer, head and neck cancer, liver cancer (e.g., cholangiocarcinoma, hepatocellular carcinoma, and fibrolamaellar hepatocellular carcinoma), urotherial cancer, melanoma. and brain cancer (including glioblastoma multiforme). In certain embodiments, the blood cancer is selected from the group consisting of acute lymphoblastic leukemia (ALL), chronic lymphocytic leukemia (CLL), acute myeloid leukemia (AML), myclofibrosis, polycythemia vera, myelodysplastic syndrome, erythroleukemia. In certain embodiments, the tumor is cancer. In certain embodiments, the cancer is a relapsed or refractory cancer. In certain embodiments, the cancer is resistant to a cancer therapy, e.g., chemotherapy.

Furthermore, the presently disclosed subject matter provides methods of increasing production of an immune-activating cytokine in response to a tumor cell in a subject. In certain embodiments, the method comprises administering to the subject the presently disclosed cells and compositions. Non-limiting examples of immune-activating cytokine include granulocyte macrophage colony stimulating factor (GM-CSF), IFN-α, IFN-β, IFN-γ, TNF-α, IL-1, IL-2, IL-3, IL-6, IL-11, IL-7, IL-8, 1.1.-12, IL-15, IL-21, interferon regulatory factor 7 (IRF7), CCL1, CCL2, CCL3, CCLS, CCL7, CCL8, CCL13, CCL16, CXCL1, CXCL3, CXCL5, CXCL9, CXCL10, and combinations thereof.

In certain embodiments, the disease or disorder is a senescence-associated pathology. In certain embodiments, the subject exhibits an increased accumulation of senescent cells compared to that observed in a healthy control subject. In certain embodiments, the senescence-associated pathology is selected from the group consisting of lung fibrosis, atherosclerosis, Alzheimer's disease, diabetes, liver fibrosis, chronic kidney disease, osteoarthritis, cardiac fibrosis, and Parkinson's disease. certain embodiments, the senescent cells exhibit a Senescence-Associated Secretory Phenotype (SASP). The Senescence-Associated Secretory Phenotype may be induced by replication, an oncogene (*e.g*., HRASG12D, NRAsG12D NRAsG12D, etc.), radiation, chemotherapy, or a drug (e.g., Cdk4/6 inhibitors, MEK inhibitors, a chemotherapy drug, etc.). Non-limiting examples of MEK inhibitors include trametinib, cobimetinib, binimetinib, selumetinib, PD-325901, TAK-733, CI-1040 (PD 184352), PD0325901, MEK162, AZD8330, GDC-0623, refametinib, pimasertib, R04987655, R05126766, WX-554, HL-085, ClnQ-03, G-573, PD184161, PD318088, PD98059, R05068760, U0126, and SL327. Non-limiting examples of CDK4/6 inhibitors include palbociclib, ribociclib, and abemaciclib. Non-limiting examples of chemotherapy drugs include cisplatin, doxorubicin, cyclophosphamide, and etoposide.

In certain embodiments, the presently disclosed methods for treating or ameliorating a disease or disorder further comprise administering to the subject a tumor specific monoclonal antibody, wherein the subject is receiving/has received a senescence-inducing therapy (e.g., chemotherapy). In certain embodiments, the tumor specific monoclonal antibody is administered subsequent to the administration of the presently disclosed cells or compositions comprising thereof. Non-limiting examples of specific senescence-inducing therapies include doxorubicin, ionizing radiation therapy, combination therapy with MEK inhibitors and CDK4/6 inhibitors, combination therapy with CDC7 inhibitors and mTOR inhibitors, and the like. Examples of CDK4/6 inhibitors include palbociclib, ribociclib, and abemaciclib. Non-limiting examples of MEK inhibitors include trametinib, cobimetinib, binimetinib, selumetinib, PD-325901, TAK-733, C1-1040 (PD184352), PD0325901, MEK162, AZD8330, GDC-0623, refametinib, pimasertib, R04987655, R05126766, WX-554, HL-085, CInQ-03. G-573, PD184161, PD318088, PD98059, R05068760, U0126, and SL327. Non-limiting examples of mTOR inhibitors include rapamycin, sertraline, sirolimus, everolimus, temsirolimus, ridaforolimus, and deforolimus. Examples of CDC7 inhibitors include TAK-931, PHA-767491, XL413, 1H-pyrrolo[2,3-b]pyridines, 2,3-dihydrothieno[3,2-d]pyrimidin-4(1H)-ones, furanone derivatives, trisubstituted thiazoles, pyrrolopyridinones, and the like.

In certain embodiments, the tumor specific monoclonal antibody is administered subsequent to the administration of the cells or compositions comprising thereof.

In certain embodiments, the subject is human.

In certain embodiments, the presently disclosed methods for treating or ameliorating a disease or disorder further comprise administering to the subject a cancer therapy. In certain embodiments, the cancer therapy is selected from the group consisting of chemotherapy, radiation therapy, immunotherapy, monoclonal antibodies, anti-cancer nucleic acids or proteins, anti-cancer viruses or microorganisms, and any combinations thereof.

In certain embodiments, the presently disclosed methods for treating or ameliorating a disease or disorder further comprise administering to the subject a cytokine. In certain embodiments, the cytokine is administered prior to, during, or subsequent to the administration of the cells or compositions comprising thereof. In certain embodiments, the cytokine is selected from the group consisting of interferon a, interferon (3, interferon y, complement C5a, IL-2, TNF-α, CD4OL, IL12, IL-23, IL15, IL17, CCL1, CCL11, CCL12, CCL13, CCL14-1, CCL14-2, CCL14-3, CCL15-1, CCL15-2, CCL16, CCL17, CCL18, CCL19, CCL19. CCL2. CCL20, CCL21, CCL22, CCL23-1, CCL23-2, CCL24, CCL25-1, CCL25-2, CCL26, CCL27, CCL28, CCL3, CCL3L1, CCL4, CCL4L1, CCL5, CCL6, CCL7, CCL8, CCL9, CCR10, CCR2, CCR5, CCR6, CCR7, CCR8, CCRL1, CCRL2, CX3CL1, CX3CR, CXCL1, CXCL10, CXCL11, CXCL12, CXCL13, CXCL14, CXCL15, CXCL16, CXCL2, CXCL3, CXCL4, CXCL5, CXCL6, CXCL7, CXCLS, CXCL9, CXCL9, CXCR1, CXCR2, CXCR4, CXCR5, CXCR6, CXCR7 and XCL2.

In certain embodiments, the chemotherapy comprises administering to the subject a chemotherapeutic agent. Non-limiting examples of chemotherapeutic agents include nitrogen mustards, ethylenimine derivatives, alkyl sulfonates, nitrosoureas, gemcitabine, triazenes, folic acid analogs, anthracyclines, taxanes. COX-2 inhibitors, pyrimidine analogs, purine analogs, antibiotics, enzyme inhibitors, epipodophyllotoxins, platinum coordination complexes, vinca alkaloids, substituted ureas, methyl hydrazine derivatives, adretiocortical suppressants, hormone antagonists, endostatin, taxols, camptothecins, SN-38, doxorubicin, doxorubicin analogs, antimetabolites, alkylating agents, antimitotics, anti-angiogenic agents, tyrosine kinase inhibitors, mTOR inhibitors, heat shock protein (HSP90) inhibitors, proteosome inhibitors, HDAC inhibitors, pro-apoptotic agents, methotrexate and CPT-11.

In certain embodiments, the disease or disorder is lung fibrosis, and the method further comprises sequentially, separately, or simultaneously administering to the subject at least one therapy selected from the group consisting of pirfenidone, sintedanib, oxygen therapy, corticosteroids (e.g., prednisone), mycophenolate mofetil/mycophenolic acid, and azathioprine.

In certain embodiments, the disease or disorder is atherosclerosis, and the method further comprises sequentially, separately, or simultaneously administering to the subject at least one therapy selected from the group consisting of statins (e.g., Atorvastatin, Fluvastatin, Lovastatin, Pitavastatin, Pravastatin, Rosuvastatin calcium, Simvastatin), fibrates (e.g., Gemfibrozil, Fenofibrate), niacin, ezetimibe, bile acid sequestrants (e.g., cholestyramine, colestipol, colesevclam), proprotein convertase subtilisin kexin type 9 (PCSK9) inhibitors, anti-platelet medications (e.g., aspirin, Clopidogrel, Ticagrelor, warfarin, prasugral), beta blockers. Angiotensin-converting enzyme (ACE) inhibitors, calcium channel blockers. and diuretics.

In certain embodiments, the disease or disorder is Alzheimer's disease, and the method further comprises sequentially, separately, or simultaneously administering to the subject at least one therapy selected from the group consisting of donepezil, galantamine, memantine, rivastigmine, memantine extended-release and donepezil (Namzaric), aducanumab, solanezumab, insulin, verubecestat, AADvac1, CSP-1103, and intepirdine.

In certain embodiments, the disease or disorder is diabetes, and the method further comprises sequentially, separately, or simultaneously administering to the subject at least one therapy selected from the group consisting of insulin, metformin, amylin analogs, glucagon, sulfonylureas (e.g., glimepiride, glipizide, glyburide, chlorpropamide, tolazamide, tolbutamide), meglitinides (e.g., nateglinide, repaglinide), thiazolidinediones (e.g., pioglitazone, rosiglitazone), alpha-glucosidase inhibitors (e.g., acarbose, miglitol), dipeptidyl peptidase (DPP4) inhibitors (e.g., alogliptin, linagliptin, sitagliptin, saxagliptin), sodium-glucose co-transporter 2 (SGLT2) inhibitors (e.g., canagliflozin, dapagliflozin, empaglifiozin, ertugliflozin), and increlin mimetics (e.g., exenatide, liraglutide, dulaglutide, lixisenatide, semaglutide).

In certain embodiments, the disease or disorder is osteoarthritis, and the method further comprises sequentially, separately, or simultaneously administering to the subject at least one therapy selected from the group consisting of analgesics (e.g., acetaminophen, tramadol, oxycodone, hydrocodone), nonsteroidal anti-inflammatory drugs (e.g., aspirin, ibuprofen, naproxen, celecoxib), cyclooxygenase-2 inhibitors, corticosteroids, and hyaluronic acid.

In certain embodiments, the disease or disorder is liver fibrosis, and the method further comprises sequentially, separately, or simultaneously administering to the subject at least one therapy selected from the group consisting of ACE inhibitors (e.g., benazepril, Lisinopril, Ramipril), a-Tocopherol, interferon-a, PPAR-antagonists, colchicine, corticosteroids, endothelin inhibitors, interleukin-10, pentoxifylline, phosphatidylcholine, S-adenosyl-methionine, and TGF-131 inhibitors.

In certain embodiments, the disease or disorder is chronic kidney disease, and the method further comprises sequentially, separately, or simultaneously administering to the subject at least one therapy selected from the group consisting of ACE inhibitors (e.g., benazepril, Lisinopril, Ramipril), statins (e.g., Atorvastatin, Fluvastatin, Lovastatin. Pitavastatin, Pravastatin, Rosuvastatin calcium, Simvastatin), furosemide, erythropoietin, phosphate binders (e.g., calcium acetate, calcium carbonate), colecalciferol, ergocalciferol, and cyclophosphamide.

Further modification can be introduced to the uPAR-specific CAR-expressing engineered immune cells (e.g., T cells) to avert or minimize the risks of immunological complications (known as "malignant T-cell transformation"), e.g., graft versus-host disease (GvHD). Modification of the engineered immune cells can include engineering a suicide gene into the uPAR-specific CAR-expressing T cells. Suitable suicide genes include, but are not limited to, Herpes simplex virus thymidine kinase (hsv-tk), inducible Caspase 9 Suicide gene (iCasp-9), and a truncated human epidermal growth factor receptor (EGFRt) polypeptide. In certain embodiments, the suicide gene is an EGFRt polypeptide. The EGFRt polypeptide can enable T cell elimination by administering anti-EGFR monoclonal antibody (e.g., cetuximab). EGFRt can be covalently joined to the C-terminus of the intracellular domain of the uPAR-specific CAR. The suicide gene can be included within the vector comprising nucleic acids encoding the presently disclosed uPAR-specific CARs. The incorporation of a suicide gene into the a presently disclosed uPAR-specific CAR gives an added level of safety with the ability to eliminate the majority of CAR T cells within a very short time period. A presently disclosed engineered immune cell (e.g., a T cell) incorporated with a suicide gene can be pre-emptively eliminated at a given time point post CAR T cell infusion, or eradicated at the earliest signs of toxicity.

### 4.9. Kits

The presently disclosed subject matter provides kits for or ameliorating a disease or disorder in a subject. In certain embodiments, the kit comprises the presently disclosed cells or a composition comprising thereof. In certain embodiments, the kit comprises a sterile container; such containers can be boxes, ampules, bottles, vials, tubes, bags, pouches, blister-packs, or other suitable container forms known in the art. Such containers can be made of plastic, glass, laminated paper, metal foil, or other materials suitable for holding medicaments. In certain non-limiting embodiments, the kit includes a nucleic acid molecule encoding a presently disclosed uPAR-targeted antigen-recognizing receptor (e.g., a CAR).

If desired, the cells and/or nucleic acid molecules are provided together with instructions for administering the cells or nucleic acid molecules to a subject having or at risk of developing a disease or disorder. The instructions generally include information about the use of the composition for the treatment and/or prevention of a tumor or neoplasm. In certain embodiments, the instructions include at least one of the following: description of the therapeutic agent; dosage schedule and administration for treatment or prevention of a tumor or neoplasm: precautions; warnings; indications; counter-indications: over-dosage information; adverse reactions; animal pharmacology; clinical studies; and/or references. The instructions may be printed directly on the container (when present), or as a label applied to the container, or as a separate sheet, pamphlet, card, or folder supplied in or with the container.

### 4.10. Exemplary Embodiments

A1. In certain non-limiting embodiments, the presently disclosed subject matter provides an antigen-recognizing receptor, comprising an extracellular antigen-binding domain, a transmembrane domain, and an intracellular signaling domain, wherein the extracellular antigen-binding domain specifically binds to uPAR.

A2. The foregoing antigen-recognizing receptor of A1, wherein the extracellular antigen-binding domain is a single-chain variable fragment (scFv).

A3. The foregoing antigen-recognizing receptor of A2. wherein the extracellular antigen-binding domain is a human scFv.

A4. The foregoing antigen-recognizing receptor of A1. wherein the extracellular antigen-binding domain is a Fab, which is optionally crosslinked.

A5. The foregoing antigen-recognizing receptor of A1, wherein the extracellular antigen-binding domain is a F(ab)₂.

A6. The foregoing antigen-recognizing receptor of any one of A2-A5, wherein one or more of the scFv, Fab and F(ab)₂ are comprised in a fusion protein with a heterologous sequence to form the extracellular antigen-binding domain.

A7. The foregoing antigen-recognizing receptor of any one of A1-A6, wherein the extracellular antigen-binding domain comprises a heavy chain variable region comprising: (a) a CDR1 comprising the amino acid sequence set forth in SEQ ID NO: 1 or a conservative modification thereof, a CDR2 comprising the amino acid sequence set forth in SEQ ID NO: 2 or a conservative modification thereof, and a CDR3 comprising the amino acid sequence set forth in SEQ ID NO: 3 or a conservative modification thereof; (b) a CDR1 comprising the amino acid sequence set forth in SEQ ID NO: 11 or a conservative modification thereof, a CDR2 comprising the amino acid sequence set forth in SEQ ID NO: 2 or a conservative modification thereof, and a CDR3 comprising the amino acid sequence set forth in SEQ ID NO: 12 or a conservative modification thereof; (c) a CDR1 comprising the amino acid sequence set forth in SEQ ID NO: 19 or a conservative modification thereof, a CDR2 comprising the amino acid sequence set forth in SEQ ID NO: 2 or a conservative modification thereof, and a CDR3 comprising the amino acid sequence set forth in SEQ ID NO: 20 or a conservative modification thereof; (d) a CDR1 comprising the amino acid sequence set forth in SEQ ID NO: 19 or a conservative modification thereof, a CDR2 comprising the amino acid sequence set forth in SEQ ID NO: 27 or a conservative modification thereof, and a CDR3 comprising the amino acid sequence set forth in SEQ ID NO: 28 or a conservative modification thereof; (c) a CDR1 comprising the amino acid sequence set forth in SEQ ID NO: 35 or a conservative modification thereof, a CDR2 comprising the amino acid sequence set forth in SEQ ID NO: 36 or a conservative modification thereof, and a CDR3 comprising the amino acid sequence set forth in SEQ ID NO: 37 or a conservative modification thereof; (f) a CDR1 comprising the amino acid sequence set forth in SEQ ID NO: 45 or a conservative modification thereof, a CDR2 comprising the amino acid sequence set forth in SEQ ID NO: 46 or a conservative modification thereof; and a CDR3 comprising the amino acid sequence set forth in SEQ ID NO: 47 or a conservative modification thereof; (g) a CDR1 comprising the amino acid sequence set forth in SEQ ID NO: 54 or a conservative modification thereof, a CDR2 comprising the amino acid sequence set forth in SEQ ID NO: 55 or a conservative modification thereof, and a CDR3 comprising the amino acid sequence set forth in SEQ ID NO: 56 or a conservative modification thereof, (h) a CDR1 comprising the amino acid sequence set forth in SEQ ID NO: 63 or a conservative modification thereof, a CDR2 comprising the amino acid sequence set forth in SEQ ID NO: 64 or a conservative modification thereof, and a CDR3 comprising the amino acid sequence set forth in SEQ ID NO: 65 or a conservative modification thereof, (i) a CDR1 comprising the amino acid sequence set forth in SEQ ID NO: 73 or a conservative modification thereof, a CDR2 comprising the amino acid sequence set forth in SEQ ID NO: 74 or a conservative modification thereof, and a CDR3 comprising the amino acid sequence set forth in SEQ ID NO: 75 or a conservative modification thereof, (j) a CDR 1 comprising the amino acid sequence set forth in SEQ ID NO: 45 or a conservative modification thereof, a CDR2 comprising the amino acid sequence set forth in SEQ ID NO: 82 or a conservative modification thereof, and a CDR3 comprising the amino acid sequence set forth in SEQ ID NO: 83 or a conservative modification thereof, (k) a CDR1 comprising the amino acid sequence set forth in SEQ ID NO: 90 or a conservative modification thereof, a CDR2 comprising the amino acid sequence set forth in SEQ ID NO: 91 or a conservative modification thereof, and a CDR3 comprising the amino acid sequence set forth in SEQ ID NO: 92 or a conservative modification thereof, (l) a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 99 or a conservative modification thereof; or (m) a CDR1 comprising the amino acid sequence set forth in SEQ ID NO: 105 or a conservative modification thereof, a CDR2 comprising the amino acid sequence set forth in SEQ ID NO: 106 or a conservative modification thereof, and a CDR3 comprising the amino acid sequence set forth in SEQ ID NO: 107 or a conservative modification thereof.

A8. The foregoing antigen-recognizing receptor of any one of A1-A7, wherein the extracellular antigen-binding domain comprises a light chain variable region comprising: (a) a CDR1 comprising the amino acid sequence set forth in SEQ ID NO: 4 or a conservative modification thereof, a CDR2 comprising the amino acid sequence set forth in SEQ ID NO: 5 or a conservative modification thereof, and a CDR3 comprising the amino acid sequence set forth in SEQ ID NO: 6 or a conservative modification thereof; (b) a CDR1 comprising the amino acid sequence set forth in SEQ ID NO: 13 or a conservative modification thereof, a CDR2 comprising the amino acid sequence set forth in SEQ ID NO: 5 or a conservative modification thereof, and a CDR3 comprising SEQ ID NO: 14 or a conservative modification thereof; (c) a CDR1 comprising the amino acid sequence set forth in SEQ ID NO: 21 or a conservative modification thereof, a CDR2 comprising the amino acid sequence set forth in SEQ ID NO: 5 or a conservative modification thereof, and a CDR3 comprising the amino acid sequence set forth in SEQ ID NO: 22 or a conservative modification thereof; (d) a CDR1 comprising the amino acid sequence set forth in SEQ ID NO: 29 or a conservative modification thereof, a CDR2 comprising SEQ ID NO: 5 or a conservative modification thereof, and a CDR3 comprising the amino acid sequence set forth in SEQ ID NO: 30 or a conservative modification thereof, (e) a CDR1 comprising the amino acid sequence set forth in SEQ ID NO: 38 or a conservative modification thereof, a CDR2 comprising the amino acid sequence set forth in SEQ ID NO: 39 or a conservative modification thereof, and a CDR3 comprising the amino acid sequence set forth in SEQ ID NO: 40 or a conservative modification thereof; (f) a CDR1 comprising the amino acid sequence set forth in SEQ ID NO: 48 or a conservative modification thereof, a CDR2 comprising the amino acid sequence set forth in SEQ ID NO: 5 or a conservative modification thereof, and a CDR3 comprising the amino acid sequence set forth in SEQ ID NO: 49 or a conservative modification thereof; (g) a CDR1 comprising the amino acid sequence set forth in SEQ ID NO: 57 or a conservative modification thereof, a CDR2 comprising the amino acid sequence set forth in SEQ ID NO: 5 or a conservative modification thereof, and a CDR3 comprising the amino acid sequence set forth in SEQ ID NO: 58 or a conservative modification thereof; (h) a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 68 or a conservative modification thereof; (i) a CDR1 comprising the amino acid sequence set forth in SEQ ID NO: 76 or a conservative modification thereof, a CDR2 comprising the amino acid sequence set forth in SEQ ID NO: 67 or a conservative modification thereof, and a CDR3 comprising the amino acid sequence set forth in SEQ ID NO: 77 or a conservative modification thereof; (j) a CDR1 comprising the amino acid sequence set forth in SEQ ID NO: 84 or a conservative modification thereof, a CDR2 comprising the amino acid sequence set forth in SEQ ID NO: 67 or a conservative modification thereof, and a CDR3 comprising the amino acid sequence set forth in SEQ ID NO: 85 or a conservative modification thereof; (k) a CDR1 comprising the amino acid sequence set forth in SEQ ID NO: 93 or a conservative modification thereof, a CDR2 comprising the amino acid sequence set forth in SEQ ID NO: 67 or a conservative modification thereof, and a CDR3 comprising the amino acid sequence set forth in SEQ ID NO: 94 or a conservative modification thereof; or (1) a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 100 or a conservative modification thereof.

A9. The foregoing antigen-recognizing receptor of any one of A 1-A8, wherein the extracellular antigen-binding domain comprises: (a) a heavy chain variable region comprising a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; and a light chain variable region comprising a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6; (b) a heavy chain variable region comprising a CDR1 comprising the amino acid sequence set forth in SEQ ID NO: 11, a CDR2 comprising the amino acid sequence set forth in SEQ ID NO: 2, and a CDR3 comprising the amino acid sequence set forth in SEQ ID NO: 12; and a light chain variable region comprising a CDR 1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14; (c) a heavy chain variable region comprising a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; and a light chain variable region comprising a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22; (d) a heavy chain variable region comprising a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28; and a light chain variable region comprising a CDR 1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30; (e) a heavy chain variable region comprising a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; and a light chain variable region comprising a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40; (f) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; and a light chain variable region comprising a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49; (g) a heavy chain variable region comprising a CDR1 comprising the amino acid sequence set forth in SEQ ID NO: 54, a CDR2 comprising the amino acid sequence set forth in SEQ ID NO: 55, and a CDR3 comprising the amino acid sequence set forth in SEQ ID NO: 56; and a light chain variable region comprising a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58; th) a heavy chain variable region comprising a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65; and a light chain variable region comprising a CDR 1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68; (i) a heavy chain variable region comprising a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; and a light chain variable region comprising a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77; (j) a heavy chain variable region comprising a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83; and a light chain variable region comprising a CDRI comprising the amino acid sequence set forth in SEQ ID NO: 8-4, a CDR2 comprising the amino acid sequence set forth in SEQ ID NO: 67, and a CDR3 comprising the amino acid sequence set forth in SEQ ID NO: 85; (k) a heavy chain variable region comprising a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92; and a light chain variable region comprising a COR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94; or (l) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.

A10. The foregoing antigen-recognizing receptor of any one of A1-A9, wherein the extracellular antigen-binding domain comprises a heavy chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108.

A11. The foregoing antigen-recognizing receptor of any one of A1-A10, wherein the extracellular antigen-binding domain comprises a heavy chain variable region comprising the amino acid sequence set forth in SEQ ID NO: 7. SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108.

A12. The foregoing antigen-recognizing receptor of any one of A1-A11, wherein the extracellular antigen-binding domain comprises a light chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 961%, about 97%, about 98% or about 99% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

A13. The foregoing antigen-recognizing receptor of any one of A1-A12, wherein the extracellular antigen-binding domain comprises a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

A14. The foregoing antigen-recognizing receptor of any one of A 1-A13, wherein the extracellular antigen-binding domain comprises: (a) a heavy chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence selected set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95. SEQ ID NO: 101, or SEQ ID NO: 108; and (b) a light chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

A15. The foregoing antigen-recognizing receptor of any one of A1-A14, wherein the extracellular antigen-binding domain comprises: (a) a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59. SEQ ID NO: 69, SEQ ID NO: 78, SEQ ID NO: 86, SEQ ID NO: 95, SEQ ID NO: 101, or SEQ ID NO: 108; and (b) a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

A16. The foregoing antigen-recognizing receptor of A15, wherein the extracellular antigen-binding domain comprises: (a) a heavy chain variable region comprising the amino acid sequence set forth in SEQ ID NO: 7, and a light chain variable region comprising the amino acid sequence set forth in SEQ ID NO: 8; (b) a heavy chain variable region comprising the amino acid sequence set forth in SEQ ID NO: 15, and a light chain variable region comprising the amino acid sequence set forth in SEQ ID NO: 16; (c) a heavy chain variable region comprising the amino acid sequence set forth in SEQ ID NO: 23, and a light chain variable region comprising the amino acid sequence set forth in SEQ ID NO: 24; (d) a heavy chain variable region comprising the amino acid sequence set forth in SEQ ID NO: 31, and a light chain variable region comprising the amino acid sequence set forth in SEQ ID NO: 32; (e) a heavy chain variable region comprising the amino acid sequence set forth in SEQ ID NO: 41, and a light chain variable region comprising the amino acid sequence set forth in SEQ ID NO: 42; (f) a heavy chain variable region comprising the amino acid sequence set forth in SEQ ID NO: 50, and a light chain variable region comprising the amino acid sequence set forth in SEQ ID NO: 51; (g) a heavy chain variable region comprising the amino acid sequence set forth in SEQ ID NO: 59, and a light chain variable region comprising the amino acid sequence set forth in SEQ ID NO: 60; (h) a heavy chain variable region comprising the amino acid sequence set forth in SEQ ID NO: 69. and a light chain variable region comprising the amino acid sequence set forth in SEQ ID NO: 70; (i) a heavy chain variable region comprising the amino acid sequence set forth in SEQ ID NO: 78, and a light chain variable region comprising the amino acid sequence set forth in SEQ ID NO: 79; (j) a heavy chain variable region comprising the amino acid sequence set forth in SEQ ID NO: 86, and a light chain variable region comprising the amino acid sequence set forth in SEQ ID NO: 87; (k) a heavy chain variable region comprising the amino acid sequence set forth in SEQ ID NO: 95, and a light chain variable region comprising the amino acid sequence set forth in SEQ ID NO: 96; or (l) a heavy chain variable region comprising the amino acid sequence set forth in SEQ ID NO: 101, and a light chain variable region comprising the amino acid sequence set forth in SEQ ID NO: 102.

A17. The foregoing antigen-recognizing receptor of any one of A1-A16, wherein the extracellular antigen-binding domain comprises a linker between a heavy chain variable region and a light chain variable region of the extracellular antigen-binding domain.

A18. The foregoing antigen-recognizing receptor of A17, wherein the linker comprises or consists of the amino acid sequence set forth in SEQ ID NO: 110, SEQ ID NO: 111, SEQ ID NO: 112, SEQ ID NO: 113, SEQ ID NO: 114, or SEQ ID NO: 115.

A19. The foregoing antigen-recognizing receptor of any one of A1-A18, wherein the extracellular antigen-binding domain comprises a signal peptide that is covalently joined to the 5' terminus of the extracellular antigen-binding domain.

A20. The foregoing antigen-recognizing receptor of any one of A1-A19, wherein the transmembrane domain comprises a CD8 polypeptide, a CD28 polypeptide, a CD3ζ polypeptide, a CD4 polypeptide, a 4-1BB polypeptide, an OX40 polypeptide, an ICOS polypeptide, a CTLA-4 polypeptide, a PD-1 polypeptide, a LAG-3 polypeptide, a 2B4 polypeptide, a BTLA polypeptide, or a combination thereof.

A21. The foregoing antigen-recognizing receptor of any one of A1-A20, wherein the intracellular signaling domain comprises a CD3ζ polypeptide.

A22. The foregoing antigen-recognizing receptor of any one of A1-A21, wherein the intracellular signaling domain further comprises at least one co-stimulatory signaling region.

A23. The foregoing antigen-recognizing receptor of A22, wherein the at least one co-stimulatory signaling region comprises a CD28 polypeptide, a 4-1BB polypeptide, an OX40 polypeptide, an ICOS polypeptide, a DAP-10 polypeptide, or a combination thereof.

A24. The foregoing antigen-recognizing receptor of any one of A1-A23, wherein the antigen-recognizing receptor is a chimeric antigen receptor (CAR), a T-cell Receptor (TCR), or a T-cell like fusion protein.

A25. The foregoing antigen-recognizing receptor of any one of A1-A24, wherein the antigen-recognizing receptor is a CAR.

A26. The foregoing antigen-recognizing receptor of any one of A I-A25, wherein the antigen-recognizing receptor is recombinantly expressed.

A27. The foregoing antigen-recognizing receptor of any one of A1-A26, wherein the antigen-recognizing receptor is expressed from a vector.

A28. The foregoing antigen-recognizing receptor of A27, wherein the vector is a γ-retroviral rector.

B1. In certain non-limiting embodiments, the presently disclosed subject matter provides a cell comprising the antigen-recognizing receptor of any one of A 1-A28.

B2. The foregoing cell of B1, wherein the cell is transduced with the antigen-recognizing receptor.

B3. The foregoing cell of B1 or B2, wherein the antigen-recognizing receptor is constitutively expressed on the surface of the cell.

B4. The foregoing cell of any one of B1-B4, wherein the cell is an immunoresponsive cell.

B5. The foregoing cell of any one of B1-B4, wherein the cell is a cell of the lymphoid lineage or a cell of the myeloid lineage.

B6. The foregoing cell of any one of B1-B5, wherein the cell is selected from the group consisting of a T cell, a Natural Killer (NK) cell, and a stem cell from which a lymphoid cell may be differentiated.

B7. The foregoing cell of any one of B1-B6, wherein the cell is a T cell.

B8. The foregoing cell of B6 or B7, wherein the T cell is a cytotoxic T lymphocyte (CTL) or a regulatory T cell.

B9. The foregoing cell of B6, wherein the stem cell is a pluripotent stem cell.

B10. The foregoing cell of B9, wherein the pluripotent stem cell is an embryoid stem cell or an induced pluripotent stem cell.

C1. In certain non-limiting embodiments, the presently disclosed subject matter provides a nucleic acid encoding the antigen-recognizing receptor of any one of A1-A28.

D1. In certain non-limiting embodiments, the presently disclosed subject matter provides a vector comprising the nucleic acid of C1.

D2. The foregoing vector of D1, wherein the vector is a γ-retroviral rector.

E1. In certain non-limiting embodiments, the presently disclosed subject matter provides a host cell expressing the nucleic acid of C1 or the vector of D1 or D2.

E2. The foregoing host cell of E1, wherein the host cell is a T cell.

F1. In certain non-limiting embodiments, the presently disclosed subject matter provides a composition comprising the cell of any one of B1-B10.

F2. The foregoing composition of F1, which is a pharmaceutical composition further comprising a pharmaceutically acceptable carrier.

G1. In certain non-limiting embodiments, the presently disclosed subject matter provides a method of treating or ameliorating a disease or disorder in a subject, comprising administering to the subject the cell of any one of B1-B10, or the composition of F1 or F2.

G2. The foregoing method of G1, wherein the disease or disorder is selected from the group consisting of tumors, senescence-associated pathologies, and tissue decline associated with aging.

G3. The foregoing method of G2, wherein the disease or disorder is a senescence-associated pathology.

G4. The foregoing method of G3, wherein the senescence-associated pathology is selected from the group consisting of lung fibrosis, atherosclerosis, Alzheimer's disease, diabetes, osteoarthritis, liver fibrosis, chronic kidney disease, cardiac fibrosis, and Parkinson's disease.

G5. The foregoing method of G2. wherein the disease or disorder is a tumor.

G6. The foregoing method of G5, wherein the tumor is selected from the group consisting of breast cancer, endometrial cancer, ovarian cancer, colon cancer, rectal cancer, lung cancer, stomach cancer, prostate cancer, gastric cancer, renal cancer, pancreatic cancer, blood cancer, cervical cancer, head and neck cancer, liver cancer, urotherial cancer, melanoma, and brain cancer

G7. The foregoing method of G6, wherein the blood cancer is selected from the group consisting of acute lymphoblastic leukemia (ALL), chronic lymphocytic leukemia (CLL), acute myeloid leukemia (AML), myelofibrosis, polycythemia vera, myelodysplastic syndrome, and erythroleukemia.

G8. The foregoing method of any one of G5-G7, wherein the tumor is cancer.

H1. In certain non-limiting embodiments, the presently disclosed subject matter provides a method of increasing production of an immune-activating cytokine in response to a tumor cell in a subject, comprising administering to the subject the cell of any one of B1-B10, or the composition of F1 or F2.

H2. The foregoing method of H1, wherein the immune-activating cytokine is selected from the group consisting of granulocyte macrophage colony stimulating factor (GM-CSF), IFN-α, IFN-β, IFN-γ, TNF-α, IL-1, IL-2, IL-3, IL-6, IL-11, IL-7, IL-8, IL-12, IL-15, IL-21, interferon regulatory factor 7 (IRF7), CCL1, CCL2, CCL3, CCL5, CCL7, CCL8, CCL13, CCL16, CXCL1, CXCL3, CXCL5. CXCL9, CXCL10, and combinations thereof.

H3. The foregoing method of any one of G1-G8 or H1-H2, wherein the subject is a human.

I1. In certain non-limiting embodiments, the presently disclosed subject matter provides a kit for treating or ameliorating a disease or disorder in a subject, and/or increasing production of an immune-activating cytokine in response to a tumor cell in a subject, comprising the cell of any one of B1-B10, the nucleic acid of claim C1, or the composition of claim F1 or F2.

12. The foregoing kit of I1, wherein the kit further comprises written instructions for using the cell or composition for treating or ameliorating a disease or disorder in a subject, and/or increasing production of an immune-activating cytokine in response to a tumor cell in a subject.

J1. In certain non-limiting embodiments, the presently disclosed subject matter provides a method for producing a uPAR-targeted antigen-recognizing receptor of any one of A1-A28, comprising introducing into the cell a nucleic acid that encodes the antigen-recognizing receptor.

### 5. EXAMPLES

The following examples are put forth so as to provide those of ordinary skill in the art with a complete disclosure and description of how to make and use the antibodies, multi-specific antibodies, compositions comprising thereof, screening, and therapeutic methods of the presently disclosed subject matter, and are not intended to limit the scope of what the inventors regard as their presently disclosed subject matter. It is understood that various other embodiments may be practiced, given the general description provided above.

### Example 1 - Generation of anti-uPAR scFvs

A portion of uPAR corresponding to the extracellular domain and amino acids His 65-Ala 265, was recombinantly produced as a soluble protein with a polyhistidine tag for purification. The extracellular domain of murine uPAR (Thr 67-Arg 267) was also produced with a polyhistidine tag to screen antibodies for cross-species reactivity.

A proprietary naive, semi-synthetic scFv phage display library was screened for antibodies that bind to the uPAR protein by using standard solid phase phage display panning techniques. Briefly, recombinant uPAR was immobilized on a polystyrene surface followed by blocking with about 5% milk and incubation with the phage library. Subsequent washing, elution and phage amplification steps were performed to complete each round of biopanning. Three rounds of panning were completed using amplified uPAR binder-enriched phage pools from the previous round of panning as input for subsequent rounds. In order to identify clones that showed high specificity for uPAR, single clones from the third round of panning were analyzed for binding to human uPAR, murine uPAR, and BSA (as a non-specific control) by enzyme-linked immunosorbent assay (ELISA) using an anti-M13 phage antibody. Only those supernatants that showed uPAR-specific binding were selected for sequencing, resulting in the identification of thirteen clones with unique sequences (3-C3-A, 3-D8-A, 3-Gl-A, 3-H4-A, 4-F5-A, 4-F12-A, 4-AS-B. 05G9, 05A6, 05B2, 05F5, 05G5, and 07B3). Some clones showed binding to both human and mouse homologs of uPAR, e.g., 3-C3-A, 3-D8-A, 40F5A.

### Embodiments of the presently disclosed subject matter

From the foregoing description, it will be apparent that variations and modifications may be made to the presently disclosed subject matter to adopt it to various usages and conditions. Such embodiments are also within the scope of the following claims.

The recitation of a listing of elements in any definition of a variable herein includes definitions of that variable as any single element or combination (or sub-combination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.
Aspects or embodiments of the invention may also be provided according to the following paragraphs:
1. An antigen-recognizing receptor, comprising an extracellular antigen-binding domain, a transmembrane domain, and an intracellular signaling domain, wherein the extracellular antigen-binding domain specifically binds to uPAR.
2. The antigen-recognizing receptor of paragraph 1, wherein the extracellular antigen-binding domain is a single-chain variable fragment (scFv).
3. The antigen-recognizing receptor of paragraph 2, wherein the extracellular antigen-binding domain is a human scFv.
4. The antigen-recognizing receptor of paragraph 1, wherein the extracellular antigen-binding domain is a Fab, which is optionally crosslinked.
5. The antigen-recognizing receptor of paragraph 1, wherein the extracellular antigen-binding domain is a F(ab)₂.
6. The antigen-recognizing receptor of any one of paragraphs 2-5, wherein one or more of the scFv, Fab and F(ab)₂ are comprised in a fusion protein with a heterologous sequence to form the extracellular antigen-binding domain.
7. The antigen-recognizing receptor of any one of paragraphs 1-6, wherein the extracellular antigen-binding domain comprises a heavy chain variable region comprising:
   (a) a CDR1 comprising the amino acid sequence set forth in SEQ ID NO: 1 or a conservative modification thereof, a CDR2 comprising the amino acid sequence set forth in SEQ ID NO: 2 or a conservative modification thereof, and a CDR3 comprising the amino acid sequence set forth in SEQ ID NO: 3 or a conservative modification thereof;
   (b) a CDR1 comprising the amino acid sequence set forth in SEQ ID NO: 11 or a conservative modification thereof, a CDR2 comprising the amino acid sequence set forth in SEQ ID NO: 2 or a conservative modification thereof, and a CDR3 comprising the amino acid sequence set forth in SEQ ID NO: 12 or a conservative modification thereof;
   (c) a CDR1 comprising the amino acid sequence set forth in SEQ ID NO: 19 or a conservative modification thereof, a CDR2 comprising the amino acid sequence set forth in SEQ ID NO: 2 or a conservative modification thereof, and a CDR3 comprising the amino acid sequence set forth in SEQ ID NO: 20 or a conservative modification thereof;
   (d) a CDR1 comprising the amino acid sequence set forth in SEQ ID NO: 19 or a conservative modification thereof, a CDR2 comprising the amino acid sequence set forth in SEQ ID NO: 27 or a conservative modification thereof, and a CDR3 comprising the amino acid sequence set forth in SEQ ID NO: 28 or a conservative modification thereof;
   (e) a CDR1 comprising the amino acid sequence set forth in SEQ ID NO: 35 or a conservative modification thereof, a CDR2 comprising the amino acid sequence set forth in SEQ ID NO: 36 or a conservative modification thereof, and a CDR3 comprising the amino acid sequence set forth in SEQ ID NO: 37 or a conservative modification thereof;
   (f) a CDR1 comprising the amino acid sequence set forth in SEQ ID NO: 45 or a conservative modification thereof, a CDR2 comprising the amino acid sequence set forth in SEQ ID NO: 46 or a conservative modification thereof; and a CDR3 comprising the amino acid sequence set forth in SEQ ID NO: 47 or a conservative modification thereof;
   (g) a CDR1 comprising the amino acid sequence set forth in SEQ ID NO: 54 or a conservative modification thereof, a CDR2 comprising the amino acid sequence set forth in SEQ ID NO: 55 or a conservative modification thereof, and a CDR3 comprising the amino acid sequence set forth in SEQ ID NO: 56 or a conservative modification thereof;
   (h) a CDR1 comprising the amino acid sequence set forth in SEQ ID NO: 63 or a conservative modification thereof, a CDR2 comprising the amino acid sequence set forth in SEQ ID NO: 64 or a conservative modification thereof, and a CDR3 comprising the amino acid sequence set forth in SEQ ID NO: 65 or a conservative modification thereof;
   (i) a CDR1 comprising the amino acid sequence set forth in SEQ ID NO: 73 or a conservative modification thereof, a CDR2 comprising the amino acid sequence set forth in SEQ ID NO: 74 or a conservative modification thereof, and a CDR3 comprising the amino acid sequence set forth in SEQ ID NO: 75 or a conservative modification thereof;
   (j) a CDR1 comprising the amino acid sequence set forth in SEQ ID NO: 45 or a conservative modification thereof, a CDR2 comprising the amino acid sequence set forth in SEQ ID NO: 82 or a conservative modification thereof, and a CDR3 comprising the amino acid sequence set forth in SEQ ID NO: 83 or a conservative modification thereof;
   (k) a CDR1 comprising the amino acid sequence set forth in SEQ ID NO: 90 or a conservative modification thereof, a CDR2 comprising the amino acid sequence set forth in SEQ ID NO: 91 or a conservative modification thereof, and a CDR3 comprising the amino acid sequence set forth in SEQ ID NO: 92 or a conservative modification thereof;
   (l) a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 99 or a conservative modification thereof; or
   (m) a CDR1 comprising the amino acid sequence set forth in SEQ ID NO: 105 or a conservative modification thereof, a CDR2 comprising the amino acid sequence set forth in SEQ ID NO: 106 or a conservative modification thereof, and a CDR3 comprising the amino acid sequence set forth in SEQ ID NO: 107 or a conservative modification thereof.
8. The antigen-recognizing receptor of any one of paragraphs 1-7, wherein the extracellular antigen-binding domain comprises a light chain variable region comprising:
   (a) a CDR1 comprising the amino acid sequence set forth in SEQ ID NO: 4 or a conservative modification thereof, a CDR2 comprising the amino acid sequence set forth in SEQ ID NO: 5 or a conservative modification thereof, and a CDR3 comprising the amino acid sequence set forth in SEQ ID NO: 6 or a conservative modification thereof;
   (b) a CDR1 comprising the amino acid sequence set forth in SEQ ID NO: 13 or a conservative modification thereof, a CDR2 comprising the amino acid sequence set forth in SEQ ID NO: 5 or a conservative modification thereof, and a CDR3 comprising SEQ ID NO: 14 or a conservative modification thereof;
   (c) a CDR1 comprising the amino acid sequence set forth in SEQ ID NO: 21 or a conservative modification thereof, a CDR2 comprising the amino acid sequence set forth in SEQ ID NO: 5 or a conservative modification thereof, and a CDR3 comprising the amino acid sequence set forth in SEQ ID NO: 22 or a conservative modification thereof;
   (d) a CDR1 comprising the amino acid sequence set forth in SEQ ID NO: 29 or a conservative modification thereof, a CDR2 comprising SEQ ID NO: 5 or a conservative modification thereof, and a CDR3 comprising the amino acid sequence set forth in SEQ ID NO: 30 or a conservative modification thereof;
   (e) a CDR1 comprising the amino acid sequence set forth in SEQ ID NO: 38 or a conservative modification thereof, a CDR2 comprising the amino acid sequence set forth in SEQ ID NO: 39 or a conservative modification thereof, and a CDR3 comprising the amino acid sequence set forth in SEQ ID NO: 40 or a conservative modification thereof;
   (f) a CDR1 comprising the amino acid sequence set forth in SEQ ID NO: 48 or a conservative modification thereof, a CDR2 comprising the amino acid sequence set forth in SEQ ID NO: 5 or a conservative modification thereof, and a CDR3 comprising the amino acid sequence set forth in SEQ ID NO: 49 or a conservative modification thereof;
   (g) a CDR1 comprising the amino acid sequence set forth in SEQ ID NO: 57 or a conservative modification thereof, a CDR2 comprising the amino acid sequence set forth in SEQ ID NO: 5 or a conservative modification thereof, and a CDR3 comprising the amino acid sequence set forth in SEQ ID NO: 58 or a conservative modification thereof;
   (h) a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 68 or a conservative modification thereof;
   (i) a CDR1 comprising the amino acid sequence set forth in SEQ ID NO: 76 or a conservative modification thereof, a CDR2 comprising the amino acid sequence set forth in SEQ ID NO: 67 or a conservative modification thereof, and a CDR3 comprising the amino acid sequence set forth in SEQ ID NO: 77 or a conservative modification thereof;
   (j) a CDR1 comprising the amino acid sequence set forth in SEQ ID NO: 84 or a conservative modification thereof, a CDR2 comprising the amino acid sequence set forth in SEQ ID NO: 67 or a conservative modification thereof, and a CDR3 comprising the amino acid sequence set forth in SEQ ID NO: 85 or a conservative modification thereof;
   (k) a CDR1 comprising the amino acid sequence set forth in SEQ ID NO: 93 or a conservative modification thereof, a CDR2 comprising the amino acid sequence set forth in SEQ ID NO: 67 or a conservative modification thereof, and a CDR3 comprising the amino acid sequence set forth in SEQ ID NO: 94 or a conservative modification thereof; or
   (l) a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 100 or a conservative modification thereof.
9. The antigen-recognizing receptor of any one of paragraphs 1-8, wherein the extracellular antigen-binding domain comprises:
   (a) a heavy chain variable region comprising a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; and a light chain variable region comprising a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6;
   (b) a heavy chain variable region comprising a CDR1 comprising the amino acid sequence set forth in SEQ ID NO: 11, a CDR2 comprising the amino acid sequence set forth in SEQ ID NO: 2, and a CDR3 comprising the amino acid sequence set forth in SEQ ID NO: 12; and a light chain variable region comprising a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14;
   (c) a heavy chain variable region comprising a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; and a light chain variable region comprising a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22;
   (d) a heavy chain variable region comprising a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28; and a light chain variable region comprising a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30;
   (e) a heavy chain variable region comprising a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; and a light chain variable region comprising a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40;
   (f) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; and a light chain variable region comprising a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49;
   (g) a heavy chain variable region comprising a CDR1 comprising the amino acid sequence set forth in SEQ ID NO: 54, a CDR2 comprising the amino acid sequence set forth in SEQ ID NO: 55, and a CDR3 comprising the amino acid sequence set forth in SEQ ID NO: 56; and a light chain variable region comprising a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58;
   (h) a heavy chain variable region comprising a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68;
   (i) a heavy chain variable region comprising a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; and a light chain variable region comprising a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77;
   (j) a heavy chain variable region comprising a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83; and a light chain variable region comprising a CDR1 comprising the amino acid sequence set forth in SEQ ID NO: 84, a CDR2 comprising the amino acid sequence set forth in SEQ ID NO: 67, and a CDR3 comprising the amino acid sequence set forth in SEQ ID NO: 85;
   (k) a heavy chain variable region comprising a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92; and a light chain variable region comprising a CDR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94; or(l) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.
10. The antigen-recognizing receptor of any one of paragraphs 1-9, wherein the extracellular antigen-binding domain comprises a heavy chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108.
11. The antigen-recognizing receptor of any one of paragraphs 1-10, wherein the extracellular antigen-binding domain comprises a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108.
12. The antigen-recognizing receptor of any one of paragraphs 1-11, wherein the extracellular antigen-binding domain comprises a light chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.
13. The antigen-recognizing receptor of any one of paragraphs 1-12, wherein the extracellular antigen-binding domain comprises a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.
14. The antigen-recognizing receptor of any one of paragraphs 1-13, wherein the extracellular antigen-binding domain comprises: (a) a heavy chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence selected set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108; and (b) a light chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.
15. The antigen-recognizing receptor of any one of paragraphs 1-14, wherein the extracellular antigen-binding domain comprises: (a) a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108; and (b) a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.
16. The antigen-recognizing receptor of paragraph 15, wherein the extracellular antigen-binding domain comprises:
   (a) a heavy chain variable region comprising the amino acid sequence set forth in SEQ ID NO: 7, and a light chain variable region comprising the amino acid sequence set forth in SEQ ID NO: 8;
   (b) a heavy chain variable region comprising the amino acid sequence set forth in SEQ ID NO: 15, and a light chain variable region comprising the amino acid sequence set forth in SEQ ID NO: 16;
   (c) a heavy chain variable region comprising the amino acid sequence set forth in SEQ ID NO: 23, and a light chain variable region comprising the amino acid sequence set forth in SEQ ID NO: 24;
   (d) a heavy chain variable region comprising the amino acid sequence set forth in SEQ ID NO: 31, and a light chain variable region comprising the amino acid sequence set forth in SEQ ID NO: 32;
   (e) a heavy chain variable region comprising the amino acid sequence set forth in SEQ ID NO: 41, and a light chain variable region comprising the amino acid sequence set forth in SEQ ID NO: 42;
   (f) a heavy chain variable region comprising the amino acid sequence set forth in SEQ ID NO: 50, and a light chain variable region comprising the amino acid sequence set forth in SEQ ID NO: 51;
   (g) a heavy chain variable region comprising the amino acid sequence set forth in SEQ ID NO: 59, and a light chain variable region comprising the amino acid sequence set forth in SEQ ID NO: 60;
   (h) a heavy chain variable region comprising the amino acid sequence set forth in SEQ ID NO: 69, and a light chain variable region comprising the amino acid sequence set forth in SEQ ID NO: 70;
   (i) a heavy chain variable region comprising the amino acid sequence set forth in SEQ ID NO: 78, and a light chain variable region comprising the amino acid sequence set forth in SEQ ID NO: 79;
   (j) a heavy chain variable region comprising the amino acid sequence set forth in SEQ ID NO: 86, and a light chain variable region comprising the amino acid sequence set forth in SEQ ID NO: 87;
   (k) a heavy chain variable region comprising the amino acid sequence set forth in SEQ ID NO: 95, and a light chain variable region comprising the amino acid sequence set forth in SEQ ID NO: 96; or
   (l) a heavy chain variable region comprising the amino acid sequence set forth in SEQ ID NO: 101, and a light chain variable region comprising the amino acid sequence set forth in SEQ ID NO: 102.
17. The antigen-recognizing receptor of any one of paragraphs 1-16, wherein the extracellular antigen-binding domain comprises a linker between a heavy chain variable region and a light chain variable region of the extracellular antigen-binding domain.
18. The antigen-recognizing receptor of paragraph 17, wherein the linker comprises or consists of the amino acid sequence set forth in SEQ ID NO: 110, SEQ ID NO: 111, SEQ ID NO: 112, SEQ ID NO: 113, SEQ ID NO: 114, or SEQ ID NO: 115.
19. The antigen-recognizing receptor of any one of paragraphs 1-18, wherein the extracellular antigen-binding domain comprises a signal peptide that is covalently joined to the 5' terminus of the extracellular antigen-binding domain.
20. The antigen-recognizing receptor of any one of paragraphs 1-19, wherein the transmembrane domain comprises a CD8 polypeptide, a CD28 polypeptide, a CD3ζ polypeptide, a CD4 polypeptide, a 4-1BB polypeptide, an OX40 polypeptide, an ICOS polypeptide, a CTLA-4 polypeptide, a PD-1 polypeptide, a LAG-3 polypeptide, a 2B4 polypeptide, a BTLA polypeptide, or a combination thereof.
21. The antigen-recognizing receptor of any one of paragraphs 1-20, wherein the intracellular signaling domain comprises a CD3ζ polypeptide.
22. The antigen-recognizing receptor of any one of paragraphs 1-21, wherein the intracellular signaling domain further comprises at least one co-stimulatory signaling region.
23. The antigen-recognizing receptor of paragraph 22, wherein the at least one co-stimulatory signaling region comprises a CD28 polypeptide, a 4-1BB polypeptide, an OX40 polypeptide, an ICOS polypeptide, a DAP-10 polypeptide, or a combination thereof.
24. The antigen-recognizing receptor of any one of paragraphs 1-23, wherein the antigen-recognizing receptor is a chimeric antigen receptor (CAR), a T-cell Receptor (TCR), or a T-cell like fusion protein.
25. The antigen-recognizing receptor of any one of paragraphs 1-24, wherein the antigen-recognizing receptor is a CAR.
26. The antigen-recognizing receptor of any one of paragraphs 1-25, wherein the antigen-recognizing receptor is recombinantly expressed.
27. The antigen-recognizing receptor of any one of paragraphs 1-26, wherein the antigen-recognizing receptor is expressed from a vector.
28. The antigen-recognizing receptor of paragraph 27, wherein the vector is a γ-retroviral rector.
29. A cell comprising the antigen-recognizing receptor of any one of paragraphs 1-28.
30. The cell of paragraph 29, wherein the cell is transduced with the antigen-recognizing receptor.
31. The cell of paragraph 29 or 30, wherein the antigen-recognizing receptor is constitutively expressed on the surface of the cell.
32. The cell of any one of paragraphs 29-31, wherein the cell is an immunoresponsive cell.
33. The cell of any one of paragraphs 29-32, wherein the cell is a cell of the lymphoid lineage or a cell of the myeloid lineage.
34. The cell of any one of paragraphs 29-33, wherein the cell is selected from the group consisting of a T cell, a Natural Killer (NK) cell, and a stem cell from which a lymphoid cell may be differentiated.
35. The cell of any one of paragraphs 29-34, wherein the cell is a T cell.
36. The cell of paragraph 34 or 35, wherein the T cell is a cytotoxic T lymphocyte (CTL) or a regulatory T cell.
37. The cell of paragraph 34, wherein the stem cell is a pluripotent stem cell.
38. The cell of paragraph 37, wherein the pluripotent stem cell is an embryoid stem cell or an induced pluripotent stem cell.
39. A nucleic acid encoding the antigen-recognizing receptor of any one of paragraphs 1-28.
40. A vector comprising the nucleic acid of paragraph 39.
41. The vector of paragraph 40, wherein the vector is a γ-retroviral rector.
42. A host cell expressing the nucleic acid of paragraph 39 or the vector of paragraph 40 or 41.
43. The host cell of paragraph 42, wherein the host cell is a T cell.
44. A composition comprising the cell of any one of paragraphs 29-38.
45. The composition of paragraph 44, which is a pharmaceutical composition further comprising a pharmaceutically acceptable carrier.
46. A method of treating or ameliorating a disease or disorder in a subject, comprising administering to the subject the cell of any one of paragraphs 29-38, or the composition of paragraph 44 or 45.
47. The method of paragraph 46, wherein the disease or disorder is selected from the group consisting of tumors, senescence-associated pathologies, and tissue decline associated with aging.
48. The method of paragraph 47, wherein the disease or disorder is a senescence-associated pathology.
49. The method of paragraph 48, wherein the senescence-associated pathology is selected from the group consisting of lung fibrosis, atherosclerosis, Alzheimer's disease, diabetes, osteoarthritis, liver fibrosis, chronic kidney disease, cardiac fibrosis, and Parkinson's disease.
50. The method of paragraph 46, wherein the disease or disorder is a tumor.
51. The method of paragraph 50, wherein the tumor is selected from the group consisting of breast cancer, endometrial cancer, ovarian cancer, colon cancer, rectal cancer, lung cancer, stomach cancer, prostate cancer, gastric cancer, renal cancer, pancreatic cancer, blood cancer, cervical cancer, head and neck cancer, liver cancer, urotherial cancer, melanoma, and brain cancer
52. The method of paragraph 51, wherein the blood cancer is selected from the group consisting of acute lymphoblastic leukemia (ALL), chronic lymphocytic leukemia (CLL), acute myeloid leukemia (AML), myelofibrosis, polycythemia vera, myelodysplastic syndrome, and erythroleukemia.
53. The method of any one of paragraphs 50-52, wherein the tumor is cancer.
54. A method of increasing production of an immune-activating cytokine in response to a tumor cell in a subject, comprising administering to the subject the cell of any one of paragraphs 29-38, or the composition of paragraph 44 or 45.
55. The method of paragraph 54, wherein the immune-activating cytokine is selected from the group consisting of granulocyte macrophage colony stimulating factor (GM-CSF), IFN-α, IFN-β, IFN-γ, TNF-α, IL-1, IL-2, IL-3, IL-6, IL-11, IL-7, IL-8, IL-12, IL-15, IL-21, interferon regulatory factor 7 (IRF7), CCL1, CCL2, CCL3, CCL5, CCL7, CCL8, CCL13, CCL16, CXCL1, CXCL3, CXCL5, CXCL9, CXCL10, and combinations thereof.
56. The method of any one of paragraphs 46-55, wherein the subject is a human.
57. A kit for treating or ameliorating a disease or disorder in a subject, and/or increasing production of an immune-activating cytokine in response to a tumor cell in a subject, comprising the cell of any one of paragraphs 29-38, the nucleic acid of paragraph 39, or the composition of paragraph 44 or 45.
58. The kit of paragraph 57, wherein the kit further comprises written instructions for using the cell or composition for treating or ameliorating a disease or disorder in a subject, and/or increasing production of an immune-activating cytokine in response to a tumor cell in a subject.
59. A method for producing a uPAR-targeted antigen-recognizing receptor of any one of paragraphs 1-28, comprising introducing into the cell a nucleic acid that encodes the antigen-recognizing receptor.

## Claims

1. An antigen-recognizing receptor, comprising an extracellular antigen-binding domain, a transmembrane domain, and an intracellular signaling domain, wherein the extracellular antigen-binding domain specifically binds to uPAR and comprises:
(a) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.
(b) a heavy chain variable region comprising a CDR1 comprising the amino acid sequence set forth in SEQ ID NO: 11, a CDR2 comprising the amino acid sequence set forth in SEQ ID NO: 2, and a CDR3 comprising the amino acid sequence set forth in SEQ ID NO: 12; and a light chain variable region comprising a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14;
(c) a heavy chain variable region comprising a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; and a light chain variable region comprising a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22;
(d) a heavy chain variable region comprising a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28; and a light chain variable region comprising a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30;
(e) a heavy chain variable region comprising a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; and a light chain variable region comprising a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40;
(f) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; and a light chain variable region comprising a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49;
(g) a heavy chain variable region comprising a CDR1 comprising the amino acid sequence set forth in SEQ ID NO: 54, a CDR2 comprising the amino acid sequence set forth in SEQ ID NO: 55, and a CDR3 comprising the amino acid sequence set forth in SEQ ID NO: 56; and a light chain variable region comprising a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58;
(h) a heavy chain variable region comprising a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68;
(i) a heavy chain variable region comprising a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; and a light chain variable region comprising a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77;
(j) a heavy chain variable region comprising a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83; and a light chain variable region comprising a CDR1 comprising the amino acid sequence set forth in SEQ ID NO: 84, a CDR2 comprising the amino acid sequence set forth in SEQ ID NO: 67, and a CDR3 comprising the amino acid sequence set forth in SEQ ID NO: 85;
(k) a heavy chain variable region comprising a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92; and a light chain variable region comprising a CDR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94; or
(l) a heavy chain variable region comprising a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; and a light chain variable region comprising a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6;

2. The antigen-recognizing receptor of claim 1, wherein the extracellular antigen-binding domain comprises:
(a) a heavy chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 101, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 108, or SEQ ID NO: 7; and a light chain variable region comprising an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 102, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 8; or
(b) a heavy chain variable region comprising the amino acid sequence set forth in SEQ ID NO: 101, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 108, or SEQ ID NO: 7; and a light chain variable region comprising the amino acid sequence set forth in SEQ ID NO: 102, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 8.

3. The antigen-recognizing receptor of claim 1 or claim 2, wherein the extracellular antigen-binding domain comprises:
(a) a heavy chain variable region comprising the amino acid sequence set forth in SEQ ID NO: 101, and a light chain variable region comprising the amino acid sequence set forth in SEQ ID NO: 102;
(b) a heavy chain variable region comprising the amino acid sequence set forth in SEQ ID NO: 15, and a light chain variable region comprising the amino acid sequence set forth in SEQ ID NO: 16;
(c) a heavy chain variable region comprising the amino acid sequence set forth in SEQ ID NO: 23, and a light chain variable region comprising the amino acid sequence set forth in SEQ ID NO: 24;
(d) a heavy chain variable region comprising the amino acid sequence set forth in SEQ ID NO: 31, and a light chain variable region comprising the amino acid sequence set forth in SEQ ID NO: 32;
(e) a heavy chain variable region comprising the amino acid sequence set forth in SEQ ID NO: 41, and a light chain variable region comprising the amino acid sequence set forth in SEQ ID NO: 42;
(f) a heavy chain variable region comprising the amino acid sequence set forth in SEQ ID NO: 50, and a light chain variable region comprising the amino acid sequence set forth in SEQ ID NO: 51;
(g) a heavy chain variable region comprising the amino acid sequence set forth in SEQ ID NO: 59, and a light chain variable region comprising the amino acid sequence set forth in SEQ ID NO: 60;
(h) a heavy chain variable region comprising the amino acid sequence set forth in SEQ ID NO: 69, and a light chain variable region comprising the amino acid sequence set forth in SEQ ID NO: 70;
(i) a heavy chain variable region comprising the amino acid sequence set forth in SEQ ID NO: 78, and a light chain variable region comprising the amino acid sequence set forth in SEQ ID NO: 79;
(j) a heavy chain variable region comprising the amino acid sequence set forth in SEQ ID NO: 86, and a light chain variable region comprising the amino acid sequence set forth in SEQ ID NO: 87;
(k) a heavy chain variable region comprising the amino acid sequence set forth in SEQ ID NO: 95, and a light chain variable region comprising the amino acid sequence set forth in SEQ ID NO: 96; or
(l) a heavy chain variable region comprising the amino acid sequence set forth in SEQ ID NO: 7, and a light chain variable region comprising the amino acid sequence set forth in SEQ ID NO: 8.

4. The antigen-recognizing receptor of any one of claims 1-3, wherein the extracellular antigen-binding domain comprises a linker between a heavy chain variable region and a light chain variable region of the extracellular antigen-binding domain; optionally wherein, the linker comprises or consists of the amino acid sequence set forth in SEQ ID NO: 110, SEQ ID NO: 111, SEQ ID NO: 112, SEQ ID NO: 113, SEQ ID NO: 114, or SEQ ID NO: 115.

5. The antigen-recognizing receptor of any one of claims 1-4, wherein:
(a) the extracellular antigen-binding domain comprises a signal peptide that is covalently joined to the 5' terminus of the extracellular antigen-binding domain;
(b) the transmembrane domain comprises a CD8 polypeptide, a CD28 polypeptide, a CD3ζ polypeptide, a CD4 polypeptide, a 4-1BB polypeptide, an OX40 polypeptide, an ICOS polypeptide, a CTLA-4 polypeptide, a PD-1 polypeptide, a LAG-3 polypeptide, a 2B4 polypeptide, a BTLA polypeptide, or a combination thereof; and/or
(c) the intracellular signaling domain comprises a CD3ζ polypeptide; optionally wherein, wherein the intracellular signaling domain further comprises at least one co-stimulatory signaling region; optionally wherein, the at least one co-stimulatory signaling region comprises a CD28 polypeptide, a 4-1BB polypeptide, an OX40 polypeptide, an ICOS polypeptide, a DAP-10 polypeptide, or a combination thereof.

6. The antigen-recognizing receptor of any one of claims 1-5, wherein the antigen-recognizing receptor is a chimeric antigen receptor (CAR), a T-cell Receptor (TCR), or a T-cell like fusion protein.

7. An immunosuppressive cell comprising the antigen-recognizing receptor of any one of claims 1-6; optionally wherein the immunosuppressive cell is a cell of the lymphoid lineage or a cell of the myeloid lineage.

8. The immunosuppressive cell of claim 7, wherein the cell is selected from the group consisting of:
(a) a T cell, optionally wherein the T cell is a cytotoxic T lymphocyte (CTL) or a regulatory T cell;
(b) a Natural Killer (NK) cell, and
(c) a stem cell from which a lymphoid cell may be differentiated, optionally wherein the stem cell is a pluripotent stem cell.

9. A host cell comprising:
(a) a nucleic acid encoding an antigen-recognizing receptor of any one of claims 1-6;
or
(b) a vector comprising a nucleic acid encoding an antigen-recognizing receptor of any one of claims 1-6.

10. A composition comprising the immunosuppressive cell of claim 7 or claim 8; optionally wherein, the composition is a pharmaceutical composition further comprising a pharmaceutically acceptable carrier.

11. The immunosuppressive cell of claim 7 or claim 8, or the composition of claim 10, for use in a method of treating or ameliorating a disease or disorder in a subject; optionally wherein, the disease or disorder is selected from the group consisting of tumors, senescence-associated pathologies, and tissue decline associated with aging.

12. The immunosuppressive cell or the composition for use of claim 11, wherein:
(a) the disease or disorder is a senescence-associated pathology; optionally wherein, the senescence-associated pathology is selected from the group consisting of lung fibrosis, atherosclerosis, Alzheimer's disease, diabetes, osteoarthritis, liver fibrosis, chronic kidney disease, cardiac fibrosis, and Parkinson's disease;
(b) the disease or disorder is a tumor; optionally wherein, the tumor is selected from the group consisting of breast cancer, endometrial cancer, ovarian cancer, colon cancer, rectal cancer, lung cancer, stomach cancer, prostate cancer, gastric cancer, renal cancer, pancreatic cancer, blood cancer, cervical cancer, head and neck cancer, liver cancer, urothelial cancer, melanoma, and brain cancer; or
(c) the disease or disorder is a blood cancer; optionally wherein, the blood cancer is selected from the group consisting of acute lymphoblastic leukemia (ALL), chronic lymphocytic leukemia (CLL), acute myeloid leukemia (AML), myelofibrosis, polycythemia vera, myelodysplastic syndrome, and erythroleukemia.

13. A method of increasing production of an immune-activating cytokine in response to a tumor cell in a subject, comprising administering to the subject the immunosuppressive cell of claim 7 or claim 8, or the composition of claim 10; optionally wherein, the immune-activating cytokine is selected from the group consisting of granulocyte macrophage colony stimulating factor (GM-CSF), IFN-α, IFN-β, IFN-γ, TNF- α, IL-1, IL-2, IL-3, IL-6, IL-11, IL-7, IL-8, IL-12, IL-15, IL-21, interferon regulatory factor 7 (IRF7), CCL1, CCL2, CCL3, CCL5, CCL7, CCL8, CCL13, CCL16, CXCL1, CXCL3, CXCL5, CXCL9, CXCL10, and combinations thereof.

14. A kit for treating or ameliorating a disease or disorder in a subject, and/or increasing production of an immune-activating cytokine in response to a tumor cell in a subject, comprising the immunosuppressive cell of claim 7 or claim 8, a nucleic acid encoding an antigen-recognizing receptor of any one of claims 1-6, or the composition of claim 10; optionally wherein, the kit further comprises written instructions for using the cell or composition for treating or ameliorating a disease or disorder in a subject, and/or increasing production of an immune-activating cytokine in response to a tumor cell in a subject..

15. A method for producing a uPAR-targeted antigen-recognizing receptor of any one of claims 1-6, comprising introducing into a cell a nucleic acid that encodes the antigen-recognizing receptor.
